# EUROPEAN PATENT APPLICATION

(11) **EP 3 480 314 A1**
(43) Date of publication of application: **08.05.2019**
(21) Application number: 17200026.7
(22) Date of filing: 03.11.2017
(51) Int. Cl.: C12N 15/82

(54) **REGULATION OF ALKALOID CONTENT**

(71) Applicant: Philip Morris Products S.A., 2000 Neuchatel (CH)
(72) Inventor: The designation of the inventor has not yet been filed
(74) Representative: Maschio & Soames IP Ltd

(57) **Abstract**

A plant cell comprising at least one modification which modulates the expression or activity of polynucleotides or polypeptides homologous to SEQ ID NOs: 1-4. A method for producing a plant with modulated expression or activity of polynucleotides or polypeptides homologous to SEQ ID NOs: 1-4. Plants with modulated alkaloid concentrations, and methods for their production.

## Description

### FIELD OF THE INVENTION

The present invention relates to modified plants having modified alkaloid levels, methods for obtaining plants having modified alkaloid levels, by altering the gene expression of putative ABC-2 transporters *NtABCG1-T* and *NtABCG1-S* and their homologues.

### BACKGROUND

Several nicotine biosynthesis enzymes are known. The tobacco quinolate phosphoribosyl transferase *(QPD)* gene has been cloned, see U.S. patent No. 6,423,520 and Sinclair et al., Plant Mol. Biol. 44: 603-17 (2000), and its suppression provides significant nicotine reductions in transgenic tobacco plants. Xie et al., Recent Advances in Tobacco Science 30: 17-37 (2004). Likewise, suppression of an endogenous putrescine methyl transferase (PMT) sequence has been shown to reduce nicotine levels but increase anatabine levels by about 2-to-6-fold. Hibi et al., Plant Cell 6: 723-35 (1994); Chintapakom and Hamill, Plant Mol. Bioi. 53:87-105 (2003); Steppuhn et al. PLoS Biol2:8:e217:1074-1080 (2004).

US20080120737 discloses and claims four genes, A622, NBB1, PMT and QPT that can be influenced to increase nicotinic alkaloid levels in Nicotiana plants. In one aspect, the invention disclosed in US20080120737 provides a method for increasing alkaloids, such as nicotine, in a Nicotiana plant, by overexpressing at least one of A622 and NBB1 relative to a control plant. US20080292735 discloses and claims MPO1 and MPO2 for either decreasing or increasing alkaloid levels in plants, in particular in Nicotiana plants. In particular, suppressing or overexpressing one or more of MPO1 and MPO2 may be used to decrease or increase nicotine and nicotinic alkaloid levels in tobacco plants.

The ATP-binding cassette (ABC) transporter gene superfamily is ubiquitous among all organisms and prominently represented in plants. ABC transporters act to transport compounds across cellular membranes and are involved in a diverse range of biological processes. There are several known functions of ABC transporters of agricultural importance. One of the most important is detoxification relating to herbicide resistance. Shared among members within the ABC transporter protein superfamily is the ability to hydrolyze adenosine triphosphate (ATP). ABC transporters have three structural types. Full transporters are composed of two transmembrane domains (TMD) and two nucleotide-binding domains (NBD). Half transporters are composed of one TMD and one NBD, which dimerize in pairs to create virtual full transporters as homodimers or heterodimers. A third type of transporter has no TMDs but two NBDs. Plant ABC transporters fall into eight subfamilies using this nomenclature: A, B, C, D, E, F, G, and I. Several studies have indicated that plant genomes have more ABCB, ABCC and/or ABCG subfamily gene members in comparison to other eukaryotes such as human and yeast, and there are significant differences in the number of unique ABC transporter gene members between various plant genera. (Lane et al., BMC Biotechnol. 2016, 16:47)

There is a continued interest in modifying the levels of alkaloids in plants. One such interest is in modifying the levels of nicotine in tobacco plants, either for use in smoking articles or as starting material for extracting nicotine for various uses, including for use in insecticides, in consumables for electronic cigarettes (e-liquids), and for medicinal purposes.

There are also ongoing medical and commercial interests in developing plants with modulated levels of alkaloids such as caffeine, codeine, and morphine.

### SUMMARY OF INVENTION

The present invention is directed, in one aspect, to a plant cell comprising *NtABCG1-T* or *NtABCG1-S* gene sequences and polypeptides, i.e. a plant cell comprising:
(i) a polynucleotide comprising, consisting or consisting essentially of a sequence having at least 80% sequence identity to SEQ ID NO: 1 or SEQ ID No: 3;
(ii) a polypeptide encoded by the polynucleotide set forth in (i);
(iii) a polypeptide comprising, consisting or consisting essentially of a sequence having at least 80% sequence identity to SEQ ID NO: 2 or SEQ ID No: 4; or
(iv) a construct, vector or expression vector comprising the isolated polynucleotide set forth in (i), wherein said plant cell comprises at least one modification which modulates the expression or activity of said polynucleotide or polypeptide as compared to a control plant cell in which the expression or activity of said polynucleotide or polypeptide has not been altered. Preferably, the plant cell also comprises a modification which modulates the expression or activity of one or more of *A622, BBLa, BBLb, JRE5L1, JRE5L2, MATE1, MATE 2, MPO1, MPO2*, *MYC2a*, *MYC2b*, *NBB1*, *nic1*, *nic2*, *NUP1*, *NUP2*, *PMT* (e.g. *PMT1*, *PMT2*, *PMT3*, *PMT4*), *QPT,* and their gene products.

The modification may be a modification of the genome or of the construct, vector, or expression vector. Alternatively, or in addition, the plant cell may be modified by the introduction of a transgene.

The modification of the genome, construct, vector, or expression vector may be a mutation or an edit. Preferred methods for mutating and editing polynucleotides are discussed in more detail below. The mutation or edit may be located in a regulatory sequence governing expression from the polynucleotide.

The modulation of expression or activity may be an increase or a decrease in expression or activity, which may also be described as upregulation and downregulation of expression or activity, or a gain of function and a reduction of function. Both forms of modulation may be achieved by the methods described herein and methods known in the art. The choice of whether to increase or decrease expression or activity depends on the particular application of the invention. Where a desired effect is produced by the expression or activity of *NtABCG1-T* or *NtABCG1-S* gene sequences and polypeptides, then the choice will be to increase expression and activity thereof. Where a desired effect is inhibited or reduced by the expression or activity of *NtABCG1-T* or *NtABCG1-S* gene sequences and polypeptides, then the choice will be to decrease expression thereof.

Therefore, in one embodiment, the polynucleotide is operably linked to a constitutive promoter. This embodiment is particularly advantageous when the desired effect is produced by the expression or activity of *NtABCG1-T* or *NtABCG1-S* gene sequences and polypeptides. A suitable constitutive promoter is the 35S promoter of the cauliflower mosaic virus. Alternatively, the polynucleotide may be operably linked to a promoter that is activated by a substance present in the growth medium, thus effectively being constitutively active when in this environment.

In another embodiment, the plant cell comprises an interference polynucleotide. This embodiment is particularly advantageous when the desired effect is reduced or inhibited by the expression or activity of *NtABCG1-T* or *NtABCG1-S* gene sequences and polypeptides because the interference polynucleotide disrupts or "interferes" with said expression or activity. A suitable interference polynucleotide comprises a sequence that is at least 80% complementary to at least 19 nucleotides of an RNA transcribed from a polynucleotide comprising a sequence having at least 80% sequence identity to SEQ ID NO: 1 or SEQ ID NO: 3.

In another aspect, the present invention is directed to a plant or part thereof comprising the heretofore described plant cell. In embodiments, the desired effect of modulating the expression or activity of *NtABCG1-T* or *NtABCG1-S* gene sequences and polypeptides is a modification, e.g. an increase or a decrease, in the concentration of at least one alkaloid in at least a part of the plant as compared to a control plant that does not comprise such a cell. Alkaloids of greatest interest include nicotine, anatabine, caffeine, codeine, and morphine.

In another aspect, the invention provides plant material including biomass, seed, stem, flowers, or leaves from the plant.

There is also provided a tobacco product comprising the plant cell, at least a part of the plant, or the plant material according to the invention. Also provided are tobacco, cured tobacco and homogenized tobacco material. The most commonly used forms of homogenized tobacco material are reconstituted tobacco sheet and cast leaf sheet. The process to form homogenized tobacco material sheets commonly comprises a step in which tobacco particles and one or more binder(s), are mixed to form a slurry. The slurry is then used to create a tobacco web, for example by casting a viscous slurry onto a moving metal belt to produce a continuous sheet of cast leaf. Alternatively, a slurry with low viscosity and high water content can be used to create reconstituted tobacco in a process that resembles paper-making. Once prepared, homogenized tobacco webs may be cut in a similar fashion as whole leaf tobacco to produce tobacco cut filler suitable for cigarettes, other smoking articles or aerosol forming material. The homogenized tobacco material may be crimped or cut to a specification suitable for making cigarettes, other smoking articles or aerosol forming material.

In a further aspect, the invention provides a method for producing a modified plant, comprising the steps of:
(a) providing a plant cell comprising a polynucleotide comprising, consisting or consisting essentially of a sequence having at least 80% sequence identity to at least one of SEQ ID NO: 1 or SEQ ID No: 3;
(b) modifying the plant cell to modulate the expression or activity of said polynucleotide or polypeptide as compared to a control plant cell in which the expression or activity of said polynucleotide or polypeptide has not been altered; and
(c) cultivating a plant comprising the plant cell,

Preferably, the plant cell is also modified to modulate the expression or activity of one or more of A622, BBLa, BBLb, JRE5L1, JRE5L2, MATE1, MATE 2, MPO1, MPO2, MYC2a, MYC2b, NBB1, nic1, nic2, NUP1, NUP2, PMT (e.g. PMT1, PMT2, PMT3, PMT4), QPT, and their gene products.

In certain embodiments, the modification of the plant (such as a tobacco plant) leads to a gain of function of the polynucleotide or the encoded polypeptide, resulting in a reduction of an alkaloid (such as nicotine) in the plant or in a plant part. In other embodiments, the modification of the plant (such as a tobacco plant) leads to a loss of function of the polynucleotide or the encoded polypeptide, resulting in an increase in an alkaloid (such as nicotine) in the plant or in a plant part. In embodiments, the step of modifying the plant cell comprises modifying the genome of the cell by a genome editing technology or by genome engineering techniques selected from CRISPR/Cas technology, zinc finger nuclease-mediated mutagenesis, chemical or radiation mutagenesis, homologous recombination, oligonucleotide-directed mutagenesis and meganuclease-mediated mutagenesis. In embodiments, the expression or activity of the nucleotide or polypeptide is modulated by modifying the genome of the plant at a location within the *NtABCG1-T* or *NtABCG1-S* gene sequence (e.g. SEQ ID NOs: 1 & 3). In some embodiments, the expression or activity of the nucleotide or polypeptide is modulated by modifying the genome of the plant at a location outside the *NtABCG1-T* or *NtABCG1-S* gene sequence (e.g. SEQ ID NOs: 1 & 3), for example in a regulatory sequence such as a promoter or a cis- or transacting enhancer.

In embodiments, the step of modifying the plant cell comprises operably linking the polynucleotide to a constitutive promoter. Where the polynucleotide is endogenous, this may be achieved by one of the genome editing techniques described herein. For example, homologous recombination may be used to insert a constitutively active or readily inducible promoter upstream of the polynucleotide. Alternatively, where the polynucleotide is exogenous, standard genetic cloning techniques can be used to generate a vector comprising a constitutively active or readily inducible promoter upstream of the polynucleotide before transfecting the vector into the plant cell. Thus, in embodiments, the step of modifying the plant cell comprises transfecting the cell with a construct comprising a polynucleotide comprising, consisting, or consisting essentially of a sequence having at least 80% sequence identity to SEQ ID NO: 1 or SEQ ID NO: 3 operably linked to a constitutive promoter.

In embodiments of the method, the step of modifying the plant cell comprises introducing an interference polynucleotide comprising a sequence that is at least 80% complementary to an RNA transcribed from the polynucleotide of step (a) into the cell. In a particular example of this embodiment, the plant cell is transfected with a construct expressing an interference polynucleotide comprising a sequence that is at least 80% complementary to at least 19 nucleotides of an RNA transcribed from the polynucleotide of step (a).

In a related aspect, the invention is directed to a method for producing cured plant material, preferably cured leaves, stems or flowers, with an altered alkaloid concentration as compared to control plant material comprising the steps of:
(a) providing a plant or plant material according to any embodiment heretofore described;
(b) optionally harvesting the plant material therefrom; and
(c) curing the plant material, preferably wherein the curing procedure is selected from the group consisting of air curing, fire curing, smoke curing, and flue curing.

In another related aspect, the invention is directed to a method for producing homogenized plant material with an altered alkaloid concentration as compared to control plant material comprising grinding the cured leaves, stems, flowers or a mixture thereof to form fine tobacco particles, mixing the fine tobacco particles with water and binder(s) to form a slurry, and forming a web of homogenized plant material from the slurry. Preferably, the homogenized plant material is homogenized tobacco material.

### BRIEF DESCRIPTION OF THE DRAWINGS

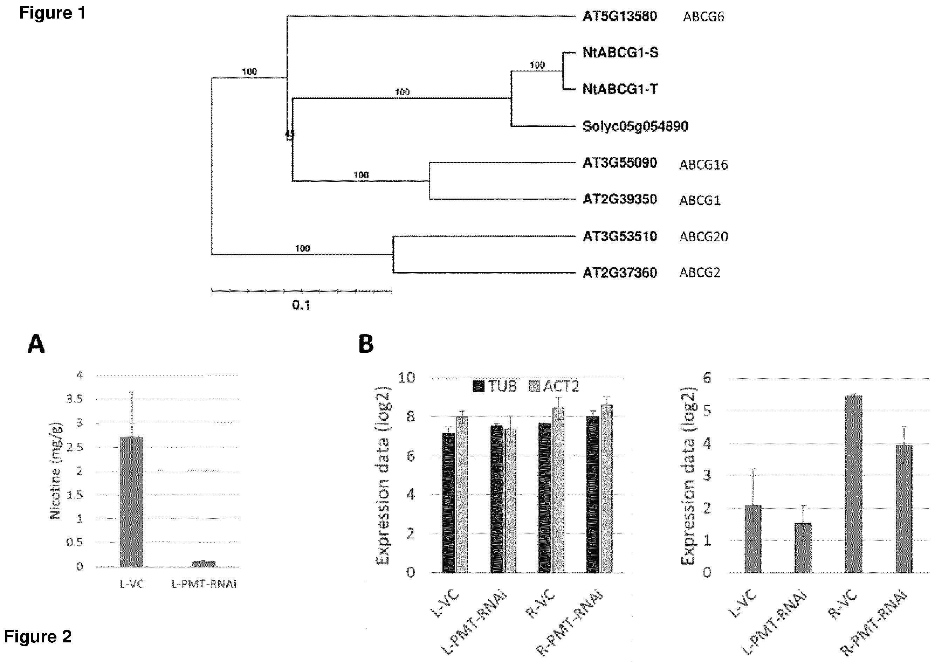

**Figure 1****.** Phylogenetic tree showing the homology of *NtABCG1-S* and *NtABCG1-T* gene products (see SEQ ID 2 and 4) with the tomato orthologue Solyc05g054890 and the closest Arabidopsis ABCG proteins.
**Figure 2****.** Leaf nicotine levels (A) and expression of *NtABCG1 S* and *T* in roots of PMT-RNAi lines (B). The expression of *NtABCG1S* and *-T* genes (probe NtPMIa1g97272e1_st) was determined using Tobarray-Affymetrix in root (R) and leaf (L) of vector control plants (R-VC and L-VC) and PMT-RNAi lines (R-PMT-RNAi and L-PMT-RNAi). PMT is putrescine methyl transferase. As control, house-keeping genes expression of tubulin (NtPMIa1g44157e1_s_st) and actin 2 (NtPMIalg48889e3_st) are shown.

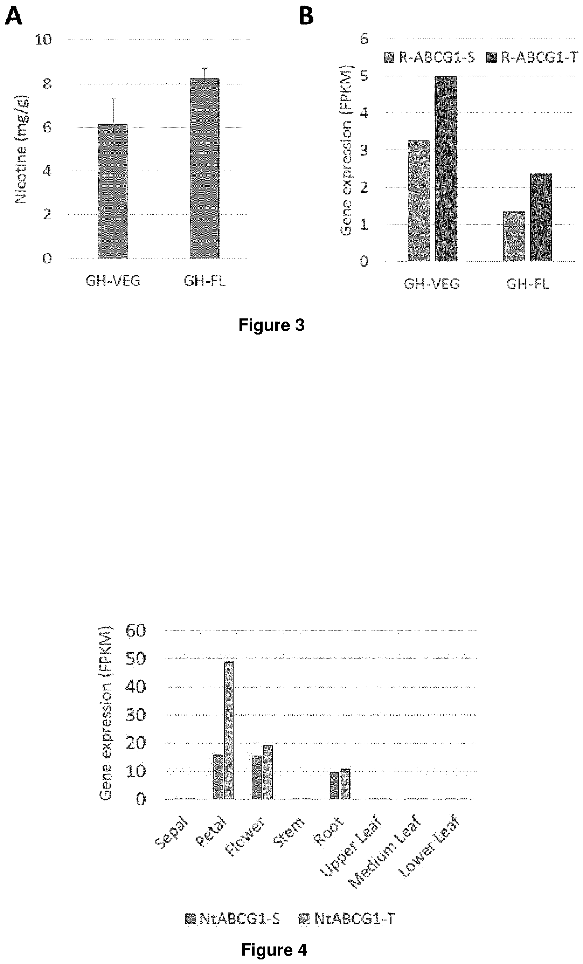

**Figure 3****.** Nicotine content in leaves of vegetative and flowering plants grown under greenhouse environment (A) and expression of *NtABCG1 S* and *T* in the roots (B). Leaves at identical mid-stalk position were collected in the greenhouse in 4 vegetative and 4 flowering plants and nicotine determined. At the same time, total RNA was isolated from roots of the same plants and subjected to RNA-seq analyses (p<0.05).
**Figure 4****.** Expression of *NtABCG1-S* and *NtABCG1-T* from field grown tobacco flowering plants.

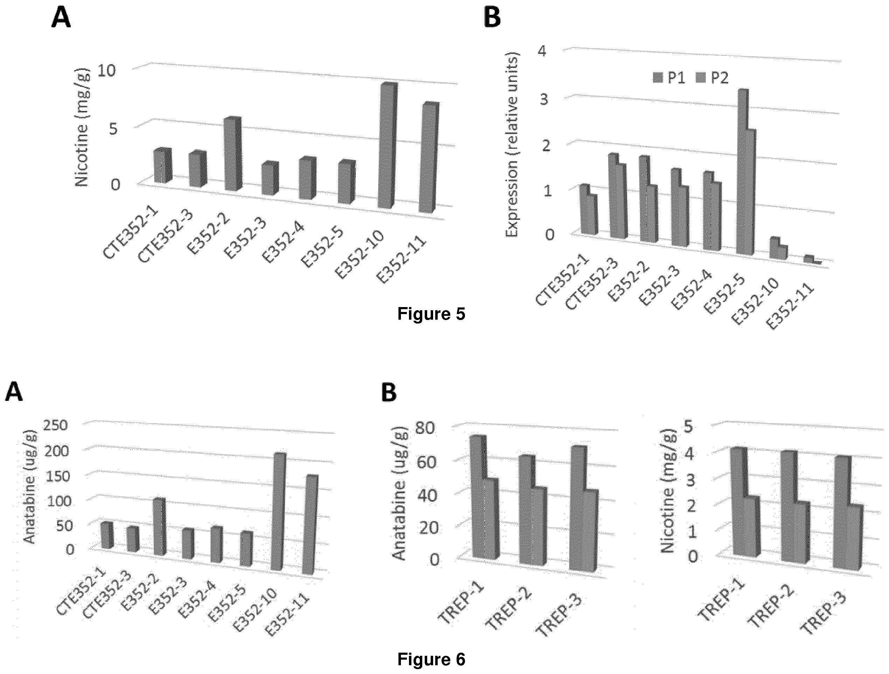

**Figure 5****.** Nicotine content in mid-bottom leaf lamina of NtABCG1-RNAi lines and control plants (A). qPCR expression of *NtABCG1-S* and *-T* genes in early flowering buds of NtABCG1-RNAi lines and control plants (B). Four mid-bottom leaves collected from each plant were dried at 45°C for 5 days, pooled and analyzed for nicotine content. Once RNA isolated from young flowers, qPCR were run on the corresponding cDNA with two different couples of primers P1 and P2 amplifying both *NtABCG1* copies.
**Figure 6****.** Anatabine content in mid-bottom leaf lamina of NtABCG1-RNAi lines and control (A). Two tobacco technical analytical replicates (3) for anatabine and nicotine estimation measurements (B).

### DETAILED DESCRIPTION

The present invention is directed, in one aspect, to a novel strategy for modulating alkaloid content in plants by altering the expression or activity of newly identified genes *NtABCG1-T* or *NtABCG1-S,* homologues of *NtABCG1-T* or *NtABCG1-S*, and their gene products. Alkaloid levels are either increased or decreased by manipulating the expression and activity of *NtABCG1-T* or *NtABCG1-S* and their gene products.

Section headings as used in this section and the entire disclosure herein are merely for organization purposes and are not intended to be limiting.

### 1. Definitions

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present invention. All publications, patent applications, patents and other references mentioned herein are incorporated by reference in their entirety. The materials, methods, and examples disclosed herein are illustrative only and not intended to be limiting.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "and" and "the" include plural references unless the context clearly dictates otherwise. The present disclosure also contemplates other embodiments "comprising," "consisting of' and "consisting essentially of," the embodiments or elements presented herein, whether explicitly set forth or not. For the recitation of numeric ranges herein, each intervening number there between with the same degree of precision is explicitly contemplated. For example, for the range 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6.9 and 7.0 are explicitly contemplated.

As used throughout the specification and the claims, the following terms have the following meanings:
"Coding sequence" or "encoding nucleic acid" as used herein means the nucleic acids (RNA or DNA molecule) that comprise a nucleotide sequence which encodes a protein. The coding sequence can further include initiation and termination signals operably linked to regulatory elements including a promoter and polyadenylation signal capable of directing expression in the cells of an individual or mammal to which the nucleic acid is administered. The coding sequence may be codon optimized.

"Complement" or "complementary" as used herein means a nucleic acid can mean Watson-Crick (*e.g.*, A-T/U and C-G) or Hoogsteen base pairing between nucleotides or nucleotide analogs of nucleic acid molecules. "Complementarity" refers to a property shared between two nucleic acid sequences, such that when they are aligned antiparallel to each other, the nucleotide bases at each position will be complementary.

"Construct" as used herein refers to a double-stranded, recombinant nucleic acid fragment comprising one or more polynucleotides. The construct comprises a "template strand" base-paired with a complementary "sense or coding strand." A given construct can be inserted into a vector in two possible orientations, either in the same (or sense) orientation or in the reverse (or anti-sense) orientation with respect to the orientation of a promoter positioned within a vector - such as an expression vector.

The term "control" as used herein in the context of a control plant or control plant cells means a plant or plant cells in which the expression or activity of *NtABCG1-T* or *NtABCG1-S* has not been modified (for example, increased or decreased) and so it can provide a comparison with a plant in which the expression or activity of *NtABCG1-T* or *NtABCG1-S* has been modified. As used herein, a "control plant" is a plant that is substantially equivalent to a test plant or modified plant in all parameters with the exception of the test parameters. For example, when referring to a plant into which a polynucleotide according to the present invention has been introduced, in certain embodiments, a control plant is an equivalent plant into which no such polynucleotide has been introduced. In certain embodiments, a control plant is an equivalent plant into which a control polynucleotide has been introduced. In such instances, the control polynucleotide is one that is expected to result in little or no phenotypic effect on the plant. The control plant may comprise an empty vector. The control plant may correspond to a wild-type plant. The control plant may be a null segregant wherein the T1 segregant no longer possesses the transgene.

"Donor DNA" or "donor template" as used interchangeably herein refers to a double-stranded DNA fragment or molecule that includes at least a portion of the gene of interest. The donor DNA may encode a full-functional protein or a partially-functional protein.

"Endogenous gene" as used herein refers to a gene that originates from the genome of the organism and has not undergone a change, such as a loss, gain, or exchange of genetic material. An endogenous gene undergoes normal gene transmission and gene expression.

"Enhancer sequences" as used herein refer to the sequences that can increase gene expression. These sequences can be located upstream, within introns or downstream of the transcribed region. The transcribed region is comprised of the exons and the intervening introns, from the promoter to the transcription termination region. The enhancement of gene expression can be through various mechanisms which include, but are not limited to, increasing transcriptional efficiency, stabilization of mature mRNA and translational enhancement.

"Expression" as used herein refers to the production of a functional product. For example, expression of a nucleic acid fragment may refer to transcription of the nucleic acid fragment (e.g., transcription resulting in mRNA or functional RNA) and/or translation of mRNA into a precursor or mature protein. "Overexpression" refers to the production of a gene product in transgenic organisms that exceeds levels of production in a null segregating (or non-transgenic) organism from the same experiment.

"Functional" and "full-functional" as used herein describes protein that has biological activity. A "functional gene" refers to a gene transcribed to mRNA, which is translated to a functional protein.

"Genetic construct" as used herein refers to the DNA or RNA molecules that comprise a nucleotide sequence that encodes a protein. The coding sequence includes initiation and termination signals operably linked to regulatory elements including a promoter and polyadenylation signal capable of directing expression in the cells of the individual to whom the nucleic acid molecule is administered.

"Genome editing" as used herein refers to changing an endogenous gene that encodes an endogenous polypeptide or protein, such that protein expression of a truncated endogenous protein or an endogenous protein having an amino acid substitution is obtained. Genome editing may include replacing the region of the endogenous gene to be targeted or replacing the entire endogenous gene with a copy of the gene that has a truncation or an amino acid substitution with a repair mechanism such as homology-directed repair (HDR). Genome editing may include may also include generating an amino acid substitution in the endogenous gene by generating a double stranded break in the endogenous gene that is then repaired using non-homologous end joining (NHEJ). NHEJ may add or delete at least one base pair during repair which may generate an amino acid substitution. Genome editing may also include deleting a gene segment by the simultaneous action of two nucleases on the same DNA strand in order to create a truncation between the two nuclease target sites and repairing the DNA break by NHEJ.

"Heterologous" as used herein with respect to a sequence means a sequence that originates from a foreign species, or, if from the same species, is substantially modified from its native form in composition and/or genomic locus by deliberate human intervention.

"Homology-directed repair" or "HDR" as used interchangeably herein refers to a mechanism in cells to repair double strand DNA lesions when a homologous piece of DNA is present in the nucleus, mostly in G2 and S phase of the cell cycle. HDR uses a donor DNA or donor template to guide repair and may be used to create specific sequence changes to the genome, including the targeted addition of whole genes. If a donor template is provided along with the site specific nuclease, then the cellular machinery will repair the break by homologous recombination, which is enhanced several orders of magnitude in the presence of DNA cleavage. When the homologous DNA piece is absent, non-homologous end joining may take place instead.

The terms "homology" or "similarity" as used herein refer to the degree of sequence similarity between two polypeptides or between two nucleic acid molecules compared by sequence alignment. The degree of homology between two discrete nucleic acid sequences being compared is a function of the number of identical, or matching, nucleotides at comparable positions.

"Identical" or "identity" as used herein in the context of two or more nucleic acids or polypeptide sequences means that the sequences have a specified percentage of residues that are the same over a specified region. The percentage may be calculated by optimally aligning the two sequences, comparing the two sequences over the specified region, determining the number of positions at which the identical residue occurs in both sequences to yield the number of matched positions, dividing the number of matched positions by the total number of positions in the specified region, and multiplying the result by 100 to yield the percentage of sequence identity. In cases where the two sequences are of different lengths or the alignment produces one or more staggered ends and the specified region of comparison includes only a single sequence, the residues of single sequence are included in the denominator but not the numerator of the calculation. When comparing DNA and RNA, thymine (T) and uracil (U) may be considered equivalent. Identity may be performed manually or by using a computer sequence algorithm such as ClustalW, ClustalX, BLAST, FASTA or Smith-Waterman.

The term "increase" or "increased" as used herein, refers to an increase of from about 10% to about 99%, or an increase of at least 10%, at least 20%, at least 25%, at least 30%, at least 40%, at least 50%, at least 60%, at least 70%, at least 75%, at least 80%, at least 90%, at least 95%, at least 98%, at least 99%, at least 100%, at least 150%, or at least 200% or more or more of a quantity or an activity, such as but not limited to nitrate levels or TSNA levels.

The term "inhibit" or "inhibited" as used herein, refers to a reduction of from about 98% to about 100%, or a reduction of at least 98%, at least 99%, but particularly of 100%, of a quantity or an activity, such as but not limited to polypeptide activity, transcriptional activity, and/or protein expression.

The term "introduced" as used herein means providing a nucleic acid (e.g., expression construct) or protein into a cell. Introduced includes reference to the incorporation of a nucleic acid into a eukaryotic cell where the nucleic acid may be incorporated into the genome of the cell, and includes reference to the transient provision of a nucleic acid or protein to the cell. Introduced includes reference to stable or transient transformation methods, as well as sexually crossing. Thus, "introduced" in the context of inserting a nucleic acid fragment (e.g., a recombinant DNA construct/expression construct) into a cell, means "transfection" or "transformation" or "transduction" and includes reference to the incorporation of a nucleic acid fragment into a eukaryotic cell where the nucleic acid fragment may be incorporated into the genome of the cell (e.g., chromosome, plasmid, plastid or mitochondrial DNA), converted into an autonomous replicon, or transiently expressed (e.g., transfected mRNA).

The terms "isolated," "purified" or "biologically pure" as used herein refer to material that is substantially or essentially free from components that normally accompany it as found in its native state. Purity and homogeneity are typically determined using analytical chemistry techniques such as polyacrylamide gel electrophoresis or high performance liquid chromatography. A protein that is the predominant species present in a preparation is substantially purified. In particular, an isolated nucleic acid of the present invention is separated from open reading frames that flank the desired gene and encode proteins other than the desired protein. The term "purified" as used herein denotes that a nucleic acid or protein gives rise to essentially one band in an electrophoretic gel. Particularly, it means that the nucleic acid or protein is at least 85% pure, more preferably at least 95% pure, and most preferably at least 99% pure.

Isolated polynucleotides may be purified from a host cell in which they naturally occur. Conventional nucleic acid purification methods known to skilled artisans may be used to obtain isolated polynucleotides. The term also embraces recombinant polynucleotides and chemically synthesized polynucleotides.

"Non-homologous end joining (NHEJ) pathway" as used herein refers to a pathway that repairs double-strand breaks in DNA by directly ligating the break ends without the need for a homologous template. The template-independent re-ligation of DNA ends by NHEJ is a stochastic, error-prone repair process that introduces random micro-insertions and micro-deletions (indels) at the DNA breakpoint. This method may be used to intentionally disrupt, delete, or alter the reading frame of targeted gene sequences. NHEJ typically uses short homologous DNA sequences called microhomologies to guide repair. These microhomologies are often present in single-stranded overhangs on the end of double-strand breaks. When the overhangs are perfectly compatible, NHEJ usually repairs the break accurately, yet imprecise repair leading to loss of nucleotides may also occur, but is much more common when the overhangs are not compatible. "Nuclease mediated NHEJ" as used herein refers to NHEJ that is initiated after a nuclease cuts double stranded DNA.

"N-terminal truncation" as used herein means a deletion of amino acids at or near the N-terminus. In some embodiments, the deletion of amino acids may occur at the N-terminal end of the polypeptide, i.e., the deletion includes the first amino acid of the transcribed or mature protein. In some embodiments, the deletion of amino acids may occur within the N-terminal end of the polypeptide, i.e., the deletion does not include the first amino acid of the transcribed or mature protein, but occurs within the first half of the polypeptide.

The term 'non-naturally occurring' as used herein describes an entity (for example, a polynucleotide, a genetic mutation, a polypeptide, a plant, a plant cell and plant material) that is not formed by nature or that does not exist in nature. Such non-naturally occurring entities or artificial entities may be made, synthesized, initiated, modified, intervened, or manipulated by methods described herein or that are known in the art. Such non-naturally occurring entities or artificial entities may be made, synthesized, initiated, modified, intervened, or manipulated by man. Thus, by way of example, a non-naturally occurring plant, a non-naturally occurring plant cell or non-naturally occurring plant material may be made using traditional plant breeding techniques - such as backcrossing - or by genetic manipulation technologies - such as antisense RNA, interfering RNA, meganuclease and the like. By way of further example, a non-naturally occurring plant, a non-naturally occurring plant cell or non-naturally occurring plant material may be made by introgression of or by transferring one or more genetic mutations (for example one or more polymorphisms) from a first plant or plant cell into a second plant or plant cell (which may itself be naturally occurring), such that the resulting plant, plant cell or plant material or the progeny thereof comprises a genetic constitution (for example, a genome, a chromosome or a segment thereof) that is not formed by nature or that does not exist in nature. The resulting plant, plant cell or plant material is thus artificial or non-naturally occurring. Accordingly, an artificial or non-naturally occurring plant or plant cell may be made by modifying a genetic sequence in a first naturally occurring plant or plant cell, even if the resulting genetic sequence occurs naturally in a second plant or plant cell that comprises a different genetic background from the first plant or plant cell. In certain embodiments, a mutation is not a naturally occurring mutation that exists naturally in a nucleotide sequence or a polypeptide - such as a gene or a protein.

Differences in genetic background can be detected by phenotypic differences or by molecular biology techniques known in the art - such as nucleic acid sequencing, presence or absence of genetic markers (for example, microsatellite RNA markers).

"Nucleic acid" or "oligonucleotide" or "polynucleotide" as used herein means at least two nucleotides covalently linked together. The depiction of a single strand also defines the sequence of the complementary strand. Thus, a nucleic acid also encompasses the complementary strand of a depicted single strand. Many variants of a nucleic acid may be used for the same purpose as a given nucleic acid. Thus, a nucleic acid also encompasses substantially identical nucleic acids and complements thereof. A single strand provides a probe that may hybridize to a target sequence under stringent hybridization conditions. Thus, a nucleic acid also encompasses a probe that hybridizes under stringent hybridization conditions.

Nucleic acids may be single stranded or double stranded, or may contain portions of both double stranded and single stranded sequence. The nucleic acid may be DNA, both genomic and cDNA, RNA, or a hybrid, where the nucleic acid may contain combinations of deoxyribo- and ribonucleotides, and combinations of bases including uracil, adenine, thymine, cytosine, guanine, inosine, xanthine hypoxanthine, isocytosine and isoguanine. Nucleic acids may be obtained by chemical synthesis methods or by recombinant methods.

The specificity of single-stranded DNA to hybridize complementary fragments is determined by the "stringency" of the reaction conditions (Sambrook et al., Molecular Cloning and Laboratory Manual, Second Ed., Cold Spring Harbor (1989)). Hybridization stringency increases as the propensity to form DNA duplexes decreases. In nucleic acid hybridization reactions, the stringency can be chosen to favor specific hybridizations (high stringency), which can be used to identify, for example, full-length clones from a library. Less-specific hybridizations (low stringency) can be used to identify related, but not exact (homologous, but not identical), DNA molecules or segments.

DNA duplexes are stabilized by: (1) the number of complementary base pairs; (2) the type of base pairs; (3) salt concentration (ionic strength) of the reaction mixture; (4) the temperature of the reaction; and (5) the presence of certain organic solvents, such as formamide, which decrease DNA duplex stability. In general, the longer the probe, the higher the temperature required for proper annealing. A common approach is to vary the temperature; higher relative temperatures result in more stringent reaction conditions.

To hybridize under "stringent conditions" describes hybridization protocols in which nucleotide sequences at least 60% homologous to each other remain hybridized. Generally, stringent conditions are selected to be about 5°C lower than the thermal melting point (Tm) for the specific sequence at a defined ionic strength and pH. The Tm is the temperature (under defined ionic strength, pH, and nucleic acid concentration) at which 50% of the probes complementary to the target sequence hybridize to the target sequence at equilibrium. Since the target sequences are generally present at excess, at Tm, 50% of the probes are occupied at equilibrium.

"Stringent hybridization conditions" are conditions that enable a probe, primer, or oligonucleotide to hybridize only to its target sequence (e.g., SEQ ID NOs: 1, 3, 5, or 7). Stringent conditions are sequence-dependent and will differ. Stringent conditions comprise: (1) low ionic strength and high temperature washes, for example 15 mM sodium chloride, 1.5 mM sodium citrate, 0.1% sodium dodecyl sulfate, at 50°C; (2) a denaturing agent during hybridization, e.g. 50% (v/v) formamide, 0.1% bovine serum albumin, 0.1% Ficoll, 0.1% polyvinylpyrrolidone, 50 mM sodium phosphate buffer (750 mM sodium chloride, 75 mM sodium citrate; pH 6.5), at 42°C; or (3) 50% formamide. Washes typically also comprise 5xSSC (0.75 M NaCl, 75 mM sodium citrate), 50 mM sodium phosphate (pH 6.8), 0.1% sodium pyrophosphate, 5xDenhardt's solution, sonicated salmon sperm DNA (50 µg/mL), 0.1% SDS, and 10% dextran sulfate at 42°C, with a wash at 42°C in 0.2xSSC (sodium chloride/sodium citrate) and 50% formamide at 55°C, followed by a high-stringency wash consisting of 0.1xSSC containing EDTA at 55°C. Preferably, the conditions are such that sequences at least about 65%, 70%, 75%, 85%, 90%, 95%, 98%, or 99% homologous to each other typically remain hybridized to each other. These conditions are presented as examples and are not meant to be limiting.

"Moderately stringent conditions" use washing solutions and hybridization conditions that are less stringent, such that a polynucleotide will hybridize to the entire, fragments, derivatives, or analogs of the target sequence (e.g., SEQ ID NOs: 1, 3, 5, or 7). One example comprises hybridization in 6xSSC, 5xDenhardt's solution, 0.5% SDS and 100 µg/mL denatured salmon sperm DNA at 55°C, followed by one or more washes in 1xSSC, 0.1% SDS at 37°C. The temperature, ionic strength, etc., can be adjusted to accommodate experimental factors such as probe length. Other moderate stringency conditions have been described (Ausubel et al., Current Protocols in Molecular Biology, Volumes 1-3, John Wiley & Sons, Inc., Hoboken, N.J. (1993); Kriegler, Gene Transfer and Expression: A Laboratory Manual, Stockton Press, New York, N.Y. (1990); Perbal, A Practical Guide to Molecular Cloning, 2nd edition, John Wiley & Sons, New York, N.Y. (1988)).

"Low stringent conditions" use washing solutions and hybridization conditions that are less stringent than those for moderate stringency, such that a polynucleotide will hybridize to the entire, fragments, derivatives, or analogs of the target sequence (e.g., SEQ ID NOs: 1, 3, 5, or 7). A nonlimiting example of low stringency hybridization conditions includes hybridization in 35% formamide, 5xSSC, 50 mM Tris HCl (pH 7.5), 5 mM EDTA, 0.02% PVP, 0.02% Ficoll, 0.2% BSA, 100 µg/mL denatured salmon sperm DNA, 10% (wt/vol) dextran sulfate at 40°C, followed by one or more washes in 2xSSC, 25 mM Tris HCl (pH 7.4), 5 mM EDTA, and 0.1% SDS at 50°C. Other conditions of low stringency, such as those for cross-species hybridizations, are well-described (Ausubel et al., 1993; Kriegler, 1990).

"Operably linked" as used herein means that expression of a gene is under the control of a promoter with which it is spatially connected. A promoter may be positioned 5' (upstream) or 3' (downstream) of a gene under its control. The distance between the promoter and a gene may be approximately the same as the distance between that promoter and the gene it controls in the gene from which the promoter is derived. As is known in the art, variation in this distance may be accommodated without loss of promoter function. "Operably linked" refers to the association of nucleic acid fragments in a single fragment so that the function of one is regulated by the other. For example, a promoter is operably linked with a nucleic acid fragment when it is capable of regulating the transcription of that nucleic acid fragment.

The term "plant" as used herein refers to any plant at any stage of its life cycle or development, and its progenies. In one embodiment, the plant is a tobacco plant, which refers to a plant belonging to the genus Nicotiana. "Plant" includes reference to whole plants, plant organs, plant tissues, plant propagules, seeds and plant cells and progeny of same. Plant cells include, without limitation, cells from seeds, suspension cultures, embryos, meristematic regions, callus tissue, leaves, roots, shoots, gametophytes, sporophytes, pollen, and microspores. Preferred species, cultivars, hybrids and varieties of tobacco plant are described herein.

"Polynucleotide", "nucleic acid sequence", "nucleotide sequence", or "nucleic acid fragment" are used interchangeably herein to refer to a polymer of RNA or DNA that is single- or double-stranded, optionally containing synthetic, non-natural or altered nucleotide bases. Nucleotides (usually found in their 5'-monophosphate form) are referred to by their single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide. A polynucleotide can be, without limitation, a genomic DNA, complementary DNA (cDNA), mRNA, or antisense RNA or a fragment(s) thereof. Moreover, a polynucleotide can be single-stranded or double-stranded, nucleic acid that is a mixture of single-stranded and double-stranded regions, a hybrid molecule comprising DNA and RNA, or a hybrid molecule with a mixture of single-stranded and double-stranded regions or a fragment(s) thereof. In addition, the polynucleotide can be composed of triple-stranded regions comprising DNA, RNA, or both or a fragment(s) thereof. A polynucleotide can contain one or more modified bases, such as phosphothioates, and can be a peptide nucleic acid (PNA). Generally, polynucleotides can be assembled from isolated or cloned fragments of cDNA, genomic DNA, oligonucleotides, or individual nucleotides, or a combination of the foregoing. Although the polynucleotide sequences described herein are shown as DNA sequences, the sequences include their corresponding RNA sequences, and their complementary (for example, completely complementary) DNA or RNA sequences, including the reverse complements thereof.

"Polypeptide", "peptide", "amino acid sequence" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers. The terms "polypeptide", "peptide", "amino acid sequence", and "protein" are also inclusive of modifications including, but not limited to, glycosylation, lipid attachment, sulfation, gamma-carboxylation of glutamic acid residues, hydroxylation and ADP-ribosylation.

"Promoter" as used herein means a synthetic or naturally-derived molecule which is capable of conferring, activating or enhancing expression of a nucleic acid in a cell. A "promoter" refers to a nucleic acid element/sequence, typically positioned upstream and operably-linked to a double-stranded nucleic acid fragment. Promoters can be derived entirely from regions proximate to a native gene of interest, or can be composed of different elements derived from different native promoters or synthetic nucleic acid segments. A promoter may comprise one or more specific transcriptional regulatory sequences to further enhance expression and/or to alter the spatial expression and/or temporal expression of same. A promoter may also comprise distal enhancer or repressor elements, which may be located as much as several thousand base pairs from the start site of transcription. A promoter may be derived from sources including viral, bacterial, fungal, plants, insects, and animals. A promoter may regulate the expression of a gene component constitutively or differentially with respect to cell, the tissue or organ in which expression occurs or, with respect to the developmental stage at which expression occurs, or in response to external stimuli such as physiological stresses, pathogens, metal ions, or inducing agents. "Tissue-specific promoter" and "tissue-preferred promoter" as used interchangeably herein refer to a promoter that is expressed predominantly but not necessarily exclusively in one tissue or organ, but that may also be expressed in one specific cell. "Developmentally regulated promoter" as used herein refers to a promoter whose activity is determined by developmental events. Promoters that cause a gene to be expressed in most cell types at most times are commonly referred to as "constitutive promoters". Inducible promoters selectively express an operably linked DNA sequence in response to the presence of an endogenous or exogenous stimulus, for example by chemical compounds (chemical inducers) or in response to environmental, hormonal, chemical, and/or developmental signals. Examples of inducible or regulated promoters include, but are not limited to, promoters regulated by light, heat, stress, flooding or drought, pathogens, phytohormones, wounding, or chemicals such as ethanol, jasmonate, salicylic acid, or safeners.

"Recombinant" as used herein refers to an artificial combination of two otherwise separated segments of sequence, e.g., by chemical synthesis or by the manipulation of isolated segments of nucleic acids by genetic engineering techniques. "Recombinant" also includes reference to a cell or vector, that has been modified by the introduction of a heterologous nucleic acid or a cell derived from a cell so modified, but does not encompass the alteration of the cell or vector by naturally occurring events (e.g., spontaneous mutation, natural transformation/transduction/transposition) such as those occurring without deliberate human intervention.

"Recombinant DNA construct" as used herein refers to a combination of nucleic acid fragments that are not normally found together in nature. Accordingly, a recombinant DNA construct may comprise regulatory sequences and coding sequences that are derived from different sources, or regulatory sequences and coding sequences derived from the same source, but arranged in a manner different than that normally found in nature. The terms "recombinant DNA construct" and "recombinant construct" are used interchangeably herein.

The term "reduce" or "reduced" as used herein, refers to a reduction of from about 10% to about 99%, or a reduction of at least 10%, at least 20%, at least 25%, at least 30%, at least 40%, at least 50%, at least 60%, at least 70%, at least 75%, at least 80%, at least 90%, at least 95%, at least 98%, at least 99%, or at least 100% or more of a quantity or an activity, such as but not limited to nitrate levels and tobacco-specific nitrosamines (TSNA) levels. The term "reduced," or the phrase "a reduced amount" is intended to refer to an amount TSNA in a modified tobacco plant or a tobacco product generated from the modified tobacco plant that is less than what would be found in a tobacco plant or a tobacco product from the same variety of tobacco processed in the same manner, which has not been modified. Thus, in some contexts, wild-type tobacco of the same variety that has been processed in the same manner is used as a control by which to measure whether a reduction in nitrate or TSNA has been obtained by the inventive methods described herein.

"Regulatory sequences" and "regulatory elements" as used interchangeably herein refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to, promoters, translation leader sequences, introns, and polyadenylation recognition sequences. The terms "regulatory sequence" and "regulatory element" are used interchangeably herein.

"Site-specific nuclease" as used herein refers to an enzyme capable of specifically recognizing and cleaving DNA sequences. The site-specific nuclease may be engineered. Examples of engineered site-specific nucleases include zinc finger nucleases (ZFNs), TAL effector nucleases (TALENs), CRISPR/Cas9-based systems, and meganucleases.

The term "tobacco", in some contexts, as used herein is used in a collective sense to refer to tobacco crops, (e.g., a plurality of tobacco plants grown in the field, i.e., not hydroponically grown tobacco) tobacco plants and parts thereof, including but not limited to, roots, stems, leaves, flowers, and seeds prepared and/or obtained, as described herein. It is understood that "tobacco" refers to *Nicotiana tabacum* plants and products thereof. The term "tobacco products" in some contexts refers to consumer tobacco products, including but not limited to, smoking materials (e.g., cigarettes, cigars, and pipe tobacco), snuff, chewing tobacco, gum, and lozenges, as well as components, materials and ingredients for manufacture of consumer tobacco products. Preferably these tobacco products are manufactured from tobacco (*Nicotiana tabacum*) leaves and stems harvested from the tobacco treated as described above and cut, dried, cured, and/or fermented according to conventional techniques in tobacco preparation.

"Transcription terminator", "termination sequences", or "terminator" as used herein refer to DNA sequences located downstream of a coding sequence, including polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor.

"Transgenic" as used herein refers to any cell, cell line, callus, tissue, plant part or plant, the genome of which has been altered by the presence of a heterologous nucleic acid, such as a recombinant DNA construct, including those initial transgenic events as well as those created by sexual crosses or asexual propagation from the initial transgenic event. The term "transgenic" as used herein does not encompass the alteration of the genome (chromosomal or extra-chromosomal) by conventional plant breeding methods or by naturally occurring events such as random cross-fertilization, non-recombinant viral infection, non-recombinant bacterial transformation, non-recombinant transposition, or spontaneous mutation.

"Transgenic plant" as used herein includes reference to a plant which comprises within its genome a heterologous polynucleotide, i.e., a plant or tree that contains recombinant genetic material not normally found in plants or trees of this type and which has been introduced into the plant in question (or into progenitors of the plant) by human manipulation. For example, the heterologous polynucleotide is stably integrated within the genome such that the polynucleotide is passed on to successive generations. The heterologous polynucleotide may be integrated into the genome alone or as part of a recombinant DNA construct. The commercial development of genetically improved germplasm has also advanced to the stage of introducing multiple traits into crop plants, often referred to as a gene stacking approach. In this approach, multiple genes conferring different characteristics of interest can be introduced into a plant. Gene stacking can be accomplished by many means including but not limited to co-transformation, retransformation, and crossing lines with different transgenes. Thus, a plant that is grown from a plant cell into which recombinant DNA is introduced by transformation is a transgenic plant, as are all offspring of that plant that contain the introduced transgene (whether produced sexually or asexually). It is understood that the term transgenic plant encompasses the entire plant or tree and parts of the plant or tree, for instance grains, seeds, flowers, leaves, roots, fruit, pollen, stems etc.

"Transgenic plant" also includes reference to plants which comprise more than one heterologous polynucleotide within their genome. Each heterologous polynucleotide may confer a different trait to the transgenic plant.

"Transcription activator-like effector" or "TALE" as used herein refers to a protein structure that recognizes and binds to a particular DNA sequence. The "TALE DNA-binding domain" refers to a DNA-binding domain that includes an array of tandem 33-35 amino acid repeats, also known as RVD modules, each of which specifically recognizes a single base pair of DNA. RVD modules may be arranged in any order to assemble an array that recognizes a defined sequence.

A binding specificity of a TALE DNA-binding domain is determined by the RVD array followed by a single truncated repeat of 20 amino acids. A TALE DNA-binding domain may have 12 to 27 RVD modules, each of which contains an RVD and recognizes a single base pair of DNA. Specific RVDs have been identified that recognize each of the four possible DNA nucleotides (A, T, C, and G). Because the TALE DNA-binding domains are modular, repeats that recognize the four different DNA nucleotides may be linked together to recognize any particular DNA sequence. These targeted DNA-binding domains may then be combined with catalytic domains to create functional enzymes, including artificial transcription factors, methyltransferases, integrases, nucleases, and recombinases.

"Transcription activator-like effector nucleases" or "TALENs" as used interchangeably herein refers to engineered fusion proteins of the catalytic domain of a nuclease, such as endonuclease *Fok*I, and a designed TALE DNA-binding domain that may be targeted to a custom DNA sequence. A "TALEN monomer" refers to an engineered fusion protein with a catalytic nuclease domain and a designed TALE DNA-binding domain. Two TALEN monomers may be designed to target and cleave a TALEN target region.

"Transgene" as used herein refers to a gene or genetic material containing a gene sequence that has been isolated from one organism and is introduced into a different organism. This non-native segment of DNA may retain the ability to produce RNA or protein in the transgenic organism, or it may alter the normal function of the transgenic organism's genetic code. The introduction of a transgene has the potential to change the phenotype of an organism.

"Variant" used herein with respect to a nucleic acid means (i) a portion or fragment of a referenced nucleotide sequence; (ii) the complement of a referenced nucleotide sequence or portion thereof; (iii) a nucleic acid that is substantially identical to a referenced nucleic acid or the complement thereof; or (iv) a nucleic acid that hybridizes under stringent conditions to the referenced nucleic acid, complement thereof, or a sequences substantially identical thereto.

"Variant" with respect to a peptide or polypeptide that differs in amino acid sequence by the insertion, deletion, or conservative substitution of amino acids, but retain at least one biological activity. Variant may also mean a protein with an amino acid sequence that is substantially identical to a referenced protein with an amino acid sequence that retains at least one biological activity. A conservative substitution of an amino acid, *i.e.*, replacing an amino acid with a different amino acid of similar properties (*e.g.*, hydrophilicity, degree and distribution of charged regions) is recognized in the art as typically involving a minor change.

The term "variety" as used herein refers to a population of plants that share constant characteristics which separate them from other plants of the same species. While possessing one or more distinctive traits, a variety is further characterized by a very small overall variation between individuals within that variety. A variety is often sold commercially.

"Vector" as used herein means refers to a nucleic acid vehicle that comprises a combination of nucleic acid components for enabling the transport of nucleic acid, nucleic acid constructs and nucleic acid conjugates and the like. A vector may be a viral vector, bacteriophage, bacterial artificial chromosome or yeast artificial chromosome. A vector may be a DNA or RNA vector. Suitable vectors include episomes capable of extra-chromosomal replication such as circular, double-stranded nucleic acid plasmids; linearized double-stranded nucleic acid plasmids; and other vectors of any origin. For example, the vector may encode an ABCG1 polypeptide comprising the amino acid sequence of any one of SEQ ID NOs: 2 or 3. An "expression vector" as used herein is a nucleic acid vehicle that comprises a combination of nucleic acid components for enabling the expression of nucleic acid, nucleic acid constructs and nucleic acid conjugates and the like. Suitable expression vectors include episomes capable of extra-chromosomal replication such as circular, double-stranded nucleic acid plasmids; linearized double-stranded nucleic acid plasmids; and other functionally equivalent expression vectors of any origin. An expression vector comprises at least a promoter positioned upstream and operably-linked to a nucleic acid, nucleic acid constructs or nucleic acid conjugate, as defined below.

"Zinc finger" as used herein refers to a protein structure that recognizes and binds to DNA sequences. The zinc finger domain is the most common DNA-binding motif in the human proteome. A single zinc finger contains approximately 30 amino acids and the domain typically functions by binding 3 consecutive base pairs of DNA via interactions of a single amino acid side chain per base pair.

"Zinc finger nuclease" or "ZFN" as used interchangeably herein refers to a chimeric protein molecule comprising at least one zinc finger DNA binding domain effectively linked to at least one nuclease or part of a nuclease capable of cleaving DNA when fully assembled.

Unless otherwise defined herein, scientific and technical terms used in connection with the present disclosure shall have the meanings that are commonly understood by those of ordinary skill in the art. For example, any nomenclatures used in connection with, and techniques of, cell and tissue culture, molecular biology, immunology, microbiology, genetics and protein and nucleic acid chemistry and hybridization described herein are those that are well known and commonly used in the art. The meaning and scope of the terms should be clear; in the event however of any latent ambiguity, definitions provided herein take precedent over any dictionary or extrinsic definition. Further, unless otherwise required by context, singular terms shall include pluralities and plural terms shall include the singular.

### 2. Polynucleotides

In one embodiment, there is provided an isolated polynucleotide comprising, consisting or consisting essentially of a polynucleotide sequence having at least 60% sequence identity to any of the sequences described herein, including any of polynucleotides shown in the sequence lisiting. Suitably, the isolated polynucleotide comprises, consists or consists essentially of a sequence having at least 60%, 61%, 62%, 63%, 64%, 65%, 66%, 67%, 68%, 69%, 70%, 75%, 80%, 85%, 87%, 88%, 89%, 90%, 91%, 92%, 93%, 94%, 95% 96%, 97%, 98%, 99% or 100% sequence identity thereto.

Suitably, the polynucleotide(s) described herein encode a protein with transporter activity that is at least about 50%, 60%, 70%, 80%, 90% 95%, 96%, 97%, 98%, 99% 100% or more of the activity of the protein set forth in SEQ ID NOs: 2 or 4. In another embodiment, there is provided an isolated polynucleotide comprising, consisting or consisting essentially of a polynucleotide sequence having at least 60% sequence identity to SEQ ID NO.1, SEQ ID NO.3, or SEQ ID NO. 5. Suitably, the isolated polynucleotide comprises, consists or consist essentially of a sequence having at least about 60%, 61%, 62%, 63%, 64%, 65%, 66%, 67%, 68%, 69%, 70%, 75%, 80%, 85%, 87%, 88%, 89%, 90%, 91%, 92%, 93%, 94%, 95% 96%, 97%, 98%, 99%, 99.1%, 99.2%, 99.3%, 99.4%, 99.5%, 99.6%, 99.7%, 99.8%, 99.9% or 100% sequence identity to SEQ ID NO.1, SEQ ID NO.3, or SEQ ID NO. 5.

In another embodiment, there is provided polynucleotides comprising, consisting or consisting essentially of polynucleotides with substantial homology (that is, sequence similarity) or substantial identity to SEQ ID NO.1, SEQ ID NO.3, or SEQ ID NO. 5.

In another embodiment, there is provided fragments of SEQ ID NO.1, SEQ ID NO.3, or SEQ ID NO. 5, and fragments of SEQ ID NO.1, SEQ ID NO.3, or SEQ ID NO.5 with substantial homology (that is, sequence similarity) or substantial identity thereto that have at least about 60%, 61%, 62%, 63%, 64%, 65%, 66%, 67%, 68%, 69%, 70%, 75%, 80%, 85%, 87%, 88%, 89%, 90%, 91%, 92%, 93%, 94%, 95% 96%, 97%, 98%, 99%, 99.1%, 99.2%, 99.3%, 99.4%, 99.5%, 99.6%, 99.7%, 99.8%, 99.9% or 100% sequence identity to the corresponding fragments of SEQ ID NO.1, SEQ ID NO.3, or SEQ ID NO.5.

In another embodiment, there is provided polynucleotides comprising a sufficient or substantial degree of identity or similarity to SEQ ID NO.1, SEQ ID NO.3 that encode a polypeptide that functions as an ABC G1 transporter.

In another embodiment, there is provided a polymer of polynucleotides which comprises, consists or consists essentially of a polynucleotide designated herein as SEQ ID NO.1, SEQ ID NO:2, SEQ ID NO.5.

Suitably, the polynucleotides described herein encode members of the ABC-2 family of ABCG1 transporters.

A polynucleotide as described herein can include a polymer of nucleotides, which may be unmodified or modified deoxyribonucleic acid (DNA) or ribonucleic acid (RNA). Accordingly, a polynucleotide can be, without limitation, a genomic DNA, complementary DNA (cDNA), mRNA, or antisense RNA or a fragment(s) thereof. Moreover, a polynucleotide can be single-stranded or double-stranded DNA, DNA that is a mixture of single-stranded and double-stranded regions, a hybrid molecule comprising DNA and RNA, or a hybrid molecule with a mixture of single-stranded and double-stranded regions or a fragment(s) thereof. In addition, the polynucleotide can be composed of triple-stranded regions comprising DNA, RNA, or both or a fragment(s) thereof. A polynucleotide can contain one or more modified bases, such as phosphothioates, and can be a peptide nucleic acid. Generally, polynucleotides can be assembled from isolated or cloned fragments of cDNA, genomic DNA, oligonucleotides, or individual nucleotides, or a combination of the foregoing. Although the polynucleotide sequences described herein are shown as DNA sequences, the sequences include their corresponding RNA sequences, and their complementary (for example, completely complementary) DNA or RNA sequences, including the reverse complements thereof.

A polynucleotide as described herein will generally contain phosphodiester bonds, although in some cases, polynucleotide analogues are included that may have alternate backbones, comprising, for example, phosphoramidate, phosphorothioate, phosphorodithioate, or O-methylphophoroamidite linkages; and peptide polynucleotide backbones and linkages. Other analogue polynucleotides include those with positive backbones; non-ionic backbones, and non-ribose backbones. Modifications of the ribose-phosphate backbone may be done for a variety of reasons, for example, to increase the stability and half-life of such molecules in physiological environments or as probes on a biochip. Mixtures of naturally occurring polynucleotides and analogues can be made; alternatively, mixtures of different polynucleotide analogues, and mixtures of naturally occurring polynucleotides and analogues may be made.

A variety of polynucleotide analogues are known, including, for example, phosphoramidate, phosphorothioate, phosphorodithioate, O-methylphophoroamidite linkages and peptide polynucleotide backbones and linkages. Other analogue polynucleotides include those with positive backbones, non-ionic backbones and non-ribose backbones. Polynucleotides containing one or more carbocyclic sugars are also included.

Other analogues include peptide polynucleotides which are peptide polynucleotide analogues. These backbones are substantially non-ionic under neutral conditions, in contrast to the highly charged phosphodiester backbone of naturally occurring polynucleotides. This may result in advantages. First, the peptide polynucleotide backbone may exhibit improved hybridization kinetics. Peptide polynucleotides have larger changes in the melting temperature for mismatched versus perfectly matched base pairs. DNA and RNA typically exhibit a 2-4 °C drop in melting temperature for an internal mismatch. With the non-ionic peptide polynucleotide backbone, the drop is closer to 7-9 °C. Similarly, due to their non-ionic nature, hybridization of the bases attached to these backbones is relatively insensitive to salt concentration. In addition, peptide polynucleotides may not be degraded or degraded to a lesser extent by cellular enzymes, and thus may be more stable.

Among the uses of the disclosed polynucleotides, and fragments thereof, is the use of fragments as probes in nucleic acid hybridisation assays or primers for use in nucleic acid amplification assays. Such fragments generally comprise at least about 10, 11, 12, 13, 14, 15, 16, 17, 18, 19 or 20 or more contiguous nucleotides of a DNA sequence. In other embodiments, a DNA fragment comprises at least about 10, 15, 20, 30, 40, 50 or 60 or more contiguous nucleotides of a DNA sequence. Thus, in one aspect, there is also provided a method for detecting a polynucleotide encoding an ABC transporter comprising the use of the probes or primers or both. Suitable primers include those listed herein under SEQ ID NOs.6-9

The basic parameters affecting the choice of hybridization conditions and guidance for devising suitable conditions are described by Sambrook, J., E. F. Fritsch, and T. Maniatis (1989, Molecular Cloning: A Laboratory Manual, Cold Spring Harbor Laboratory Press, Cold Spring Harbor, N.Y.). Using knowledge of the genetic code in combination with the amino acid sequences described herein, sets of degenerate oligonucleotides can be prepared. Such oligonucleotides are useful as primers, for example, in polymerase chain reactions (PCR), whereby DNA fragments are isolated and amplified. In certain embodiments, degenerate primers can be used as probes for genetic libraries. Such libraries would include but are not limited to cDNA libraries, genomic libraries, and even electronic express sequence tag or DNA libraries. Homologous sequences identified by this method would then be used as probes to identify homologues of the sequences identified herein.

Also of potential use are polynucleotides and oligonucleotides (for example, primers or probes) that hybridize under reduced stringency conditions, typically moderately stringent conditions, and commonly highly stringent conditions to the polynucleotide(s) as described herein. The basic parameters affecting the choice of hybridization conditions and guidance for devising suitable conditions are set forth by Sambrook, J., E. F. Fritsch, and T. Maniatis (1989, Molecular Cloning: A Laboratory Manual, Cold Spring Harbor Laboratory Press, Cold Spring Harbor, N.Y. and can be readily determined by those having ordinary skill in the art based on, for example, the length or base composition of the polynucleotide.

One way of achieving moderately stringent conditions involves the use of a prewashing solution containing 5x Standard Sodium Citrate, 0.5% Sodium Dodecyl Sulphate, 1.0 mM Ethylenediaminetetraacetic acid (pH 8.0), hybridization buffer of about 50% formamide, 6x Standard Sodium Citrate, and a hybridization temperature of about 55 °C (or other similar hybridization solutions, such as one containing about 50% formamide, with a hybridization temperature of about 42°C), and washing conditions of about 60°C, in 0.5x Standard Sodium Citrate, 0.1% Sodium Dodecyl Sulphate. Generally, highly stringent conditions are defined as hybridization conditions as above, but with washing at approximately 68 °C, 0.2x Standard Sodium Citrate, 0.1% Sodium Dodecyl Sulphate. SSPE (1 x SSPE is 0.15 M sodium chloride, 10 mM sodium phosphate, and 1.25 mM Ethylenediaminetetraacetic acid, pH 7.4) can be substituted for Standard Sodium Citrate (1 x Standard Sodium Citrate is 0.15 M sodium chloride and 15 mM sodium citrate) in the hybridization and wash buffers; washes are performed for 15 minutes after hybridization is complete. It should be understood that the wash temperature and wash salt concentration can be adjusted as necessary to achieve a desired degree of stringency by applying the basic principles that govern hybridization reactions and duplex stability, as known to those skilled in the art and described further below (see, for example, Sambrook, J., E. F. Fritsch, and T. Maniatis (1989, Molecular Cloning: A Laboratory Manual, Cold Spring Harbor Laboratory Press, Cold Spring Harbor, N.Y). When hybridizing a polynucleotide to a target polynucleotide of unknown sequence, the hybrid length is assumed to be that of the hybridizing polynucleotide. When polynucleotides of known sequence are hybridized, the hybrid length can be determined by aligning the sequences of the polynucleotides and identifying the region or regions of optimal sequence complementarity. The hybridization temperature for hybrids anticipated to be less than 50 base pairs in length should be 5 to 10 °C less than the melting temperature of the hybrid, where melting temperature is determined according to the following equations. For hybrids less than 18 base pairs in length, melting temperature (°C)=2(number of A+T bases)+4(number of G+C bases). For hybrids above 18 base pairs in length, melting temperature (°C)=81.5+16.6(log10 [Na+])+0.41(% G+C)-(600/N), where N is the number of bases in the hybrid, and [Na+] is the concentration of sodium ions in the hybridization buffer ([Na+] for 1x Standard Sodium Citrate=0.165M). Typically, each such hybridizing polynucleotide has a length that is at least 25% (commonly at least 50%, 60%, or 70%, and most commonly at least 80%) of the length of a polynucleotide to which it hybridizes, and has at least 60% sequence identity (for example, at least 70%, 75%, 80%, 85%, 90%, 95%, 96%, 97%, 98%, 99% or 100%) with a polynucleotide to which it hybridizes.

As will be understood by the person skilled in the art, a linear DNA has two possible orientations: the 5'-to-3' direction and the 3'-to-5' direction. For example, if a reference sequence is positioned in the 5'-to-3' direction, and if a second sequence is positioned in the 5'-to-3' direction within the same polynucleotide molecule/strand, then the reference sequence and the second sequence are orientated in the same direction, or have the same orientation. Typically, a promoter sequence and a gene of interest under the regulation of the given promoter are positioned in the same orientation. However, with respect to the reference sequence positioned in the 5'-to-3' direction, if a second sequence is positioned in the 3'-to-5' direction within the same polynucleotide molecule/strand, then the reference sequence and the second sequence are orientated in anti-sense direction, or have anti-sense orientation. Two sequences having anti-sense orientations with respect to each other can be alternatively described as having the same orientation, if the reference sequence (5'-to-3' direction) and the reverse complementary sequence of the reference sequence (reference sequence positioned in the 5'-to-3') are positioned within the same polynucleotide molecule/strand. The sequences set forth herein are shown in the 5'-to-3' direction.

### 3. Polypeptide

In another aspect, there is provided an isolated polypeptide comprising, consisting or consisting essentially of a polypeptide sequence having at least 60% sequence identity to any of the polypeptide sequences described herein, including any of the polypeptides shown in the sequence lisiting. Suitably, the isolated polypeptide comprises, consists or consists essentially of a sequence having at least 60%, 61%, 62%, 63%, 64%, 65%, 66%, 67%, 68%, 69%, 70%, 75%, 80%, 85%, 87%, 88%, 89%, 90%, 91%, 92%, 93%, 94%, 95% 96%, 97%, 98%, 99%, 99.1%, 99.2%, 99.3%, 99.4%, 99.5%, 99.6%, 99.7%, 99.8%, 99.9% or 100% sequence identity thereto. In one embodiment, there is provided a polypeptide encoded by SEQ ID NO: 1 or SEQ ID NO: 3.

In another emboidment, there is provided an isolated polypeptide comprising, consisting or consisting essentially of a sequence having at least 60%, 61%, 62%, 63%, 64%, 65%, 66%, 67%, 68%, 69%, 70%, 75%, 80%, 85%, 87%, 88%, 89%, 90%, 91%, 92%, 93%, 94%, 95% 96%, 97%, 98%, 99%, 99.1%, 99.2%, 99.3%, 99.4%, 99.5%, 99.6%, 99.7%, 99.8%, 99.9% or 100% sequence identity to SEQ ID NO.2 or SEQ ID NO.4.

The polypeptide can include sequences comprising a sufficient or substantial degree of identity or similarity to SEQ ID NOs: 2 or 4 to function as ABC transporters. The fragments of the polypeptide(s) typically retain some or all of the activity of the full length sequence.

As discussed herein, the polypeptides also include mutants produced by introducing any type of alterations (for example, insertions, deletions, or substitutions of amino acids; changes in glycosylation states; changes that affect refolding or isomerizations, three-dimensional structures, or self-association states), which can be deliberately engineered or isolated naturally provided that they still have some or all of their function or activity as an ABC transporter. Suitably, the function or activity as an ABC transporter is modulated, increased or reduced.

A deletion refers to removal of one or more amino acids from a protein. An insertion refers to one or more amino acid residues being introduced into a predetermined site in a polypeptide. Insertions may comprise intra-sequence insertions of single or multiple amino acids. A substitution refers to the replacement of amino acids of the polypeptide with other amino acids having similar properties (such as similar hydrophobicity, hydrophilicity, antigenicity, propensity to form or break a-helical structures or β-sheet structures). Amino acid substitutions are typically of single residues, but may be clustered depending upon functional constraints placed upon the polypeptide and may range from about 1 to about 10 amino acids. The amino acid substitutions are preferably conservative amino acid substitutions as described below. Amino acid substitutions, deletions and/or insertions can be made using peptide synthetic techniques - such as solid phase peptide synthesis or by recombinant DNA manipulation. Methods for the manipulation of DNA sequences to produce substitution, insertion or deletion variants of a protein are well known in the art. The variant may have alterations which produce a silent change and result in a functionally equivalent protein. Deliberate amino acid substitutions may be made on the basis of similarity in polarity, charge, solubility, hydrophobicity, hydrophilicity and the amphipathic nature of the residues as long as the secondary binding activity of the substance is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine. Conservative substitutions may be made, for example according to the Table below. Amino acids in the same block in the second column and preferably in the same line in the third column may be substituted for each other:

| | | |
|---|---|---|
| ALIPHATIC | Non-polar | Gly Ala Pro |
| | | Ile Leu Val |
| | Polar - uncharged | Cys Ser Thr Met |
| | | Asn Gly |
| | Polar - charged | Asp Glu |
| | | Lys Arg |
| AROMA TIC | | His Phe TrpTyr |

The polypeptide may be a mature protein or an immature protein or a protein derived from an immature protein. Polypeptides may be in linear form or cyclized using known methods. Polypeptides typically comprise at least 10, at least 20, at least 30, or at least 40 contiguous amino acids.

### 4. Modifying plants

### a. Transformation

Recombinant constructs provided herein can be used to transform plants or plant cells in order to modulate protein expression and/or activity levels. A recombinant polynucleotide construct can comprise a polynucleotide encoding one or more polynucleotides as described herein, operably linked to a regulatory region suitable for expressing the polypeptide. Thus, a polynucleotide can comprise a coding sequence that encodes the polypeptide as described herein. Plants or plant cells in which protein expression and/or activity levels are modulated can include mutant, non-naturally occurring, transgenic, man-made or genetically engineered plants or plant cells. Suitably, the transgenic plant or plant cell comprises a genome that has been altered by the stable integration of recombinant DNA. Recombinant DNA includes DNA which has been genetically engineered and constructed outside of a cell and includes DNA containing naturally occurring DNA or cDNA or synthetic DNA. A transgenic plant can include a plant regenerated from an originally-transformed plant cell and progeny transgenic plants from later generations or crosses of a transformed plant. Suitably, the transgenic modification alters the expression or activity of the polynucleotide or the polypeptide described herein as compared to a control plant.

The polypeptide encoded by a recombinant polynucleotide can be a native polypeptide, or can be heterologous to the cell. In some cases, the recombinant construct contains a polynucleotide that modulates expression, operably linked to a regulatory region. Examples of suitable regulatory regions are described herein.

Vectors containing recombinant polynucleotide constructs such as those described herein are also provided. Suitable vector backbones include, for example, those routinely used in the art such as plasmids, viruses, artificial chromosomes, bacterial artificial chromosomes, yeast artificial chromosomes, or bacteriophage artificial chromosomes. Suitable expression vectors include, without limitation, plasmids and viral vectors derived from, for example, bacteriophage, baculoviruses, and retroviruses. Numerous vectors and expression systems are commercially available.

The vectors can include, for example, origins of replication, scaffold attachment regions or markers. A marker gene can confer a selectable phenotype on a plant cell. For example, a marker can confer biocide resistance, such as resistance to an antibiotic (for example, kanamycin, G418, bleomycin, or hygromycin), or an herbicide (for example, glyphosate, chlorsulfuron or phosphinothricin). In addition, an expression vector can include a tag sequence designed to facilitate manipulation or detection (for example, purification or localization) of the expressed polypeptide. Tag sequences, such as luciferase, beta-glucuronidase, green fluorescent protein, glutathione S-transferase, polyhistidine, c-myc or hemagglutinin sequences typically are expressed as a fusion with the encoded polypeptide. Such tags can be inserted anywhere within the polypeptide, including at either the carboxyl or amino terminus.

A plant or plant cell can be transformed by having the recombinant polynucleotide integrated into its genome to become stably transformed. The plant or plant cell described herein can be stably transformed. Stably transformed cells typically retain the introduced polynucleotide with each cell division. A plant or plant cell can be transiently transformed such that the recombinant polynucleotide is not integrated into its genome. Transiently transformed cells typically lose all or some portion of the introduced recombinant polynucleotide with each cell division such that the introduced recombinant polynucleotide cannot be detected in daughter cells after a sufficient number of cell divisions.

A number of methods are available in the art for transforming a plant cell which are all encompassed herein, including biolistics, gene gun techniques, Agrobacterium-mediated transformation, viral vector-mediated transformation, freeze-thaw method, microparticle bombardment, direct DNA uptake, sonication, microinjection, plant virus-mediated transfer, and electroporation. The Agrobacterium system for integration of foreign DNA into plant chromosomes has been extensively studied, modified, and exploited for plant genetic engineering. Naked recombinant DNA molecules comprising DNA sequences corresponding to the subject purified tobacco protein operably linked, in the sense or antisense orientation, to regulatory sequences are joined to appropriate T-DNA sequences by conventional methods. These are introduced into tobacco protoplasts by polyethylene glycol techniques or by electroporation techniques, both of which are standard. Alternatively, such vectors comprising recombinant DNA molecules encoding the subject purified tobacco protein are introduced into live Agrobacterium cells, which then transfer the DNA into the plant cells. Transformation by naked DNA without accompanying T-DNA vector sequences can be accomplished via fusion of tobacco protoplasts with DNA-containing liposomes or via electroporation. Naked DNA unaccompanied by T-DNA vector sequences can also be used to transform tobacco cells via inert, high velocity microprojectiles.

If a cell or cultured tissue is used as the recipient tissue for transformation, plants can be regenerated from transformed cultures if desired, by techniques known to those skilled in the art. The choice of regulatory regions to be included in a recombinant construct depends upon several factors, including, but not limited to, efficiency, selectability, inducibility, desired expression level, and cell- or tissue-preferential expression. It is a routine matter for one of skill in the art to modulate the expression of a coding sequence by appropriately selecting and positioning regulatory regions relative to the coding sequence. Transcription of a polynucleotide can be modulated in a similar manner. Some suitable regulatory regions initiate transcription only, or predominantly, in certain cell types. Methods for identifying and characterizing regulatory regions in plant genomic DNA are known in the art.

Suitable promoters include tissue-specific promoters recognized by tissue-specific factors present in different tissues or cell types (for example, root-specific promoters, shoot-specific promoters, xylem-specific promoters), or present during different developmental stages, or present in response to different environmental conditions. Suitable promoters include constitutive promoters that can be activated in most cell types without requiring specific inducers. Examples of suitable promoters for controlling RNAi polypeptide production include the cauliflower mosaic virus 35S (CaMV/35S), SSU, OCS, lib4, usp, STLS1, B33, nos or ubiquitin- or phaseolin-promoters. Persons skilled in the art are capable of generating multiple variations of recombinant promoters.

Tissue-specific promoters are transcriptional control elements that are only active in particular cells or tissues at specific times during plant development, such as in vegetative tissues or reproductive tissues. Tissue-specific expression can be advantageous, for example, when the expression of polynucleotides in certain tissues is preferred. Examples of tissue-specific promoters under developmental control include promoters that can initiate transcription only (or primarily only) in certain tissues, such as vegetative tissues, for example, roots or leaves, or reproductive tissues, such as fruit, ovules, seeds, pollen, pistols, flowers, or any embryonic tissue. Reproductive tissue-specific promoters may be, for example, anther-specific, ovule-specific, embryo-specific, endosperm-specific, integument-specific, seed and seed coat-specific, pollen-specific, petal-specific, sepal-specific, or combinations thereof.

Suitable leaf-specific promoters include pyruvate, orthophosphate dikinase (PPDK) promoter from C4 plant (maize), cab-m1Ca+2 promoter from maize, the Arabidopsis thaliana myb-related gene promoter (Atmyb5), the ribulose biphosphate carboxylase (RBCS) promoters (for example, the tomato RBCS 1, RBCS2 and RBCS3A genes expressed in leaves and light-grown seedlings, RBCS1 and RBCS2 expressed in developing tomato fruits or ribulose bisphosphate carboxylase promoter expressed almost exclusively in mesophyll cells in leaf blades and leaf sheaths at high levels).

Suitable senescence-specific promoters include a tomato promoter active during fruit ripening, senescence and abscission of leaves, a maize promoter of gene encoding a cysteine protease, the promoter of 82E4 and the promoter of SAG genes. Suitable anther-specific promoters can be used. Suitable root-preferred promoters known to persons skilled in the art may be selected. Suitable seed-preferred promoters include both seed-specific promoters (those promoters active during seed development such as promoters of seed storage proteins) and seed-germinating promoters (those promoters active during seed germination). Such seed-preferred promoters include, but are not limited to, Cim1 (cytokinin-induced message); cZ19B1 (maize 19 kDa zein); milps (myo-inositol-1-phosphate synthase); mZE40-2, also known as Zm-40; nuclc; and celA (cellulose synthase). Gama-zein is an endosperm-specific promoter. Glob-1 is an embryo-specific promoter. For dicots, seed-specific promoters include, but are not limited to, bean beta-phaseolin, napin, β-conglycinin, soybean lectin, cruciferin, and the like. For monocots, seed-specific promoters include, but are not limited to, a maize 15 kDa zein promoter, a 22 kDa zein promoter, a 27 kDa zein promoter, a g-zein promoter, a 27 kDa gamma-zein promoter (such as gzw64A promoter, see Genbank Accession number S78780), a waxy promoter, a shrunken 1 promoter, a shrunken 2 promoter, a globulin 1 promoter (see Genbank Accession number L22344), an Itp2 promoter, cim1 promoter, maize end1 and end2 promoters, nuc1 promoter, Zm40 promoter, eep1 and eep2; lecl, thioredoxin H promoter; mlip15 promoter, PCNA2 promoter; and the shrunken-2 promoter.

Examples of inducible promoters include promoters responsive to pathogen attack, anaerobic conditions, elevated temperature, light, drought, cold temperature, or high salt concentration. Pathogen-inducible promoters include those from pathogenesis-related proteins (PR proteins), which are induced following infection by a pathogen (for example, PR proteins, SAR proteins, beta-1,3-glucanase, chitinase).

In addition to plant promoters, other suitable promoters may be derived from bacterial origin for example, the octopine synthase promoter, the nopaline synthase promoter and other promoters derived from Ti plasmids, or may be derived from viral promoters (for example, 35S and 19S RNA promoters of cauliflower mosaic virus (CaMV), constitutive promoters of tobacco mosaic virus, cauliflower mosaic virus (CaMV) 19S and 35S promoters, or figwort mosaic virus 35S promoter).

Suitable methods of introducing nucleotide sequences into plant cells and subsequent insertion into the plant genome include microinjection (Crossway et al., Biotechniques 4:320-334 (1986)), electroporation (Riggs et al., Proc. Natl. Acad. Sci. USA 83:5602-5606 (1986)), *Agrobacterium-*mediated transformation (U.S. Pat. Nos. 5,981,840 and 5,563,055), direct gene transfer (Paszkowski et al., EMBO J. 3:2717-2722 (1984)), and ballistic particle acceleration (see, for example, U.S. Pat. Nos. 4,945,050; 5,879,918; 5,886,244; 5,932,782; Tomes et al., in Plant Cell, Tissue, and Organ Culture: Fundamental Methods, ed. Gamborg and Phillips (Springer-Verlag, Berlin) (1995); and McCabe et al., Biotechnology 6:923-926 (1988)).

### b. Mutation

A plant or plant cell comprising a mutation in a gene encoding an ABC transporter as described herein is disclosed, wherein said mutation results in modulated expression or modulated function of said transporter. The expression or function of the transporter(s) may be enhanced. Aside from one or more mutations in said transporter, the mutant plants or plant cells can have one or more further mutations in one or more other genes or polypeptides. In certain embodiments, aside from the one or more mutations in a transporter gene, the mutants can have one or more further mutations in one or more other genes or polypeptides such as one or more other transporter genes or polypeptides as described in the Sequence Listing.

There is also provided a method for modulating the level of a transporter in a (cured) plant or in (cured) plant material said method comprising introducing into the genome of said plant one or more mutations that modulate expression of at least one ABC transporter gene, wherein said at least one ABC transporter gene is selected from SEQ ID Nos: 1 and 3.

There is also provided a method for identifying a plant with modulated levels of NtABCG1-S or NtABCG1-T, said method comprising screening a nucleic acid sample from a plant of interest for the presence of one or more mutations in SEQ ID NOs:1 and/or 3, and optionally correlating the identified mutation(s) with mutation(s) that are known to modulate levels of ABC transporters.

There is also disclosed a plant or plant cell that is heterozygous or homozygous for mutations in a gene encoding an ABC transporter, wherein said mutation results in modulated (enhanced or reduced) expression or function of said transporter.

A number of approaches can be used to combine mutations in one plant including sexual crossing. A plant having one or more favourable heterozygous or homozygous mutations in an ABC transporter gene that enhances or reduces ABC transporter expression or activity can be crossed with a plant having one or more favourable heterozygous or homozygous mutations in one or more other ABC transporter genes that enhance or reduce transporter activity. In one embodiment, crosses are made in order to introduce one or more favourable heterozygous or homozygous mutations within am ABC transporter gene within the same plant.

The activity of one or more ABC transporter polypeptides in a plant is reduced or enhanced according to the present disclosure if the transporter activity is lower or higher than the transporter activity of the same ABC transporter(s) in a plant that has not been modified to inhibit the activity of that ABC transporter polypeptide and which has been cultured, harvested and cured using the same protocols.

In some embodiments, the mutation(s) is introduced into a plant or plant cell using a mutagenesis approach, and the introduced mutation is identified or selected using methods known to those of skill in the art - such as Southern blot analysis, DNA sequencing, PCR analysis, or phenotypic analysis. Mutations that impact gene expression or that interfere with the function of the encoded protein can be determined using methods that are well known in the art. Insertional mutations in gene exons usually result in null-mutants. Mutations in conserved residues can be particularly effective in inhibiting the metabolic function of the encoded protein. It will be appreciated, for example, that a mutation in one or more of the highly conserved regions would likely alter polypeptide function, while a mutation outside of those highly conserved regions would likely have little to no effect on polypeptide function. In addition, a mutation in a single nucleotide can create a stop codon, which would result in a truncated polypeptide and, depending on the extent of truncation, loss of function.

Methods for obtaining mutant polynucleotides and polypeptides are also disclosed. Any plant of interest, including a plant cell or plant material can be genetically modified by various methods known to induce mutagenesis, including site-directed mutagenesis, oligonucleotide-directed mutagenesis, chemically-induced mutagenesis, irradiation-induced mutagenesis, mutagenesis utilizing modified bases, mutagenesis utilizing gapped duplex DNA, double-strand break mutagenesis, mutagenesis utilizing repair-deficient host strains, mutagenesis by total gene synthesis, DNA shuffling and other equivalent methods.

Fragments of ABC transporter polynucleotides and polypeptides encoded thereby are also disclosed. Fragments of a polynucleotide may encode protein fragments that retain the biological activity of the native protein and hence are involved in the metabolite transport network in a plant. Alternatively, fragments of a polynucleotide that are useful as hybridization probes or PCR primers generally do not encode fragment proteins retaining biological activity. Furthermore, fragments of the disclosed nucleotide sequences include those that can be assembled within recombinant constructs as discussed herein. Fragments of a polynucleotide sequence may range from at least about 25 nucleotides, about 50 nucleotides, about 75 nucleotides, about 100 nucleotides about 150 nucleotides, about 200 nucleotides, about 250 nucleotides, about 300 nucleotides, about 400 nucleotides, about 500 nucleotides, about 600 nucleotides, about 700 nucleotides, about 800 nucleotides, about 900 nucleotides, about 1000 nucleotides, about 1100 nucleotides, about 1200 nucleotides, about 1300 nucleotides or about 1400 nucleotides and up to the full-length polynucleotide encoding the polypeptides described herein. Fragments of a polypeptide sequence may range from at least about 25 amino acids, about 50 amino acids, about 75 amino acids, about 100 amino acids about 150 amino acids, about 200 amino acids, about 250 amino acids, about 300 amino acids, about 400 amino acids, about 500 amino acids, and up to the full-length polypeptide described herein. Mutant polypeptide variants can be used to create mutant, non-naturally occurring or transgenic plants (for example, mutant, non-naturally occurring, transgenic, man-made or genetically engineered plants) or plant cells comprising one or more mutant polypeptide variants. Suitably, mutant polypeptide variants retain the activity of the unmutated polypeptide. The activity of the mutant polypeptide variant may be higher, lower or about the same as the unmutated polypeptide.

Mutations in the nucleotide sequences and polypeptides described herein can include man-made mutations or synthetic mutations or genetically engineered mutations. Mutations in the nucleotide sequences and polypeptides described herein can be mutations that are obtained or obtainable via a process which includes an *in vitro* or an *in vivo* manipulation step. Mutations in the nucleotide sequences and polypeptides described herein can be mutations that are obtained or obtainable via a process which includes intervention by man.

Methods that introduce a mutation randomly in a gene sequence can include chemical mutagenesis and radiation mutagenesis. Chemical mutagenesis involves the use of exogenously added chemicals - such as mutagenic, teratogenic, or carcinogenic organic compounds - to induce mutations. Mutagens that create primarily point mutations and short deletions, insertions, missense mutations, simple sequence repeats, transversions, and/or transitions, including chemical mutagens or radiation, may be used to create the mutations. Mutagens include, but are not limited to, ethyl methanesulfonate, methylmethane sulfonate, N-ethyl-N-nitrosurea, triethylmelamine, N-methyl-N-nitrosourea, procarbazine, chlorambucil, cyclophosphamide, diethyl sulfate, acrylamide monomer, melphalan, nitrogen mustard, vincristine, dimethylnitrosamine, N-methyl-N'-nitro-Nitrosoguanidine, nitrosoguanidine, 2-aminopurine, 7,12 dimethyl-benz(a)anthracene, ethylene oxide, hexamethylphosphoramide, bisulfan, diepoxyalkanes (diepoxyoctane, diepoxybutane, and the like), 2-methoxy-6-chloro-9[3-(ethyl-2-chloro-ethyl)aminopropylamino]acridine dihydrochloride and formaldehyde.

Spontaneous mutations in the locus that may not have been directly caused by the mutagen are also contemplated provided that they result in the desired phenotype. Suitable mutagenic agents can also include, for example, ionising radiation - such as X-rays, gamma rays, fast neutron irradiation and UV radiation. The dosage of the mutagenic chemical or radiation is determined experimentally for each type of plant tissue such that a mutation frequency is obtained that is below a threshold level characterized by lethality or reproductive sterility. Any method of plant nucleic acid preparation known to those of skill in the art may be used to prepare the plant nucleic acid for mutation screening.

The mutation process may include one or more plant crossing steps.

After mutation, screening can be performed to identify mutations that create premature stop codons or otherwise non-functional genes. After mutation, screening can be performed to identify mutations that create functional genes that are capable of being expressed at elevated or reduced levels. Screening of mutants can be carried out by sequencing, or by the use of one or more probes or primers specific to the gene or protein. Specific mutations in polynucleotides can also be created that can result in modulated gene expression, modulated stability of mRNA, or modulated stability of protein. Such plants are referred to herein as "non-naturally occurring" or "mutant" plants. Typically, the mutant or non-naturally occurring plants will include at least a portion of foreign or synthetic or man-made nucleic acid (for example, DNA or RNA) that was not present in the plant before it was manipulated. The foreign nucleic acid may be a single nucleotide, two or more nucleotides, two or more contiguous nucleotides or two or more non-contiguous nucleotides - such as at least 10, 20, 30, 40, 50,100, 200, 300, 400, 500, 600, 700, 800, 900, 1000, 1100, 1200, 1300, 1400 or 1500 or more contiguous or non-contiguous nucleotides.

### c. Transgenics and gene editing

Other than mutagenesis, compositions that can modulate the expression or the activity of one or more of the polynucleotides or polypeptides described herein include, but are not limited to, sequence-specific polynucleotides that can interfere with the transcription of one or more endogenous gene(s); sequence-specific polynucleotides that can interfere with the translation of RNA transcripts (for example, double-stranded RNAs, siRNAs, ribozymes); sequence-specific polypeptides that can interfere with the stability of one or more proteins; sequence-specific polynucleotides that can interfere with the enzymatic activity of one or more proteins or the binding activity of one or more proteins with respect to substrates or regulatory proteins; antibodies that exhibit specificity for one or more proteins; small molecule compounds that can interfere with the stability of one or more proteins or the enzymatic activity of one or more proteins or the binding activity of one or more proteins; zinc finger proteins that bind one or more polynucleotides; and meganucleases that have activity towards one or more polynucleotides. Gene editing technologies, genetic editing technologies and genome editing technologies are well known in the art.

### d. Zinc finger nucleases

Zinc finger proteins can be used to modulate the expression or the activity of one or more of the polynucleotides described herein. In various embodiments, a genomic DNA sequence comprising a part of or all of the coding sequence of the polynucleotide is modified by zinc finger nuclease-mediated mutagenesis. The genomic DNA sequence is searched for a unique site for zinc finger protein binding. Alternatively, the genomic DNA sequence is searched for two unique sites for zinc finger protein binding wherein both sites are on opposite strands and close together, for example, 1, 2, 3, 4, 5, 6 or more base pairs apart. Accordingly, zinc finger proteins that bind to polynucleotides are provided.

A zinc finger protein may be engineered to recognize a selected target site in a gene. A zinc finger protein can comprise any combination of motifs derived from natural zinc finger DNA-binding domains and non-natural zinc finger DNA-binding domains by truncation or expansion or a process of site-directed mutagenesis coupled to a selection method such as, but not limited to, phage display selection, bacterial two-hybrid selection or bacterial one-hybrid selection. The term "non-natural zinc finger DNA-binding domain" refers to a zinc finger DNA-binding domain that binds a three-base pair sequence within the target nucleic acid and that does not occur in the cell or organism comprising the nucleic acid which is to be modified. Methods for the design of zinc finger protein which binds specific nucleotide sequences which are unique to a target gene are known in the art.

In other embodiments, a zinc finger protein may be selected to bind to a regulatory sequence of a polynucleotide. More specifically, the regulatory sequence may comprise a transcription initiation site, a start codon, a region of an exon, a boundary of an exon-intron, a terminator, or a stop codon. Accordingly, the invention provides a mutant, non-naturally occurring or transgenic plant or plant cells, produced by zinc finger nuclease-mediated mutagenesis in the vicinity of or within one or more polynucleotides described herein, and methods for making such a plant or plant cell by zinc finger nuclease-mediated mutagenesis. Methods for delivering zinc finger protein and zinc finger nuclease to a plant are similar to those described below for delivery of meganuclease.

### e. Meganucleases

In another aspect, methods for producing mutant, non-naturally occurring or transgenic or otherwise genetically-modified plants using meganucleases, such as I-CreI, are described. Naturally occurring meganucleases as well as recombinant meganucleases can be used to specifically cause a double-stranded break at a single site or at relatively few sites in the genomic DNA of a plant to allow for the disruption of one or more polynucleotides described herein. The meganuclease may be an engineered meganuclease with altered DNA-recognition properties. Meganuclease proteins can be delivered into plant cells by a variety of different mechanisms known in the art.

The inventions encompass the use of meganucleases to inactivate a polynucleotide(s) described herein (or any combination thereof as described herein) in a plant cell or plant. Particularly, the invention provides a method for inactivating a polynucleotide in a plant using a meganuclease comprising: a) providing a plant cell comprising a polynucleotide as described herein; (b) introducing a meganuclease or a construct encoding a meganuclease into said plant cell; and (c) allowing the meganuclease to substantially inactivate the polynucleotide(s)

Meganucleases can be used to cleave meganuclease recognition sites within the coding regions of a polynucleotide. Such cleavage frequently results in the deletion of DNA at the meganuclease recognition site following mutagenic DNA repair by non-homologous end joining. Such mutations in the gene coding sequence are typically sufficient to inactivate the gene. This method to modify a plant cell involves, first, the delivery of a meganuclease expression cassette to a plant cell using a suitable transformation method. For highest efficiency, it is desirable to link the meganuclease expression cassette to a selectable marker and select for successfully transformed cells in the presence of a selection agent. This approach will result in the integration of the meganuclease expression cassette into the genome, however, which may not be desirable if the plant is likely to require regulatory approval. In such cases, the meganuclease expression cassette (and linked selectable marker gene) may be segregated away in subsequent plant generations using conventional breeding techniques.

Following delivery of the meganuclease expression cassette, plant cells are grown, initially, under conditions that are typical for the particular transformation procedure that was used. This may mean growing transformed cells on media at temperatures below 26°C, frequently in the dark. Such standard conditions can be used for a period of time, preferably 1-4 days, to allow the plant cell to recover from the transformation process. At any point following this initial recovery period, growth temperature may be raised to stimulate the activity of the engineered meganuclease to cleave and mutate the meganuclease recognition site.

### f. TALENs

One method of gene editing involves the use of transcription activator-like effector nucleases (TALENs) which induce double-strand breaks which cells can respond to with repair mechanisms. Non-homologous end joining reconnects DNA from either side of a double-strand break where there is very little or no sequence overlap for annealing. This repair mechanism induces errors in the genome via insertion or deletion, or chromosomal rearrangement. Any such errors may render the gene products coded at that location non-functional. For certain applications, it may be desirable to precisely remove the polynucleotide from the genome of the plant. Such applications are possible using a pair of engineered meganucleases, each of which cleaves a meganuclease recognition site on either side of the intended deletion. TALENs that are able to recognize and bind to a gene and introduce a double-strand break into the genome can also be used. Thus, in another aspect, methods for producing mutant, non-naturally occurring or transgenic or otherwise genetically-modified plants as described herein using TAL Effector Nucleases are contemplated.

### g. CRISPR/Cas

Another method of gene editing involves the use of the bacterial CRISPR/Cas system. Bacteria and archaea exhibit chromosomal elements called clustered regularly interspaced short palindromic repeats (CRISPR) that are part of an adaptive immune system that protects against invading viral and plasmid DNA. In Type II CRISPR systems, CRISPR RNAs (crRNAs) function with trans-activating crRNA (tracrRNA) and CRISPR-associated (Cas) proteins to introduce double-stranded breaks in target DNA. Target cleavage by Cas9 requires base-pairing between the crRNA and tracrRNA as well as base pairing between the crRNA and the target DNA. Target recognition is facilitated by the presence of a short motif called a protospacer-adjacent motif (PAM) that conforms to the sequence NGG. This system can be harnessed for genome editing. Cas9 is normally programmed by a dual RNA consisting of the crRNA and tracrRNA. However, the core components of these RNAs can be combined into a single hybrid 'guide RNA' for Cas9 targeting. The use of a noncoding RNA guide to target DNA for site-specific cleavage promises to be significantly more straightforward than existing technologies - such as TALENs. Using the CRISPR/Cas strategy, retargeting the nuclease complex only requires introduction of a new RNA sequence and there is no need to reengineer the specificity of protein transcription factors. CRISPR/Cas technology was implemented in plants in the method of international application WO 2015/189693 A1, which discloses a viral-mediated genome editing platform that is broadly applicable across plant species. The RNA2 genome of the tobacco rattle virus (TRV) was engineered to carry and deliver guide RNA into *Nicotiana benthamiana* plants overexpressing Cas9 endonuclease. In the context of the present invention, a guide RNA may be derived from any of the sequences disclosed herein and the teaching of WO 2015/189693 A1 applied to edit the genome of a plant cell and obtain a desired mutant plant. The fast pace of the development of the technology has generated a great variety of protocols with broad applicability in plantae, which have been well catalogued in a number of recent scientific review articles (e.g. Schiml et al. Plant Methods 2016 12:8; and Khatodia et al. Front Plant Sci. 2016; 7: 506). A review of CRISPR/Cas systems with a particular focus on its application in plants is given by Bortesi and Fischer (Biotechnology Advances Volume 33, Issue 1, January-February 2015, Pages 41-52). Bortesi and Fischer also make comparisons between the CRISPR/Cas technology, zinc finger nucleases, and TALENs. More recent developments in the use of CRISPR/Cas for manipulating plant genomes are described and discussed by Liu et al. in Acta Pharmaceutica Sinica B (Volume 7, Issue 3, May 2017, Pages 292-302), and the cutting edge of the technology is discussed by Puchta in Curr. Op. in Plant Biol. 2017 (36:1-8), demonstrating how rapidly the field is expanding. CRISPR/Cas9 plasmids for use in plants are listed in "addgene", the non-profit plasmid repository (addgene.org), and CRISPR/Cas plasmids are commercially available from Sigma Aldrich. Some studies have investigated improvements to specificity and applicability of CRISPR/Cas in plants, achieving mutation rates up to 32.8% without any off-target effects (Osakabi et al., 2016, Scientific Reports 6, Article number: 26685). Some applications of CRISPR/Cas in plants have focused on speeding up the process of plant breeding (Cao et al., Int. J. of Genomics, Volume 2016, Article ID 5078796).

### h. Antisense modification

Antisense technology is another well-known method that can be used to modulate the expression of a polypeptide. A polynucleotide of the gene to be repressed is cloned and operably linked to a regulatory region and a transcription termination sequence so that the antisense strand of RNA is transcribed. The recombinant construct is then transformed into a plant cell and the antisense strand of RNA is produced. The polynucleotide need not be the entire sequence of the gene to be repressed, but typically will be substantially complementary to at least a portion of the sense strand of the gene to be repressed.

A polynucleotide may be transcribed into a ribozyme, or catalytic RNA, that affects expression of an mRNA. Ribozymes can be designed to specifically pair with virtually any target RNA and cleave the phosphodiester backbone at a specific location, thereby functionally inactivating the target RNA. Heterologous polynucleotides can encode ribozymes designed to cleave particular mRNA transcripts, thus preventing expression of a polypeptide. Hammerhead ribozymes are useful for destroying particular mRNAs, although various ribozymes that cleave mRNA at site-specific recognition sequences can be used. Hammerhead ribozymes cleave mRNAs at locations dictated by flanking regions that form complementary base pairs with the target mRNA. The sole requirement is that the target RNA contains a 5'-UG-3' nucleotide sequence. The construction and production of hammerhead ribozymes is known in the art. Hammerhead ribozyme sequences can be embedded in a stable RNA such as a transfer RNA (tRNA) to increase cleavage efficiency *in vivo*.

In one embodiment, the sequence-specific polynucleotide that can interfere with the translation of RNA transcript(s) is interfering RNA. RNA interference or RNA silencing is an evolutionarily conserved process by which specific mRNAs can be targeted for enzymatic degradation. A double-stranded RNA (double-stranded RNA) is introduced or produced by a cell (for example, double-stranded RNA virus, or interfering RNA polynucleotides) to initiate the interfering RNA pathway. The double-stranded RNA can be converted into multiple small interfering RNA duplexes of 21-24 bp length by RNases III, which are double-stranded RNA-specific endonucleases. The small interfering RNAs can be subsequently recognized by RNA-induced silencing complexes that promote the unwinding of small interfering RNA through an ATP-dependent process. The unwound antisense strand of the small interfering RNA guides the activated RNA-induced silencing complexes to the targeted mRNA comprising a sequence complementary to the small interfering RNA anti-sense strand. The targeted mRNA and the anti-sense strand can form an A-form helix, and the major groove of the A-form helix can be recognized by the activated RNA-induced silencing complexes. The target mRNA can be cleaved by activated RNA-induced silencing complexes at a single site defined by the binding site of the 5'-end of the small interfering RNA strand. The activated RNA-induced silencing complexes can be recycled to catalyze another cleavage event.

Interfering RNA expression vectors may comprise interfering RNA constructs encoding interfering RNA polynucleotides that exhibit RNA interference activity by reducing the expression level of mRNAs, pre-mRNAs, or related RNA variants. The expression vectors may comprise a promoter positioned upstream and operably-linked to an Interfering RNA construct, as further described herein. Interfering RNA expression vectors may comprise a suitable minimal core promoter, a Interfering RNA construct of interest, an upstream (5') regulatory region, a downstream (3') regulatory region, including transcription termination and polyadenylation signals, and other sequences known to persons skilled in the art, such as various selection markers.

The polynucleotides can be produced in various forms, including as double stranded structures (that is, a double-stranded RNA molecule comprising an antisense strand and a complementary sense strand), double-stranded hairpin-like structures, or single-stranded structures (that is, a ssRNA molecule comprising just an antisense strand). The structures may comprise a duplex, asymmetric duplex, hairpin or asymmetric hairpin secondary structure, having self-complementary sense and antisense strands. The double stranded interfering RNA can be enzymatically converted to double-stranded small interfering RNAs. One of the strands of the small interfering RNA duplex can anneal to a complementary sequence within the target mRNA and related RNA variants. The small interfering RNA/mRNA duplexes are recognized by RNA-induced silencing complexes that can cleave RNAs at multiple sites in a sequence-dependent manner, resulting in the degradation of the target mRNA and related RNA variants.

The double-stranded RNA molecules may include small interfering RNA molecules assembled from a single oligonucleotide in a stem-loop structure, wherein self-complementary sense and antisense regions of the small interfering RNA molecule are linked by means of a polynucleotide based or non-polynucleotide-based linker(s), as well as circular single-stranded RNA having two or more loop structures and a stem comprising self-complementary sense and antisense strands, wherein the circular RNA can be processed either *in vivo* or *in vitro* to generate an active small interfering RNA molecule capable of mediating interfering RNA.

The use of small hairpin RNA molecules is also contemplated. They comprise a specific antisense sequence in addition to the reverse complement (sense) sequence, typically separated by a spacer or loop sequence. Cleavage of the spacer or loop provides a single-stranded RNA molecule and its reverse complement, such that they may anneal to form a double-stranded RNA molecule (optionally with additional processing steps that may result in addition or removal of one, two, three or more nucleotides from the 3' end or the 5' end of either or both strands). The spacer can be of a sufficient length to permit the antisense and sense sequences to anneal and form a double-stranded structure (or stem) prior to cleavage of the spacer (and, optionally, subsequent processing steps that may result in addition or removal of one, two, three, four, or more nucleotides from the 3' end or the 5' end of either or both strands). The spacer sequence is typically an unrelated nucleotide sequence that is situated between two complementary nucleotide sequence regions which, when annealed into a double-stranded polynucleotide, comprise a small hairpin RNA. The spacer sequence generally comprises between about 3 and about 100 nucleotides.

Any RNA polynucleotide of interest can be produced by selecting a suitable sequence composition, loop size, and stem length for producing the hairpin duplex. A suitable range for designing stem lengths of a hairpin duplex, includes stem lengths of at least about 10, 11, 12, 13, 14, 15, 16, 17, 18, 19 or 20 nucleotides - such as about 14-30 nucleotides, about 30-50 nucleotides, about 50-100 nucleotides, about 100-150 nucleotides, about 150-200 nucleotides, about 200-300 nucleotides, about 300-400 nucleotides, about 400-500 nucleotides, about 500-600 nucleotides, and about 600-700 nucleotides. A suitable range for designing loop lengths of a hairpin duplex, includes loop lengths of about 4-25 nucleotides, about 25-50 nucleotides, or longer if the stem length of the hair duplex is substantial. In certain embodiments, a double-stranded RNA or ssRNA molecule is between about 15 and about 40 nucleotides in length. In another embodiment, the small interfering RNA molecule is a double-stranded RNA or ssRNA molecule between about 15 and about 35 nucleotides in length. In another embodiment, the small interfering RNA molecule is a double-stranded RNA or ssRNA molecule between about 17 and about 30 nucleotides in length. In another embodiment, the small interfering RNA molecule is a double-stranded RNA or ssRNA molecule between about 19 and about 25 nucleotides in length. In another embodiment, the small interfering RNA molecule is a double-stranded RNA or ssRNA molecule between about 21 to about 23 nucleotides in length. In certain embodiments, hairpin structures with duplexed regions longer than 21 nucleotides may promote effective small interfering RNA-directed silencing, regardless of loop sequence and length. An exemplary sequence for RNA interference is set forth in SEQ ID NO: 5.

The target mRNA sequence is typically between about 14 to about 50 nucleotides in length. The target mRNA can, therefore, be scanned for regions between about 14 and about 50 nucleotides in length that preferably meet one or more of the following criteria for a target sequence: an A+T/G+C ratio of between about 2:1 and about 1:2; an AA dinucleotide or a CA dinucleotide at the 5' end of the target sequence; a sequence of at least 10 consecutive nucleotides unique to the target mRNA (that is, the sequence is not present in other mRNA sequences from the same plant); and no "runs" of more than three consecutive guanine (G) nucleotides or more than three consecutive cytosine (C) nucleotides. These criteria can be assessed using various techniques known in the art, for example, computer programs such as BLAST can be used to search publicly available databases to determine whether the selected target sequence is unique to the target mRNA. Alternatively, a target sequence can be selected (and a small interfering RNA sequence designed) using computer software available commercially (for example, OligoEngine, Target Finder and the small interfering RNA Design Tool which are commercially available).

In one embodiment, target mRNA sequences are selected that are between about 14 and about 30 nucleotides in length that meet one or more of the above criteria. In another embodiment, target sequences are selected that are between about 16 and about 30 nucleotides in length that meet one or more of the above criteria. In a further embodiment, target sequences are selected that are between about 19 and about 30 nucleotides in length that meet one or more of the above criteria. In another embodiment, target sequences are selected that are between about 19 and about 25 nucleotides in length that meet one or more of the above criteria.

In an exemplary embodiment, the small interfering RNA molecules comprise a specific antisense sequence that is complementary to at least 12, 13, 14, 15, 16, 17, 18, 19, 20, 21, 22, 23, 24, 25, 26, 27, 28, 29, 30, or more contiguous nucleotides of any one of the polynucleotide sequences described herein.

The specific antisense sequence comprised by the small interfering RNA molecule can be identical or substantially identical to the complement of the target sequence. In one embodiment, the specific antisense sequence comprised by the small interfering RNA molecule is at least about 75%, 80%, 85%, 90%, 95%, 96%, 97%, 98%, 99% or 100% identical to the complement of the target mRNA sequence. Methods of determining sequence identity are known in the art and can be determined, for example, by using the BLASTN program of the University of Wisconsin Computer Group (GCG) software or provided on the NCBI website.

The specific antisense sequence of the small interfering RNA molecules may exhibit variability by differing (for example, by nucleotide substitution, including transition or transversion) at one, two, three, four or more nucleotides from the sequence of the target mRNA. When such nucleotide substitutions are present in the antisense strand of a double-stranded RNA molecule, the complementary nucleotide in the sense strand with which the substitute nucleotide would typically form hydrogen bond base-pairing may or may not be correspondingly substituted. Double-stranded RNA molecules in which one or more nucleotide substitution occurs in the sense sequence, but not in the antisense strand, are also contemplated. When the antisense sequence of an small interfering RNA molecule comprises one or more mismatches between the nucleotide sequence of the small interfering RNA and the target nucleotide sequence, as described above, the mismatches may be found at the 3' terminus, the 5' terminus or in the central portion of the antisense sequence.

In another embodiment, the small interfering RNA molecules comprise a specific antisense sequence that is capable of selectively hybridizing under stringent conditions to a portion of a naturally occurring target gene or target mRNA. As known to those of ordinary skill in the art, variations in stringency of hybridization conditions may be achieved by altering the time, temperature or concentration of the solutions used for the hybridization and wash steps. Suitable conditions can also depend in part on the particular nucleotide sequences used, for example the sequence of the target mRNA or gene.

One method for inducing double stranded RNA-silencing in plants is transformation with a gene construct producing hairpin RNA (see Smith et al. (2000) Nature, 407, 319-320). Such constructs comprise inverted regions of the target gene sequence, separated by an appropriate spacer. The insertion of a functional plant intron region as a spacer fragment additionally increases the efficiency of the gene silencing induction, due to generation of an intron spliced hairpin RNA (Wesley et al. (2001) Plant J., 27, 581-590). Suitably, the stem length is about 50 nucleotides to about 1 kilobases in length. Methods for producing intron spliced hairpin RNA are well described in the art (see for example, Bioscience, Biotechnology, and Biochemistry (2008) 72, 2, 615-617).

Interfering RNA molecules having a duplex or double-stranded structure, for example double-stranded RNA or small hairpin RNA, can have blunt ends, or can have 3' or 5' overhangs. As used herein, "overhang" refers to the unpaired nucleotide or nucleotides that protrude from a duplex structure when a 3'-terminus of one RNA strand extends beyond the 5'-terminus of the other strand (3' overhang), or vice versa (5' overhang). The nucleotides comprising the overhang can be ribonucleotides, deoxyribonucleotides or modified versions thereof. In one embodiment, at least one strand of the interfering RNA molecule has a 3' overhang from about 1 to about 6 nucleotides in length. In other embodiments, the 3' overhang is from about 1 to about 5 nucleotides, from about 1 to about 3 nucleotides and from about 2 to about 4 nucleotides in length.

When the interfering RNA molecule comprises a 3' overhang at one end of the molecule, the other end can be blunt-ended or have also an overhang (5' or 3'). When the interfering RNA molecule comprises an overhang at both ends of the molecule, the length of the overhangs may be the same or different. In one embodiment, the interfering RNA molecule comprises 3' overhangs of about 1 to about 3 nucleotides on both ends of the molecule. In a further embodiment, the interfering RNA molecule is a double-stranded RNA having a 3' overhang of 2 nucleotides at both ends of the molecule. In yet another embodiment, the nucleotides comprising the overhang of the interfering RNA are TT dinucleotides or UU dinucleotides.

When determining the percentage identity of the interfering RNA molecule comprising one or more overhangs to the target mRNA sequence, the overhang(s) may or may not be taken into account. For example, the nucleotides from a 3' overhang and up to 2 nucleotides from the 5'- or 3'-terminus of the double strand may be modified without significant loss of activity of the small interfering RNA molecule.

The interfering RNA molecules can comprise one or more 5' or 3'-cap structures. The interfering RNA molecule can comprise a cap structure at the 3'-end of the sense strand, the antisense strand, or both the sense and antisense strands; or at the 5'-end of the sense strand, the antisense strand, or both the sense and antisense strands of the interfering RNA molecule. Alternatively, the interfering RNA molecule can comprise a cap structure at both the 3'-end and 5'-end of the interfering RNA molecule. The term "cap structure" refers to a chemical modification incorporated at either terminus of an oligonucleotide, which protects the molecule from exonuclease degradation, and may also facilitate delivery or localisation within a cell.

Another modification applicable to interfering RNA molecules is the chemical linkage to the interfering RNA molecule of one or more moieties or conjugates which enhance the activity, cellular distribution, cellular uptake, bioavailability or stability of the interfering RNA molecule. The polynucleotides may be synthesized or modified by methods well established in the art. Chemical modifications may include, but are not limited to 2' modifications, introduction of non-natural bases, covalent attachment to a ligand, and replacement of phosphate linkages with thiophosphate linkages. In this embodiment, the integrity of the duplex structure is strengthened by at least one, and typically two, chemical linkages. Chemical linking may be achieved by any of a variety of well-known techniques, for example by introducing covalent, ionic or hydrogen bonds; hydrophobic interactions, van der Waals or stacking interactions; by means of metal-ion coordination, or through use of purine analogues.

The nucleotides at one or both of the two single strands may be modified to modulate the activation of cellular enzymes, such as, for example, without limitation, certain nucleases. Techniques for reducing or inhibiting the activation of cellular enzymes are known in the art including, but not limited to, 2'-amino modifications, 2'-fluoro modifications, 2'-alkyl modifications, uncharged backbone modifications, morpholino modifications, 2'-O-methyl modifications, and phosphoramidate. Thus, at least one 2'-hydroxyl group of the nucleotides on a double-stranded RNA is replaced by a chemical group. Also, at least one nucleotide may be modified to form a locked nucleotide. Such locked nucleotide contains a methylene or ethylene bridge that connects the 2'-oxygen of ribose with the 4'-carbon of ribose. Introduction of a locked nucleotide into an oligonucleotide improves the affinity for complementary sequences and increases the melting temperature by several degrees.

Ligands may be conjugated to an interfering RNA molecule, for example, to enhance its cellular absorption. In certain embodiments, a hydrophobic ligand is conjugated to the molecule to facilitate direct permeation of the cellular membrane. These approaches have been used to facilitate cell permeation of antisense oligonucleotides. In certain instances, conjugation of a cationic ligand to oligonucleotides often results in improved resistance to nucleases. Representative examples of cationic ligands include propylammonium and dimethylpropylammonium. Anti-sense oligonucleotides can retain their high binding affinity to mRNA when the cationic ligand is dispersed throughout the oligonucleotide.

The molecules and polynucleotides described herein may be prepared using well-known techniques of solid-phase synthesis. Any other means for such synthesis known in the art may additionally or alternatively be employed.

"Targeted Induced Local Lesions In Genomes" (TILLING) is another mutagenesis technology that can be used to generate and/or identify polynucleotides encoding polypeptides with modified expression and/or activity. TILLING also allows selection of plants carrying such mutants. TILLING combines high-density mutagenesis with high-throughput screening methods. Methods for TILLING are well known in the art (see McCallum et al., (2000) Nat Biotechnol 18: 455-457 and Stemple (2004) Nat Rev Genet 5(2): 145-50).

Various embodiments are directed to expression vectors comprising one or more of the polynucleotides or interfering RNA constructs that comprise one or more polynucleotides described herein.

Various embodiments are directed to expression vectors comprising one or more of the polynucleotides or one or more interfering RNA constructs described herein.

Various embodiments are directed to expression vectors comprising one or more polynucleotides or one or more interfering RNA constructs encoding one or more interfering RNA polynucleotides described herein that are capable of self-annealing to form a hairpin structure, in which the construct comprises (a) one or more of the polynucleotides described herein; (b) a second sequence encoding a spacer element that forms a loop of the hairpin structure; and (c) a third sequence comprising a reverse complementary sequence of the first sequence, positioned in the same orientation as the first sequence, wherein the second sequence is positioned between the first sequence and the third sequence, and the second sequence is operably-linked to the first sequence and to the third sequence.

The disclosed sequences can be utilised for constructing various polynucleotides that do not form hairpin structures. For example, a double-stranded RNA can be formed by (1) transcribing a first strand of the DNA by operably-linking to a first promoter, and (2) transcribing the reverse complementary sequence of the first strand of the DNA fragment by operably-linking to a second promoter. Each strand of the polynucleotide can be transcribed from the same expression vector, or from different expression vectors. The RNA duplex having RNA interference activity can be enzymatically converted to small interfering RNAs to modulate RNA levels.

Thus, various embodiments are directed to expression vectors comprising one or more polynucleotides or interfering RNA constructs described herein encoding interfering RNA polynucleotides capable of self-annealing, in which the construct comprises (a) one or more of the polynucleotides described herein; and (b) a second sequence comprising a complementary (for example, reverse complementary) sequence of the first sequence, positioned in the same orientation as the first sequence.

Various compositions and methods are provided for modulating the endogenous expression levels of one or more of the polypeptides described herein (or any combination thereof as described herein) by promoting co-suppression of gene expression.

Various compositions and methods are provided for modulating the endogenous gene expression level by modulating the translation of mRNA. A host (tobacco) plant cell can be transformed with an expression vector comprising: a promoter operably-linked to a polynucleotide, positioned in anti-sense orientation with respect to the promoter to enable the expression of RNA polynucleotides having a sequence complementary to a portion of mRNA.

Various expression vectors for modulating the translation of mRNA may comprise: a promoter operably-linked to a polynucleotide in which the sequence is positioned in anti-sense orientation with respect to the promoter. The lengths of anti-sense RNA polynucleotides can vary, and may be from about 15-20 nucleotides, about 20-30 nucleotides, about 30-50 nucleotides, about 50-75 nucleotides, about 75-100 nucleotides, about 100-150 nucleotides, about 150-200 nucleotides, and about 200-300 nucleotides.

### i. Mobile genetic elements

Alternatively, genes can be targeted for inactivation by introducing transposons (for example, IS elements) into the genomes of plants of interest. These mobile genetic elements can be introduced by sexual cross-fertilization and insertion mutants can be screened for loss in protein activity. The disrupted gene in a parent plant can be introduced into other plants by crossing the parent plant with plant not subjected to transposon-induced mutagenesis by, for example, sexual cross-fertilization. Any standard breeding techniques known to persons skilled in the art can be utilized. In one embodiment, one or more genes can be inactivated by the insertion of one or more transposons. Mutations can result in homozygous disruption of one or more genes, in heterozygous disruption of one or more genes, or a combination of both homozygous and heterozygous disruptions if more than one gene is disrupted. Suitable transposable elements include retrotransposons, retroposons, and SINE-like elements. Such methods are known to persons skilled in the art.

### j. Ribozymes

Alternatively, genes can be targeted for inactivation by introducing ribozymes derived from a number of small circular RNAs that are capable of self-cleavage and replication in plants. These RNAs can replicate either alone (viroid RNAs) or with a helper virus (satellite RNAs). Examples of suitable RNAs include those derived from avocado sunblotch viroid and satellite RNAs derived from tobacco ringspot virus, lucerne transient streak virus, velvet tobacco mottle virus, solanum nodiflorum mottle virus, and subterranean clover mottle virus. Various target RNA-specific ribozymes are known to persons skilled in the art.

### 5. Plants

The mutant or non-naturally occurring plants or plant cells can have any combination of one or more mutations in one or more genes which results in modulated expression or activity of those genes or their products. For example, the mutant or non-naturally occurring plants or plant cells may have a single mutation in a single gene; multiple mutations in a single gene; a single mutation in two or more or three or more or four or more genes; or multiple mutations in two or more or three or more or four or more genes. Examples of such mutations are described herein. By way of further example, the mutant or non-naturally occurring plants or plant cells may have one or more mutations in a specific portion of the gene(s) - such as in a region of the gene that encodes an active site of the protein or a portion thereof. By way of further example, the mutant or non-naturally occurring plants or plant cells may have one or more mutations in a region outside of one or more gene(s) - such as in a region upstream or downstream of the gene it regulates provided that they modulate the activity or expression of the gene(s). Upstream elements can include promoters, enhancers or transription factors. Some elements - such as enhancers - can be positioned upstream or downstream of the gene it regulates. The element(s) need not be located near to the gene that it regulates since some elements have been found located several hundred thousand base pairs upstream or downstream of the gene that it regulates. The mutant or non-naturally occurring plants or plant cells may have one or more mutations located within the first 100 nucleotides of the gene(s), within the first 200 nucleotides of the gene(s), within the first 300 nucleotides of the gene(s), within the first 400 nucleotides of the gene(s), within the first 500 nucleotides of the gene(s), within the first 600 nucleotides of the gene(s), within the first 700 nucleotides of the gene(s), within the first 800 nucleotides of the gene(s), within the first 900 nucleotides of the gene(s), within the first 1000 nucleotides of the gene(s), within the first 1100 nucleotides of the gene(s), within the first 1200 nucleotides of the gene(s), within the first 1300 nucleotides of the gene(s), within the first 1400 nucleotides of the gene(s) or within the first 1500 nucleotides of the gene(s). The mutant or non-naturally occurring plants or plant cells may have one or more mutations located within the first, second, third, fourth, fifth, sixth, seventh, eighth, ninth, tenth, eleventh, twelfth, thirteenth, fourteenth or fifteenth set of 100 nucleotides of the gene(s) or combinations thereof. Mutant or non-naturally occurring plants or plant cells (for example, mutant, non-naturally occurring or transgenic plants or plant cells and the like, as described herein) comprising the mutant polypeptide variants are disclosed.

In one embodiment, seeds from plants are mutagenised and then grown into first generation mutant plants. The first generation plants are then allowed to self-pollinate and seeds from the first generation plant are grown into second generation plants, which are then screened for mutations in their loci. Though the mutagenized plant material can be screened for mutations, an advantage of screening the second generation plants is that all somatic mutations correspond to germline mutations. One of skill in the art would understand that a variety of plant materials, including but not limited to, seeds, pollen, plant tissue or plant cells, may be mutagenised in order to create the mutant plants. However, the type of plant material mutagenised may affect when the plant nucleic acid is screened for mutations. For example, when pollen is subjected to mutagenesis prior to pollination of a non-mutagenized plant the seeds resulting from that pollination are grown into first generation plants. Every cell of the first generation plants will contain mutations created in the pollen; thus these first generation plants may then be screened for mutations instead of waiting until the second generation.

### a. Preparation of modified plants, screening, and crossing

Prepared nucleic acid from individual plants, plant cells, or plant material can optionally be pooled in order to expedite screening for mutations in the population of plants originating from the mutagenized plant tissue, cells or material. One or more subsequent generations of plants, plant cells or plant material can be screened. The size of the optionally pooled group is dependent upon the sensitivity of the screening method used.

After the nucleic acid samples are optionally pooled, they can be subjected to polynucleotide-specific amplification techniques, such as Polymerase Chain Reaction. Any one or more primers or probes specific to the gene or the sequences immediately adjacent to the gene may be utilized to amplify the sequences within the optionally pooled nucleic acid sample. Suitably, the one or more primers or probes are designed to amplify the regions of the locus where useful mutations are most likely to arise. Most preferably, the primer is designed to detect mutations within regions of the polynucleotide. Additionally, it is preferable for the primer(s) and probe(s) to avoid known polymorphic sites in order to ease screening for point mutations. To facilitate detection of amplification products, the one or more primers or probes may be labelled using any conventional labelling method. Primer(s) or probe(s) can be designed based upon the sequences described herein using methods that are well understood in the art.

To facilitate detection of amplification products, the primer(s) or probe(s) may be labelled using any conventional labelling method. These can be designed based upon the sequences described herein using methods that are well understood in the art.

Polymorphisms may be identified by means known in the art and some have been described in the literature.

In some embodiments, a plant may be regenerated or grown from the plant, plant tissue or plant cell. Any suitable methods for regenerating or growing a plant from a plant cell or plant tissue may be used, such as, without limitation, tissue culture or regeneration from protoplasts. Suitably, plants may be regenerated by growing transformed plant cells on callus induction media, shoot induction media and/or root induction media. See, for example, McCormick et al., Plant Cell Reports 5:81-84 (1986). These plants may then be grown, and either pollinated with the same transformed strain or different strains, and the resulting hybrid having expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. Thus as used herein, "transformed seeds" refers to seeds that contain the nucleotide construct stably integrated into the plant genome.

Accordingly, in a further aspect there is provided a method of preparing a mutant plant. The method involves providing at least one cell of a plant comprising a gene encoding a functional polynucleotide described herein (or any combination thereof as described herein). Next, the at least one cell of the plant is treated under conditions effective to modulate the activity of the polynucleotide(s) described herein. The at least one mutant plant cell is then propagated into a mutant plant, where the mutant plant has a modulated level of polypeptide(s) described (or any combination thereof as described herein) as compared to that of a control plant. In one embodiment of this method of making a mutant plant, the treating step involves subjecting the at least one cell to a chemical mutagenising agent as described above and under conditions effective to yield at least one mutant plant cell. In another embodiment of this method, the treating step involves subjecting the at least one cell to a radiation source under conditions effective to yield at least one mutant plant cell. The term "mutant plant" includes mutant plants in which the genotype is modified as compared to a control plant, suitably by means other than genetic engineering or genetic modification.

In certain embodiments, the mutant plant, mutant plant cell or mutant plant material may comprise one or more mutations that have occured naturally in another plant, plant cell or plant material and confer a desired trait. This mutation can be incorporated (for example, introgressed) into another plant, plant cell or plant material (for example, a plant, plant cell or plant material with a different genetic background to the plant from which the mutation was derived) to confer the trait thereto. Thus by way of example, a mutation that occurred naturally in a first plant may be introduced into a second plant - such as a second plant with a different genetic background to the first plant. The skilled person is therefore able to search for and identify a plant carrying naturally in its genome one or more mutant alleles of the genes described herein which confer a desired trait. The mutant allele(s) that occurs naturally can be transferred to the second plant by various methods including breeding, backcrossing and introgression to produce a lines, varieties or hybrids that have one or more mutations in the genes described herein. The same technique can also be applied to the introgression of one or more non-naturally occurring mutation(s) from a first plant into a second plant. Plants showing a desired trait may be screened out of a pool of mutant plants. Suitably, the selection is carried out utilising the knowledge of the nucleotide sequences as described herein. Consequently, it is possible to screen for a genetic trait as compared to a control. Such a screening approach may involve the application of conventional nucleic acid amplification and/or hybridization techniques as discussed herein. Thus, a further aspect of the present invention relates to a method for identifying a mutant plant comprising the steps of: (a) providing a sample comprising nucleic acid from a plant; and (b) determining the nucleic acid sequence of the polynucleotide, wherein a difference in the sequence of the polynucleotide as compared to the polynucleotide sequence of a control plant is indicative that said plant is a mutant plant. In another aspect there is provided a method for identifying a mutant plant which accumulates increased or reduced levels of alkaloids as compared to a control plant comprising the steps of: (a) providing a sample from a plant to be screened; (b) determining if said sample comprises one or more mutations in one or more of the polynucleotides described herein; and (c) determining the level of at least one alkaloid of said plant. Suitably the level of at least one alkaloid is determined in green leaves. In another aspect there is provided a method for preparing a mutant plant which has increased or reduced levels of at least one alkaloid as compared to a control plant comprising the steps of: (a) providing a sample from a first plant; (b) determining if said sample comprises one or more mutations in one or more the polynucleotides described herein that result in modulated levels of at least one alkaloid; and (c) transferring the one or more mutations into a second plant. Suitably the level of at least one alkaloid is determined in green leaves. The mutation(s) can be transferred into the second plant using various methods that are known in the art - such as by genetic engineering, genetic manipulation, introgression, plant breeding, backcrossing and the like. In one embodiment, the first plant is a naturally occurring plant. In one embodiment, the second plant has a different genetic background to the first plant. In another aspect there is provided a method for preparing a mutant plant which has increased or reduced levels of at least one alkaloid as compared to a control plant comprising the steps of: (a) providing a sample from a first plant; (b) determining if said sample comprises one or more mutations in one or more of the polynucleotides described herein that results in modulated levels of at least one alkaloid; and (c) introgressing the one or more mutations from the first plant into a second plant. Suitably the level of at least one alkaloid is determined in green leaves. In one embodiment, the step of introgressing comprises plant breeding, optionally including backcrossing and the like. In one embodiment, the first plant is a naturally occurring plant. In one embodiment, the second plant has a different genetic background to the first plant. In one embodiment, the first plant is not a cultivar or an elite cultivar. In one embodiment, the second plant is a cultivar or an elite cultivar. A further aspect relates to a mutant plant (including a cultivar or elite cultivar mutant plant) obtained or obtainable by the methods described herein. In certain embodiments, the "mutant plants" may have one or more mutations localised only to a specific region of the plant - such as within the sequence of the one or more polynucleotide(s) described herein. According to this embodiment, the remaining genomic sequence of the mutant plant will be the same or substantially the same as the plant prior to the mutagenesis.

In certain embodiments, the mutant plants may have one or more mutations localised in more than one genomic region of the plant - such as within the sequence of one or more of the polynucleotides described herein and in one or more further regions of the genome. According to this embodiment, the remaining genomic sequence of the mutant plant will not be the same or will not be substantially the same as the plant prior to the mutagenesis. In certain embodiments, the mutant plants may not have one or more mutations in one or more, two or more, three or more, four or more or five or more exons of the polynucleotide(s) described herein; or may not have one or more mutations in one or more, two or more, three or more, four or more or five or more introns of the polynucleotide(s) described herein; or may not have one or more mutations in a promoter of the polynucleotide(s) described herein; or may not have one or more mutations in the 3' untranslated region of the polynucleotide(s) described herein; or may not have one or more mutations in the 5' untranslated region of the polynucleotide(s) described herein; or may not have one or more mutations in the coding region of the polynucleotide(s) described herein; or may not have one or more mutations in the non-coding region of the polynucleotide(s) described herein; or any combination of two or more, three or more, four or more, five or more; or six or more thereof parts thereof.

In a further aspect there is provided a method of identifying a plant, a plant cell or plant material comprising a mutation in a gene encoding a polynucleotide described herein comprising: (a) subjecting a plant, a plant cell or plant material to mutagenesis; (b) obtaining a nucleic acid sample from said plant, plant cell or plant material or descendants thereof; and (c) determining the nucleic acid sequence of the gene encoding a polynucleotide described herein or a variant or a fragment thereof, wherein a difference in said sequence is indicative of one or more mutations therein. This method also allows the selection of plants having mutation(s) that occur(s) in genomic regions that affect the expression of the gene in a plant cell, such as a transcription initiation site, a start codon, a region of an intron, a boundary of an exon-intron, a terminator, or a stop codon.

### b. Plant families, species, varieties, seeds, and tissue culture

Plants suitable for use in genetic modification include, but are not limited to, monocotyledonous and dicotyledonous plants and plant cell systems, including species from one of the following families: Acanthaceae, Alliaceae, Alstroemeriaceae, Amaryllidaceae, Apocynaceae, Arecaceae, Asteraceae, Berberidaceae, Bixaceae, Brassicaceae, Bromeliaceae, Cannabaceae, Caryophyllaceae, Cephalotaxaceae, Chenopodiaceae, Colchicaceae, Cucurbitaceae, Dioscoreaceae, Ephedraceae, Erythroxylaceae, Euphorbiaceae, Fabaceae, Lamiaceae, Linaceae, Lycopodiaceae, Malvaceae, Melanthiaceae, Musaceae, Myrtaceae, Nyssaceae, Papaveraceae, Pinaceae, Plantaginaceae, Poaceae, Rosaceae, Rubiaceae, Salicaceae, Sapindaceae, Solanaceae, Taxaceae, Theaceae, or Vitaceae.

Suitable species may include members of the genera Abelmoschus, Abies, Acer, Agrostis, Allium, Alstroemeria, Ananas, Andrographis, Andropogon, Artemisia, Arundo, Atropa, Berberis, Beta, Bixa, Brassica, Calendula, Camellia, Camptotheca, Cannabis, Capsicum, Carthamus, Catharanthus, Cephalotaxus, Chrysanthemum, Cinchona, Citrullus, Coffea, Colchicum, Coleus, Cucumis, Cucurbita, Cynodon, Datura, Dianthus, Digitalis, Dioscorea, Elaeis, Ephedra, Erianthus, Erythroxylum, Eucalyptus, Festuca, Fragaria, Galanthus, Glycine, Gossypium, Helianthus, Hevea, Hordeum, Hyoscyamus, Jatropha, Lactuca, Linum, Lolium, Lupinus, Lycopersicon, Lycopodium, Manihot, Medicago, Mentha, Miscanthus, Musa, Nicotiana, Oryza, Panicum, Papaver, Parthenium, Pennisetum, Petunia, Phalaris, Phleum, Pinus, Poa, Poinsettia, Populus, Rauwolfia, Ricinus, Rosa, Saccharum, Salix, Sanguinaria, Scopolia, Secale, Solanum, Sorghum, Spartina, Spinacea, Tanacetum, Taxus, Theobroma, Triticosecale, Triticum, Uniola, Veratrum, Vinca, Vitis, and Zea.

Suitable species may include Panicum spp., Sorghum spp., Miscanthus spp., Saccharum spp., Erianthus spp., Populus spp., Andropogon gerardii (big bluestem), Pennisetum purpureum (elephant grass), Phalaris arundinacea (reed canarygrass), Cynodon dactylon (bermudagrass), Festuca arundinacea (tall fescue), Spartina pectinata (prairie cord-grass), Medicago sativa (alfalfa), Arundo donax (giant reed), Secale cereale (rye), Salix spp. (willow), Eucalyptus spp. (eucalyptus), Triticosecale (tritic wheat times rye), bamboo, Helianthus annuus (sunflower), Carthamus tinctorius (safflower), Jatropha curcas (jatropha), Ricinus communis (castor), Elaeis guineensis (palm), Linum usitatissimum (flax), Brassica juncea, Beta vulgaris (sugarbeet), Manihot esculenta (cassaya), Lycopersicon esculentum (tomato), Lactuca sativa (lettuce), Musyclise alca (banana), Solanum tuberosum (potato), Brassica oleracea (broccoli, cauliflower, Brussels sprouts), Camellia sinensis (tea), Fragaria ananassa (strawberry), Theobroma cacao (cocoa), Coffeycliseca (coffee), Vitis vinifera (grape), Ananas comosus (pineapple), Capsicum annum (hot & sweet pepper), Allium cepa (onion), Cucumis melo (melon), Cucumis sativus (cucumber), Cucurbita maxima (squash), Cucurbita moschata (squash), Spinacea oleracea (spinach), Citrullus lanatus (watermelon), Abelmoschus esculentus (okra), Solanum melongena (eggplant), Rosa spp. (rose), Dianthus caryophyllus (carnation), Petunia spp. (petunia), Poinsettia pulcherrima (poinsettia), Lupinus albus (lupin), Uniola paniculata (oats), bentgrass (Agrostis spp.), Populus tremuloides (aspen), Pinus spp. (pine), Abies spp. (fir), Acer spp. (maple), Hordeum vulgare (barley), Poa pratensis (bluegrass), Lolium spp. (ryegrass) and Phleum pratense (timothy), Panicum virgatum (switchgrass), Sorghuycliseor (sorghum, sudangrass), Miscanthus giganteus (miscanthus), Saccharum sp. (energycane), Populus balsamifera (poplar), Zea mays (corn), Glycine max (soybean), Brassica napus (canola), Triticum aestivum (wheat), Gossypium hirsutum (cotton), Oryza sativa (rice), Helianthus annuus (sunflower), Medicago sativa (alfalfa), Beta vulgaris (sugarbeet), or Pennisetum glaucum (pearl millet).

Various embodiments are directed to mutant tobacco, non-naturally occurring tobacco or transgenic tobacco plants or plant cells modified to modulate gene expression levels thereby producing a plant or plant cell - such as a tobacco plant or plant cell - in which the expression level of a polypeptide is modulated within tissues of interest as compared to a control. The disclosed compositions and methods can be applied to any species of the genus *Nicotiana,* including *N. rustica* and *N. tabacum* (for example, LA B21, LN KY171, TI 1406, Basma, Galpao, Perique, Beinhart 1000-1, and Petico). Other species include *N. acaulis, N. acuminata, N. africana, N. alata, N. ameghinoi, N. amplexicaulis, N. arentsii, N. attenuata, N. azambujae, N. benavidesii, N. benthamiana, N. bigelovii, N. bonariensis, N. cavicola, N. clevelandii, N. cordifolia, N. corymbosa, N. debneyi, N. excelsior, N. forgetiana, N. fragrans, N. glauca, N. glutinosa, N. goodspeedii, N. gossei, N. hybrid, N. ingulba, N. kawakamii, N. knightiana, N. langsdorffii, N. linearis, N. longiflora, N. maritima, N. megalosiphon, N. miersii, N. noctiflora, N. nudicaulis, N. obtusifolia, N. occidentalis, N. occidentalis subsp. hesperis, N. otophora, N. paniculata, N. pauciflora, N. petunioides, N. plumbaginifolia, N. quadrivalvis, N. raimondii, N. repanda, N. rosulata, N. rosulata subsp. ingulba, N. rotundifolia, N. setchellii, N. simulans, N. solanifolia, N. spegazzinii, N. stocktonii, N. suaveolens, N. sylvestris, N. thyrsiflora, N. tomentosa, N. tomentosiformis, N. trigonophylla, N. umbratica, N. undulata, N. velutina, N. wigandioides, and N. x sanderae.*

The use of tobacco cultivars and elite tobacco cultivars is also contemplated herein. The transgenic, non-naturally occurring or mutant plant may therefore be a tobacco variety or elite tobacco cultivar that comprises one or more transgenes, or one or more genetic mutations or a combiantion thereof. The genetic mutation(s) (for example, one or more polymorphisms) can be mutations that do not exist naturally in the individual tobacco variety or tobacco cultivar (for example, elite tobacco cultivar) or can be genetic mutation(s) that do occur naturally provided that the mutation does not occur naturally in the individual tobacco variety or tobacco cultivar (for example, elite tobacco cultivar).

Particularly useful *Nicotiana tabacum* varieties include Burley type, dark type, flue-cured type, and Oriental type tobaccos. Non-limiting examples of varieties or cultivars are: BD 64, CC 101, CC 200, CC 27, CC 301, CC 400, CC 500, CC 600, CC 700, CC 800, CC 900, Coker 176, Coker 319, Coker 371 Gold, Coker 48, CD 263, DF911, DT 538 LC Galpao tobacco, GL 26H, GL 350, GL 600, GL 737, GL 939, GL 973, HB 04P, HB 04P LC, HB3307PLC, Hybrid 403LC, Hybrid 404LC, Hybrid 501 LC, K 149, K 326, K 346, K 358, K394, K 399, K 730, KDH 959, KT 200, KT204LC, KY10, KY14, KY 160, KY 17, KY 171, KY 907, KY907LC, KY14xL8 LC, Little Crittenden, McNair 373, McNair 944, msKY 14xL8, Narrow Leaf Madole, Narrow Leaf Madole LC, NBH 98, N-126, N-777LC, N-7371LC, NC 100, NC 102, NC 2000, NC 291, NC 297, NC 299, NC 3, NC 4, NC 5, NC 6, NC7, NC 606, NC 71, NC 72, NC 810, NC BH 129, NC 2002, Neal Smith Madole, OXFORD 207, PD 7302 LC, PD 7309 LC, PD 7312 LC, 'Perique' tobacco, PVH03, PVH09, PVH19, PVH50, PVH51, R 610, R 630, R 7-11, R 7-12, RG 17, RG 81, RG H51, RGH 4, RGH 51, RS 1410, Speight 168, Speight 172, Speight 179, Speight 210, Speight 220, Speight 225, Speight 227, Speight 234, Speight G-28, Speight G-70, Speight H-6, Speight H20, Speight NF3, TI 1406, TI 1269, TN 86, TN86LC, TN 90, TN 97, TN97LC, TN D94, TN D950, TR (Tom Rosson) Madole, VA 309, VA359, AA 37-1, B13P, Xanthi (Mitchell-Mor), Bel-W3, 79-615, Samsun Holmes NN, KTRDC number 2 Hybrid 49, Burley 21, KY8959, KY9, MD 609, PG01, PG04, PO1, PO2, PO3, RG11, RG 8, VA509, AS44, Banket A1, Basma Drama B84/31, Basma I Zichna ZP4/B, Basma Xanthi BX 2A, Batek, Besuki Jember, C104, Coker 347, Criollo Misionero, Delcrest, Djebel 81, DVH 405, Galpão Comum, HB04P, Hicks Broadleaf, Kabakulak Elassona, Kutsage E1, LA BU 21, NC 2326, NC 297, PVH 2110, Red Russian, Samsun, Saplak, Simmaba, Talgar 28, Wislica, Yayaldag, Prilep HC-72, Prilep P23, Prilep PB 156/1, Prilep P12-2/1, Yaka JK-48, Yaka JB 125/3, TI-1068, KDH-960, TI-1070, TW136, Basma, TKF 4028, L8, TKF 2002, GR141, Basma xanthi, GR149, GR153, Petit Havana. Low converter subvarieties of the above, even if not specifically identified herein, are also contemplated.

Embodiments are also directed to compositions and methods for producing mutant plants, non-naturally occurring plants, hybrid plants, or transgenic plants that have been modified to modulate the expression or activity of a polynucleotide(s) described herein (or any combination thereof as described herein). Advantageously, the mutant plants, non-naturally occurring plants, hybrid plants, or transgenic plants that are obtained may be similar or substantially the same in overall appearance to control plants. Various phenotypic characteristics such as degree of maturity, number of leaves per plant, stalk height, leaf insertion angle, leaf size (width and length), internode distance, and lamina-midrib ratio can be assessed by field observations.

One aspect relates to a seed of a mutant plant, a non-naturally occurring plant, a hybrid plant or a transgenic plant described herein. Preferably, the seed is a tobacco seed. A further aspect relates to pollen or an ovule of a mutant plant, a non-naturally occurring plant, a hybrid plant or a transgenic plant that is described herein. In addition, there is provided a mutant plant, a non-naturally occurring plant, a hybrid plant or a transgenic plant as described herein which further comprises a nucleic acid conferring male sterility.

Also provided is a tissue culture of regenerable cells of the mutant plant, non-naturally occurring plant, hybrid plant, or transgenic plant or a part thereof as described herein, which culture regenerates plants capable of expressing all the morphological and physiological characteristics of the parent. The regenerable cells include but are not limited to cells from leaves, pollen, embryos, cotyledons, hypocotyls, roots, root tips, anthers, flowers and a part thereof, ovules, shoots, stems, stalks, pith and capsules or callus or protoplasts derived therefrom.

### c. Modulation of alkaloid

One object is to provide mutant, transgenic or non-naturally occurring plants or parts thereof that exhibit modulated (eg. increased or reduced) levels of at least one alkaloid in the plant material, for example, in cured leaves. Suitably, mutant, transgenic or non-naturally occurring plants or parts thereof that exhibit modulated (eg. Increased or reduced) levels of at least nicotine, anatabine, caffeine, codeine, and/or morphine as compared to a control plant. Suitably, the mutant, transgenic or non-naturally occurring plants or parts thereof have substantially the same visual appearance as the control plant.

Accordingly, there is described herein mutant, transgenic or non-naturally occurring plants or parts thereof or plant cells that have modulated (eg. increased or reduced) levels of at least one alkaloid compared to control cells or control plants. The mutant, transgenic or non-naturally occurring plants or plant cells have been modified to modulate (eg. increase or reduce) the synthesis or activity of one or more of the polypeptides described herein by modulating the expression of one or more of the corresponding polynucleotide sequences described herein. Suitably, the modulated levels of at least one alkaloid are observed in at least the green leaves, suitably cured leaves. In certain embodiments, the level of total alkaloids in the plant - such as the green leaves, suitably cured leaves or cured tobacco - may be modulated (eg. increased or reduced). In certain embodiments, the level of nicotine, anatabine, caffeine, codeine, or morphine in the plant - such as the green leaves, suitably cured leaves or cured tobacco - may be modulated (eg. increased or reduced).

A further aspect, relates to a mutant, non-naturally occurring or transgenic plant or cell, wherein the expression of or the activity of one or more of the polypeptides described herein is modulated (eg. increased or reduced) and a part of the plant (for example, the green leaves, suitably cured leaves or cured tobacco) have reduced levels of at least one alkaloid of at least 5% therein as compared to a control plant in which the expression or the activity of said polypeptide(s) has not been modulated. In certain embodiments, the level of nicotine, anatabine, caffeine, codeine, or morphine in the plant - such as the green leaves, suitably cured leaves or cured tobacco - may be modulated (eg. increased or reduced), for example, by at least about 5%.

A still further aspect, relates to a cured plant material - such as cured leaf or cured tobacco - derived or derivable from a mutant, non-naturally occurring or transgenic plant or cell, wherein expression of one or more of the polynucleotides described herein or the activity of the protein encoded thereby is modulated and wherein the level of at least one alkaloid is modulated by at least 5% as compared to a control plant.

Suitably the visual appearance of said plant or part thereof (for example, leaf) is substantially the same as the control plant. Suitably, the plant is a tobacco plant or a coffee plant.

Embodiments are also directed to compositions and methods for producing mutant, non-naturally occurring or transgenic plants or plant cells that have been modified to modulate the expression or activity of the one or more of the polynucleotides or polypeptides described herein which can result in plants or plant components (for example, leaves - such as green leaves or cured leaves - or tobacco) or plant cells with modulated alkaloid content.

Advantageously, the mutant, non-naturally occurring or transgenic plants that are obtained according to the methods described herein are similar or substantially the same in visual appearance to the control plants. In one embodiment, the leaf weight of the mutant, non-naturally occurring or transgenic plant is substantially the same as the control plant. In one embodiment, the leaf number of the mutant, non-naturally occurring or transgenic plant is substantially the same as the control plant. In one embodiment, the leaf weight and the leaf number of the mutant, non-naturally occurring or transgenic plant is substantially the same as the control plant. In one embodiment, the stalk height of the mutant, non-naturally occurring or transgenic plants is substantially the same as the control plants at, for example, one, two or three or more months after field transplant or 10, 20, 30 or 36 or more days after topping. For example, the stalk height of the mutant, non-naturally occurring or transgenic plants is not less than the stalk height of the control plants. In another embodiment, the chlorophyll content of the mutant, non-naturally occurring or transgenic plants is substantially the same as the control plants. In another embodiment, the stalk height of the mutant, non-naturally occurring or transgenic plants is substantially the same as the control plants and the chlorophyll content of the mutant, non-naturally occurring or transgenic plants is substantially the same as the control plants. In other embodiments, the size or form or number or colouration of the leaves of the mutant, non-naturally occurring or transgenic plants is substantially the same as the control plants. Suitably, the plant is a tobacco plant or a coffee plant.

In another aspect, there is provided a method for modulating (eg. increasing or reducing) the amount of at least one alkaloid in at least a part of a plant (for example, the leaves - such as cured leaves - or in tobacco), comprising the steps of: (i) modulating (eg. increasing or reducing) the expression or activity of an one or more of the polypeptides described herein (or any combination thereof as described herein), suitably, wherein the polypeptide(s) is encoded by the corresponding polynucleotide sequence described herein; (ii) measuring the level of the at least one alkaloid in at least a part (for example, the leaves - such as cured leaves - or tobacco or in smoke) of the mutant, non-naturally occurring or transgenic plant obtained in step (i); and (iii) identifying a mutant, non-naturally occurring or transgenic plant in which the level of the at least one alkaloid therein has been modulated (*eg.* increased or reduced) in comparison to a control plant. Suitably, the visual appearance of said mutant, non-naturally occurring or transgenic plant is substantially the same as the control plant. Suitably, the plant is a tobacco plant or a coffee plant.

In another aspect, there is provided a method for modulating (*eg*. increasing or reducing) the amount of at least one alkaloid in at least a part of cured plant material - such as cured leaf - comprising the steps of: (i) modulating (*eg*. increasing or reducing) the expression or activity of an one or more of the polypeptides (or any combination thereof as described herein), suitably, wherein the polypeptide(s) is encoded by the corresponding polynucleotide sequence described herein; (ii) harvesting plant material - such as one or more of the leaves - and curing for a period of time; (iii) measuring the level of the at least one alkaloid in at least a part of the cured plant material obtained in step (ii) or during step (ii); and (iv) identifying cured plant material in which the level of the at least one alkaloid therein has been modulated (*eg*. increased or reduced) in comparison to a control plant.

An increase in expression as compared to the control may be from about 5 % to about 100 %, or an increase of at least 10 %, at least 20 %, at least 25 %, at least 30 %, at least 40 %, at least 50 %, at least 60 %, at least 70 %, at least 75 %, at least 80 %, at least 90 %, at least 95 %, at least 98 %, or 100 % or more - such as 200%, 300%, 500%, 1000% or more, which includes an increase in transcriptional activity or polynucleotide expression or polypeptide expression or a combination thereof.

An increase in activity as compared to a control may be from about 5 % to about 100 %, or an increase of at least 10 %, at least 20 %, at least 25 %, at least 30 %, at least 40 %, at least 50 %, at least 60 %, at least 70 %, at least 75 %, at least 80 %, at least 90 %, at least 95 %, at least 98 %, or 100 % or more - such as 200%, 300%, 500%, 1000% or more.

A reduction in expression as compared to a control may be from about 5 % to about 100 %, or a reduction of at least 10 %, at least 20 %, at least 25 %, at least 30 %, at least 40 %, at least 50 %, at least 60 %, at least 70 %, at least 75 %, at least 80 %, at least 90 %, at least 95 %, at least 98 %, or 100 %, which includes a reduction in transcriptional activity or polynucleotide expression or polypeptide expression or a combination thereof.

A reduction in activity as compared to a control may be from about 5 % to about 100 %, or a reduction of at least 10 %, at least 20 %, at least 25 %, at least 30 %, at least 40 %, at least 50 %, at least 60 %, at least 70 %, at least 75 %, at least 80 %, at least 90 %, at least 95 %, at least 98 %, or 100 %.

Polynucleotides and recombinant constructs described herein can be used to modulate the expression of the ABC transporters described herein in a plant species of interest, suitably tobacco.

A number of polynucleotide based methods can be used to increase gene expression in plants and plant cells. By way of example, a construct, vector or expression vector that is compatible with the plant to be transformed can be prepared which comprises the gene of interest together with an upstream promoter that is capable of overexpressing the gene in the plant or plant cell. Exemplary promoters are described herein. Following transformation and when grown under suitable conditions, the promoter can drive expression in order to modulate (for example, reduce) the levels of this enzyme in the plant, or in a specific tissue thereof. In one exemplary embodiment, a vector carrying one or more polynucleotides described herein (or any combination thereof as described herein) is generated to overexpress the gene in a plant or plant cell. The vector carries a suitable promoter - such as the cauliflower mosaic virus CaMV 35S promoter - upstream of the transgene driving its constitutive expression in all tissues of the plant.

The vector also carries an antibiotic resistance gene in order to confer selection of the transformed calli and cell lines.

The expression of sequences from promoters can be enhanced by including expression control sequences, including enhancers, chromatin activating elements, transcription factor responsive elements and the like. Such control sequences may be constitutive, and upregulate transcription in a universal manner; or they may be facultative, and upregulate transcription in response to specific signals. Signals associated with senescence and signals which are active during the curing procedure are specifically indicated.

Various embodiments are therefore directed to methods for modulating (for example, increasing or reducing) the expression level of one or more polynucleotides described herein (or any combination thereof as described herein) by integrating multiple copies of the polynucleotide into a plant genome, comprising: transforming a plant cell host with an expression vector that comprises a promoter operably-linked to one or more polynucleotides described herein. The polypeptide encoded by a recombinant polynucleotide can be a native polypeptide, or can be heterologous to the cell.

### d. Breeding

A plant carrying a mutant allele of one or more polynucleotides described herein (or any combination thereof as described herein) can be used in a plant breeding program to create useful lines, varieties and hybrids. In particular, the mutant allele is introgressed into the commercially important varieties described above. Thus, methods for breeding plants are provided, that comprise crossing a mutant plant, a non-naturally occurring plant or a transgenic plant as described herein with a plant comprising a different genetic identity. The method may further comprise crossing the progeny plant with another plant, and optionally repeating the crossing until a progeny with the desirable genetic traits or genetic background is obtained. One purpose served by such breeding methods is to introduce a desirable genetic trait into other varieties, breeding lines, hybrids or cultivars, particularly those that are of commercial interest. Another purpose is to facilitate stacking of genetic modifications of different genes in a single plant variety, lines, hybrids or cultivars. Intraspecific as well as interspecific matings are contemplated. The progeny plants that arise from such crosses, also referred to as breeding lines, are examples of non-naturally occurring plants of the invention.

In one embodiment, a method is provided for producing a non-naturally occurring plant comprising: (a) crossing a mutant or transgenic plant with a second plant to yield progeny tobacco seed; (b) growing the progeny tobacco seed, under plant growth conditions, to yield the non-naturally occurring plant. The method may further comprises: (c) crossing the previous generation of non-naturally occurring plant with itself or another plant to yield progeny tobacco seed; (d) growing the progeny tobacco seed of step (c) under plant growth conditions, to yield additional non-naturally occurring plants; and (e) repeating the crossing and growing steps of (c) and (d) multiple times to generate further generations of non-naturally occurring plants. The method may optionally comprises prior to step (a), a step of providing a parent plant which comprises a genetic identity that is characterized and that is not identical to the mutant or transgenic plant. In some embodiments, depending on the breeding program, the crossing and growing steps are repeated from 0 to 2 times, from 0 to 3 times, from 0 to 4 times, 0 to 5 times, from 0 to 6 times, from 0 to 7 times, from 0 to 8 times, from 0 to 9 times or from 0 to 10 times, in order to generate generations of non-naturally occurring plants. Backcrossing is an example of such a method wherein a progeny is crossed with one of its parents or another plant genetically similar to its parent, in order to obtain a progeny plant in the next generation that has a genetic identity which is closer to that of one of the parents. Techniques for plant breeding, particularly plant breeding, are well known and can be used in the methods of the invention. The invention further provides non-naturally occurring plants produced by these methods. Certain embodiments exclude the step of selecting a plant.

In some embodiments of the methods described herein, lines resulting from breeding and screening for variant genes are evaluated in the field using standard field procedures. Control genotypes including the original unmutagenized parent are included and entries are arranged in the field in a randomized complete block design or other appropriate field design. For tobacco, standard agronomic practices are used, for example, the tobacco is harvested, weighed, and sampled for chemical and other common testing before and during curing. Statistical analyses of the data are performed to confirm the similarity of the selected lines to the parental line. Cytogenetic analyses of the selected plants are optionally performed to confirm the chromosome complement and chromosome pairing relationships.

DNA fingerprinting, single nucleotide polymorphism, microsatellite markers, or similar technologies may be used in a marker-assisted selection (MAS) breeding program to transfer or breed mutant alleles of a gene into other tobaccos, as described herein. For example, a breeder can create segregating populations from hybridizations of a genotype containing a mutant allele with an agronomically desirable genotype. Plants in the F2 or backcross generations can be screened using a marker developed from a genomic sequence or a fragment thereof, using one of the techniques listed herein. Plants identified as possessing the mutant allele can be backcrossed or self-pollinated to create a second population to be screened. Depending on the expected inheritance pattern or the MAS technology used, it may be necessary to self-pollinate the selected plants before each cycle of backcrossing to aid identification of the desired individual plants. Backcrossing or other breeding procedure can be repeated until the desired phenotype of the recurrent parent is recovered.

According to the disclosure, in a breeding program, successful crosses yield F1 plants that are fertile. Selected F1 plants can be crossed with one of the parents, and the first backcross generation plants are self-pollinated to produce a population that is again screened for variant gene expression (for example, the null version of the the gene). The process of backcrossing, self-pollination, and screening is repeated, for example, at least 4 times until the final screening produces a plant that is fertile and reasonably similar to the recurrent parent. This plant, if desired, is self-pollinated and the progeny are subsequently screened again to confirm that the plant exhibits variant gene expression. In some embodiments, a plant population in the F2 generation is screened for variant gene expression, for example, a plant is identified that fails to express a polypeptide due to the absence of the gene according to standard methods, for example, by using a PCR method with primers based upon the nucleotide sequence information for the polynucleotide(s) described herein (or any combination thereof as described herein).

Hybrid tobacco varieties can be produced by preventing self-pollination of female parent plants (that is, seed parents) of a first variety, permitting pollen from male parent plants of a second variety to fertilize the female parent plants, and allowing F1 hybrid seeds to form on the female plants. Self-pollination of female plants can be prevented by emasculating the flowers at an early stage of flower development. Alternatively, pollen formation can be prevented on the female parent plants using a form of male sterility. For example, male sterility can be produced by cytoplasmic male sterility (CMS), or transgenic male sterility wherein a transgene inhibits microsporogenesis and/or pollen formation, or self-incompatibility. Female parent plants containing CMS are particularly useful. In embodiments in which the female parent plants are CMS, pollen is harvested from male fertile plants and applied manually to the stigmas of CMS female parent plants, and the resulting F1 seed is harvested.

Varieties and lines described herein can be used to form single-cross tobacco F1 hybrids. In such embodiments, the plants of the parent varieties can be grown as substantially homogeneous adjoining populations to facilitate natural cross-pollination from the male parent plants to the female parent plants. The F1 seed formed on the female parent plants is selectively harvested by conventional means. One also can grow the two parent plant varieties in bulk and harvest a blend of F1 hybrid seed formed on the female parent and seed formed upon the male parent as the result of self-pollination. Alternatively, three-way crosses can be carried out wherein a single-cross F1 hybrid is used as a female parent and is crossed with a different male parent. As another alternative, double-cross hybrids can be created wherein the F1 progeny of two different single-crosses are themselves crossed.

A population of mutant, non-naturally occurring or transgenic plants can be screened or selected for those members of the population that have a desired trait or phenotype. For example, a population of progeny of a single transformation event can be screened for those plants having a desired level of expression or activity of the polypeptide(s) encoded thereby. Physical and biochemical methods can be used to identify expression or activity levels. These include Southern analysis or PCR amplification for detection of a polynucleotide; Northern blots, S1 RNase protection, primer-extension, or RT-PCR amplification for detecting RNA transcripts; enzymatic assays for detecting enzyme or ribozyme activity of polypeptides and polynucleotides; and protein gel electrophoresis, Western blots, immunoprecipitation, and enzyme-linked immunoassays to detect polypeptides. Other techniques such as in situ hybridization, enzyme staining, and immunostaining and enzyme assays also can be used to detect the presence or expression or activity of polypeptides or polynucleotides.

Mutant, non-naturally occurring or transgenic plant cells and plants are described herein comprising one or more recombinant polynucleotides, one or more polynucleotide constructs, one or more double-stranded RNAs, one or more conjugates or one or more vectors/expression vectors.

### 6. Modification of other genes

Without limitation, the plants described herein may be modified either before or after the expression or activity has been modulated according to the present invention. One or more of the following genetic modifications can be present in the mutant, non-naturally occurring or transgenic plants. In embodiments, one or more genes that are involved in the nicotine synthesis pathway is modified resulting in plants or parts of plants (such as leaves or tobacco) that when cured, produces modulated levels of nicotine than control plants or parts thereof. The nicotine synthesis genes include *A622, NBB1, PMT* (e.g. *PMT1, PMT2, PMT3, PMT4), QPT, MPO1,* and *MPO2.* In embodiments, one or more genes that are involved in the control of the level of one or more alkaloids is modified resulting in plants or parts of plants that produce modulated levels of alkaloid than control plants or parts thereof. Alkaloid level controlling genes include *BBLa* and *BBLb* (SEQ ID NO: 4 in WO_20061109197_A2; SEQ ID NO: 2 in US_2008/0120737_A1), *JRE5L1 and JRE5L2* (SEQ ID NO: 13 in US_2012/0284816_A1), *MATE1 and MATE 2* (SEQ ID NO: 28 in WO_2015/085299_A1), *MYC2a and MYC2b* (SEQ ID NO: 4 in US_2011/0047660_A1), *nic1 and nic2* (SEQ ID NO: 13 in US_2012/0284816_A1), and *NUP1* and *NUP2* (SEQ ID NO: 8 in WO_2015/085299_A1).

In the passages below, an increase in the expression or activity of a gene or its product (e.g. a polypeptide) is also referred to as a "gain of function" (abbreviated to "gof"), and a reduction in the expression or activity of a gene or its product (e.g. a polypeptide) is also referred to as a "reduction of function" (abbreviated to "rof"). A reduction of function also includes a complete loss of function ("lof"), e.g. by complete silencing, "switch off', or other total absence of expression from the gene or activity of its product(s).

The expression or activity of some of the genes disclosed herein is directly (i.e. positively) correlated with the level of one or more alkaloids in at least a part of a plant as described herein. Accordingly, modification resulting in a reduction of expression or activity (reduction of function - "rof') of these genes also results in a reduction in the level of the one or more alkaloids in that part of the plant. Accordingly, modification resulting in an increase in expression or activity (gain of function - "gof") of these genes also results in an increase in the level of the one or more alkaloids in that part of the plant.

The expression or activity of some of the genes disclosed herein is inversely (i.e. negatively) correlated with the level of one or more alkaloids in at least a part of a plant as described herein. Accordingly, modification resulting in a reduction of function (rof) of these genes conversely results in an increase in the level of the one or more alkaloids in that part of the plant.

Accordingly, modification resulting in a gain of function (gof) of these genes conversely results in a reduction in the level of the one or more alkaloids in that part of the plant.

Therefore, in order to increase the level of one or more alkaloids, modifications are selected to increase the expression or activity of genes directly correlated with the level of one or more alkaloids and decrease the expression or activity of genes inversely correlated with the level of one or more alkaloids. Conversely, in order the decrease the level of one or more alkaloids, modifications are selected to increase the expression or activity of genes inversely correlated with the level of one or more alkaloids and decrease the expression or activity of genes directly correlated with the level of one or more alkaloids.

When modifications modulate the expression or activity of more than one gene, it is advantageous to take care that the modifications modulate the level of the one or more alkaloids in the same direction; i.e. either the modifications modulate the expression or activity of two or more genes, all of which result in an increase in the level of the one or more alkaloids, or the modifications modulate expression or activity of two or more genes, all of which result in a reduction in the level of the one or more alkaloids. This requires modifications to cause either:
i. gain of function only of genes the expression or activity of which is **directly** correlated with the level of at least one alkaloid and reduction of function only of genes the expression of which is **inversely** correlated with the level of at least one alkaloid (thus increasing the level of the at least one alkaloid); or
ii. gain of function only of genes the expression or activity of which is **inversely** correlated with the level of at least one alkaloid and reduction of function only of genes the expression of which is **directly** correlated with the level of at least one alkaloid (thus reducing the level of the at least one alkaloid).

### a. Increasing the level of one or more alkaloids

Accordingly, in one embodiment, the level of at least one alkaloid is **increased** in at least a part of a plant by modification(s) resulting in an increase or reduction in the expression or activity of two or more genes. Suitably, the modification(s) result in a gain or reduction of function (as indicated) in one of the following gene pairs: *NtABCG1-T (rof)* and *A622 (gof); NtABCG1-T (rof)* and *BBLa (gof); NtABCG1-T (rof)* and *BBLb (gof); NtABCG1-T (rof)* and *JRE5L1(gof); NtABCG1-T (rof)* and *JRE5L2(gof); NtABCG1-T (rof)* and *MATE1 (rof); NtABCG1-T (rof)* and *MATE 2 (rof); NtABCG1-T (rof)* and *MPO1 (gof); NtABCG1-T (rof)* and *MPO2 (gof); NtABCG1-T (rof)* and *MYC2a (gof); NtABCG1-T (rof)* and *MYC2b (gof); NtABCG1-T (rof)* and *NBB1 (gof); NtABCG1-T (rof)* and *nic1 (gof); NtABCG1-T (rof)* and *nic2 (gof); NtABCG1-T (rof)* and *NUP1 (gof); NtABCG1-T (rof)* and *NUP2 (gof); NtABCG1-T (rof)* and *PMT1 (gof); NtABCG1-T (rof)* and *PMT2 (gof); NtABCG1-T (rof)* and *PMT3 (gof); NtABCG1-T (rof)* and *PMT4 (gof); NtABCG1-T (rof)* and *QPT (gof);; NtABCG1-S (rof)* and *A622 (gof); NtABCG1-S (rof)* and *BBLa (gof); NtABCG1-S (rof)* and *BBLb (gof); NtABCG1-S (rof)* and *JRESL1(gof); NtABCG1-S (rof)* and *JRE5L2(gof); NtABCG1-S (rof)* and *MATE1 (rof); NtABCG1-S (rof)* and *MATE 2 (rof); NtABCG1-S (rof)* and *MPO1 (gof); NtABCG1-S (rof)* and *MPO2 (gof); NtABCG1-S (rof)* and *MYC2a (gof); NtABCG1-S (rof)* and *MYC2b (gof); NtABCG1-S (rof)* and *NBB1 (gof); NtABCG1-S (rof)* and *nic1 (gof); NtABCG1-S (rof)* and *nic2 (gof); NtABCG1-S (rof)* and *NUP1 (gof); NtABCG1-S (rof)* and *NUP2 (gof); NtABCG1-S (rof)* and *PMT1 (gof); NtABCG1-S (rof)* and *PMT2 (gof); NtABCG1-S (rof)* and *PMT3 (gof); NtABCG1-S (rof)* and *PMT4 (gof); NtABCG1-S (rof)* and *QPT (gof).*

Alternatively or in addition, the level of at least one alkaloid is **increased** in at least a part of a plant by modification(s) resulting in an increase or reduction in the expression or activity of three or more genes. Suitably, the modification(s) result in a gain or reduction of function (as indicated) in one of the following gene triplets: *NtABCG1-T (rof), A622 (gof),* and *NBB (gof); NtABCG1-T (rof), A622 (gof),* and *QPT (gof); NtABCG1-T (rof), QPT (gof),* and *PMT1 (gof); NtABCG1-T (rof), QPT (gof),* and *PMT2 (gof); NtABCG1-T (rof), QPT (gof),* and *PMT3 (gof); NtABCG1-T (rof), QPT (gof),* and *PMT4 (gof); NtABCG1-T (rof), MYC2a (gof),* and *PMT1 (gof); NtABCG1-T (rof), MYC2a (gof),* and *PMT2 (gof); NtABCG1-T (rof), MYC2a (gof),* and *PMT3 (gof); NtABCG1-T (rof), MYC2a (gof),* and *PMT4 (gof); NtABCG1-T (rof), MYC2b (gof),* and *PMT1 (gof); NtABCG1-T (rof), MYC2b (gof),* and *PMT2 (gof); NtABCG1-T (rof), MYC2b (gof),* and *PMT3 (gof); NtABCG1-T (rof), MYC2b (gof),* and *PMT4 (gof); NtABCG1-T (rof), MPO1 (gof),* and *PMT1 (gof); NtABCG1-T (rof), MPO1 (gof),* and *PMT2 (gof); NtABCG1-T (rof), MPO1 (gof), and PMT3 (gof); NtABCG1-T (rof), MPO1 (gof), and PMT4 (gof); NtABCG1-T (rof), MPO1 (gof),* and *A622 (gof); NtABCG1-T (rof), MPO1 (gof),* and *NBB1 (gof); NtABCG1-T (rof), MPO1 (gof),* and *QPT (gof); NtABCG1-T (rof), MPO2 (gof), and PMT1 (gof); NtABCG1-T (rof), MPO2 (gof),* and *PMT2 (gof); NtABCG1-T (rof), MPO2 (gof),* and *PMT3 (gof); NtABCG1-T (rof), MPO2 (gof),* and *PMT4 (gof); NtABCG1-T (rof), MPO2 (gof),* and *A622 (gof); NtABCG1-T (rof), MPO2 (gof), and NBB1 (gof); NtABCG1-T (rof), MPO2 (gof),* and *QPT (gof);; NtABCG1-S (rof), A622 (gof),* and *NBB (gof); NtABCG1-S (rof), A622 (gof),* and *QPT (gof); NtABCG1-S (rof), QPT (gof),* and *PMT1 (gof); NtABCG1-S (rof), QPT (gof),* and *PMT2 (gof); NtABCG1-S (rof), QPT (gof),* and *PMT3 (gof); NtABCG1-S (rof), QPT (gof),* and *PMT4 (gof); NtABCG1-S (rof), MYC2a (gof),* and *PMT1 (gof); NtABCG1-S (rof), MYC2a (gof),* and *PMT2 (gof); NtABCG1-S (rof), MYC2a (gof),* and *PMT3 (gof); NtABCG1-S (rof), MYC2a (gof),* and *PMT4 (gof); NtABCG1-S (rof), MYC2b (gof),* and *PMT1 (gof); NtABCG1-S (rof), MYC2b (gof),* and *PMT2 (gof); NtABCG1-S (rof), MYC2b (gof),* and *PMT3 (gof); NtABCG1-S (rof), MYC2b (gof),* and *PMT4 (gof); NtABCG1-S (rof), MPO1 (gof),* and *PMT1 (gof); NtABCG1-S (rof), MPO1 (gof),* and *PMT2 (gof); NtABCG1-S (rof), MPO1 (gof),* and *PMT3 (gof); NtABCG1*-*S (rof), MPO1 (gof), and PMT4 (gof); NtABCG1*-*S (rof), MPO1 (gof), and A622 (gof); NtABCG1-S (rof), MPO1 (gof), and NBB1 (gof); NtABCG1-S (rof), MPO1 (gof),* and *QPT (gof); NtABCG1-S (rof), MPO2 (gof),* and *PMT1 (gof); NtABCG1-S (rof), MPO2 (gof),* and *PMT2 (gof); NtABCG1-S (rof), MPO2 (gof),* and *PMT3 (gof); NtABCG1-S (rof), MPO2 (gof),* and *PMT4 (gof); NtABCG1-S (rof), MPO2 (gof), and A622 (gof); NtABCG1-S (rof), MPO2 (gof),* and *NBB1 (gof); NtABCG1-S (rof), MPO2 (gof),* and *QPT (gof);*

### b. Reducing the level of one or more alkaloids

In other embodiments, the level of at least one alkaloid is **reduced** in at least a part of a plant by modification(s) resulting in an increase or reduction in the expression or activity of two or more genes. Suitably, the modification(s) result in a gain or reduction of function (as indicated) in one of the following gene pairs: *NtABCG1*-*T (gof) and A622 (rof); NtABCG1*-*T (gof) and BBLa (rof); NtABCG1-T (gof)* and *BBLb (rof); NtABCG1-T (gof)* and *JRE5L1(rof); NtABCG1-T (gof)* and *JRE5L2(rof); NtABCG1-T (gof)* and *MATE1 (gof); NtABCG1-T (gof)* and *MATE 2 (gof); NtABCG1-T (gof)* and *MPO1 (rof); NtABCG1-T (gof)* and *MPO2 (rof); NtABCG1-T (gof)* and *MYC2a (rof); NtABCG1-T (gof)* and *MYC2b (rof); NtABCG1-T (gof) and NBB1 (rof); NtABCG1-T (gof)* and *nic1 (rof); NtABCG1-T (gof)* and *nic2 (rof); NtABCG1-T (gof)* and *NUP1 (rof); NtABCG1-T (gof)* and *NUP2 (rof); NtABCG1-T (gof)* and *PMT1 (rof); NtABCG1-T (gof)* and *PMT2 (rof); NtABCG1-T (gof)* and *PMT3 (rof); NtABCG1-T (gof)* and *PMT4 (rof); NtABCG1-T (gof)* and *QPT (rof);; NtABCG1-S (gof)* and *A622 (rof); NtABCG1-S (gof)* and *BBLa (rof); NtABCG1-S (gof)* and *BBLb (rof); NtABCG1-S (gof)* and *JRE5L1(rof); NtABCG1-S (gof)* and *JRE5L2(rof); NtABCG1-S (gof)* and *MATE1 (gof); NtABCG1-S (gof)* and *MATE 2 (gof); NtABCG1-S (gof)* and *MPO1 (rof); NtABCG1-S (gof)* and *MPO2 (rof); NtABCG1-S (gof)* and *MYC2a (rof); NtABCG1-S (gof)* and *MYC2b (rof); NtABCG1-S (gof)* and *NBB1 (rof); NtABCG1-S (gof)* and *nic1 (rof); NtABCG1-S (gof)* and *nic2 (rof); NtABCG1-S (gof)* and *NUP1 (rof); NtABCG1-S (gof)* and *NUP2 (rof); NtABCG1-S (gof)* and *PMT1 (rof); NtABCG1-S (gof)* and *PMT2 (rof); NtABCG1-S (gof)* and *PMT3 (rof); NtABCG1-S (gof)* and *PMT4 (rof); NtABCG1-S (gof)* and *QPT (rof).*

Alternatively or in addition, the level of at least one alkaloid is **reduced** in at least a part of a plant by modification(s) resulting in an increase or reduction in the expression or activity of three or more genes. Suitably, the modification(s) result in a gain or reduction of function (as indicated) in one of the following gene triplets: *NtABCG1*-*T (gof), A622 (rof),* and *NBB (rof); NtABCG1-T (gof), A622 (rof),* and *QPT (rof); NtABCG1-T (gof), QPT (rof),* and *PMT1 (rof); NtABCG1-T (gof), QPT (rof),* and *PMT2 (rof); NtABCG1-T (gof), QPT (rof),* and *PMT3 (rof); NtABCG1-T (gof), QPT (rof),* and *PMT4 (rof); NtABCG1-T (gof), MYC2a (rof),* and *PMT1 (rof); NtABCG1-T (gof), MYC2a (rof),* and *PMT2 (rof); NtABCG1-T (gof), MYC2a (rof),* and *PMT3 (rof); NtABCG1-T (gof), MYC2a (rof),* and *PMT4 (rof); NtABCG1-T (gof), MYC2b (rof),* and *PMT1 (rof); NtABCG1-T (gof), MYC2b (rof),* and *PMT2 (rof); NtABCG1-T (gof), MYC2b (rof),* and *PMT3 (rof); NtABCG1-T (gof), MYC2b (rof),* and *PMT4 (rof); NtABCG1-T (gof), MPO1 (rof),* and *PMT1 (rof); NtABCG1-T (gof), MPO1 (rof),* and *PMT2 (rof); NtABCG1-T (gof), MPO1 (rof),* and *PMT3 (rof); NtABCG1-T (gof), MPO1 (rof),* and *PMT4 (rof); NtABCG1-T (gof), MPO1 (rof),* and *A622 (rof); NtABCG1-T(gof), MPO1 (rof),* and *NBB1 (rof); NtABCG1-T (gof), MPO1 (rof),* and *QPT (rof); NtABCG1-T (gof), MPO2 (rof),* and *PMT1 (rof); NtABCG1-T (gof), MPO2 (rof),* and *PMT2 (rof); NtABCG1*-*T (gof), MPO2 (rof),* and *PMT3 (rof); NtABCG1-T (gof), MPO2 (rof),* and *PMT4 (rof); NtABCG1-T (gof), MPO2 (rof),* and *A622 (rof); NtABCG1*-*T (gof), MPO2 (rof), and NBB1 (rof); NtABCG1*-*T (gof), MPO2 (rof),* and *QPT (rof);; NtABCG1-S (gof), A622 (rof),* and *NBB (rof); NtABCG1-S (gof), A622 (rof),* and *QPT (rof); NtABCG1-S (gof), QPT (rof),* and *PMT1 (rof); NtABCG1-S (gof), QPT (rof),* and *PMT2 (rof); NtABCG1-S (gof), QPT (rof),* and *PMT3 (rof); NtABCG1-S (gof), QPT (rof),* and *PMT4 (rof); NtABCG1-S (gof), MYC2a (rof),* and *PMT1 (rof); NtABCG1-S (gof), MYC2a (rof),* and *PMT2 (rof); NtABCG1-S (gof), MYC2a (rof),* and *PMT3 (rof); NtABCG1-S (gof), MYC2a (rof),* and *PMT4 (rof); NtABCG1-S (gof), MYC2b (rof),* and *PMT1 (rof); NtABCG1-S (gof), MYC2b (rof),* and *PMT2 (rof); NtABCG1-S (gof), MYC2b (rof),* and *PMT3 (rof); NtABCG1-S (gof), MYC2b (rof),* and *PMT4 (rof); NtABCG1-S (gof), MPO1 (rof),* and *PMT1 (rof); NtABCG1-S (gof), MPO1 (rof),* and *PMT2 (rof); NtABCG1-S (gof), MPO1 (rof),* and *PMT3 (rof); NtABCG1-S (gof), MPO1 (rof), and PMT4 (rof); NtABCG1-S (gof), MPO1 (rof), and A622 (rof); NtABCG1-S (gof), MPO1 (rof),* and *NBB1 (rof); NtABCG1-S (gof), MPO1 (rof),* and *QPT (rof); NtABCG1-S (gof), MPO2 (rof),* and *PMT1 (rof); NtABCG1-S (gof), MPO2 (rof),* and *PMT2 (rof); NtABCG1-S (gof), MPO2 (rof),* and *PMT3 (rof); NtABCG1-S (gof), MPO2 (rof),* and *PMT4 (rof); NtABCG1-S (gof), MPO2 (rof), and A622 (rof); NtABCG1-S (gof), MPO2 (rof), and NBB1 (rof); NtABCG1-S (gof), MPO2 (rof),* and *QPT (rof);*

The modulating effect may be synergistic with one or more of the modifications described herein that modulate the levels of one or more alkaloids.

### c. Other genes

In one embodiment, one or more genes that are involved in the conversion of nitrogenous metabolic intermediates is modified resulting in plants or parts of plants (such as leaves or tobacco) that when cured, produces lower levels of at least one tobacco-specific nitrosamine than control plants or parts thereof. Non-limiting examples of genes that can be modified include genes encoding a nicotine demethylase, such as CYP82E4, CYP82E5 and CYP82E10 which participate in the conversion of nicotine to nornicotine and are described in WO2006091194, WO2008070274, WO2009064771 and PCT/US2011/021088 and as described in detail herein. In another embodiment, one or more genes that are involved in heavy metal uptake or heavy metal transport are modified resulting in plants or parts of plants (such as leaves) having a lower heavy metal content than control plants or parts thereof without the modification(s). Non-limiting examples include genes in the family of multidrug resistance associated proteins, the family of cation diffusion facilitators (CDF), the family of Zrt-, Irt-like proteins (ZIP), the family of cation exchangers (CAX), the family of copper transporters (COPT), the family of heavy-metal P-type ATPases (for example, HMAs, as described in WO2009074325), the family of homologs of natural resistance-associated macrophage proteins (NRAMP), and other members of the family of ATP-binding cassette (ABC) transporters (for example, MRPs), as described in WO2012/028309, which participate in transport of heavy metals, such as cadmium. The term heavy metal as used herein includes transition metals. Examples of other modifications include herbicide tolerance, for example, glyphosate is an active ingredient of many broad spectrum herbicides. Glyphosate resistant transgenic plants have been developed by transferring the *aroA* gene (a glyphosate EPSP synthetase from *Salmonella typhimurium* and *E.coli*)*.* Sulphonylurea resistant plants have been produced by transforming the mutant *ALS* (acetolactate synthetase) gene from Arabidopsis. OB protein of photosystem II from mutant *Amaranthus hybridus* has been transferred in to plants to produce atrazine resistant transgenic plants; and bromoxynil resistant transgenic plants have been produced by incorporating the *bxn* gene from the bacterium *Klebsiella pneumoniae.* Another exemplary modification results in plants that are resistant to insects. *Bacillus thuringiensis* (Bt) toxins can provide an effective way of delaying the emergence of Bt-resistant pests, as recently illustrated in broccoli where pyramided *cry1Ac* and *cry1C* Bt genes controlled diamondback moths resistant to either single protein and significantly delayed the evolution of resistant insects. Another exemplary modification results in plants that are resistant to diseases caused by pathogens (for example, viruses, bacteria, fungi). Plants expressing the *Xa21* gene (resistance to bacterial blight) with plants expressing both a Bt fusion gene and a chitinase gene (resistance to yellow stem borer and tolerance to sheath) have been engineered. Another exemplary modification results in altered reproductive capability, such as male sterility. Another exemplary modification results in plants that are tolerant to abiotic stress (for example, drought, temperature, salinity), and tolerant transgenic plants have been produced by transferring acyl glycerol phosphate enzyme from Arabidopsis; genes coding mannitol dehydrogenase and sorbitol dehydrogenase which are involved in synthesis of mannitol and sorbitol improve drought resistance. Other exemplary modifications can result in plants with improved storage proteins and oils, plants with enhanced photosynthetic efficiency, plants with prolonged shelf life, plants with enhanced carbohydrate content, and plants resistant to fungi; plants encoding an enzyme involved in the biosynthesis of alkaloids. Transgenic plants in which the expression of S-adenosyl-L-methionine (SAM) and/or cystathionine gamma-synthase (CGS) has been modulated are also contemplated.

One or more such traits may be introgressed into the mutant, non-naturally occuring or transgenic plants from another cultivar or may be directly transformed into it. The introgression of the trait(s) into the mutant, non-naturally occuring or transgenic plants of the invention maybe achieved by any method of plant breeding known in the art, for example, pedigree breeding, backcrossing, doubled-haploid breeding, and the like (see, Wernsman, E. A, and Rufty, R. C. 1987. Chapter Seventeen. Tobacco. Pages 669-698 In: Cultivar Development. Crop Species. W. H. Fehr (ed.), MacMillan Publishing Co, Inc., New York, N.Y 761 pp.). Molecular biology-based techniques described above, in particular RFLP and microsatelite markers, can be used in such backcrosses to identify the progenies having the highest degree of genetic identity with the recurrent parent. This permits one to accelerate the production of tobacco varieties having at least 90%, preferably at least 95%, more preferably at least 99% genetic identity with the recurrent parent, yet more preferably genetically identical to the recurrent parent, and further comprising the trait(s) introgressed from the donor parent. Such determination of genetic identity can be based on molecular markers known in the art.

The last backcross generation can be selfed to give pure breeding progeny for the nucleic acid(s) being transferred. The resulting plants generally have essentially all of the morphological and physiological characteristics of the mutant, non-naturally occuring or transgenic plants of the invention, in addition to the transferred trait(s) (for example, one or more single gene traits). The exact backcrossing protocol will depend on the trait being altered to determine an appropriate testing protocol. Although backcrossing methods are simplified when the trait being transferred is a dominant allele, a recessive allele may also be transferred. In this instance, it may be necessary to introduce a test of the progeny to determine if the desired trait has been successfully transferred.

Various embodiments provide mutant plants, non-naturally occurring plants or transgenic plants, as well as biomass in which the expression level of a polynucleotide (or any combination thereof as described herein) is modulated to modulate the level of at least one alkaloid therein.

### 7. Consumable products

Parts of the plants described herein, particularly the leaf lamina and midrib of such plants, can be incorporated into or used in making various consumable products including but not limited to aerosol forming materials, aerosol forming devices, smoking articles, smokable articles, smokeless products, medicinal or cosmetic products, intravenous preparations, tablets, powders, and tobacco products. Examples of aerosol forming materials include but are not limited to tobacco compositions, tobaccos, tobacco extract, cut tobacco, cut filler, cured tobacco, expanded tobacco, homogenized tobacco, reconstituted tobacco, and pipe tobaccos. Smoking articles and smokable articles are types of aerosol forming devices. Examples of smoking articles or smokable articles include but are not limited to cigarettes, cigarillos, and cigars. Examples of smokeless products comprise chewing tobaccos, and snuffs. In certain aerosol forming devices, rather than combustion, a tobacco composition or another aerosol forming material is heated by one or more electrical heating elements to produce an aerosol. In another type of heated aerosol forming device, an aerosol is produced by the transfer of heat from a combustible fuel element or heat source to a physically separate aerosol forming material, which may be located within, around or downstream of the heat source. Smokeless tobacco products and various tobacco-containing aerosol forming materials may contain tobacco in any form, including as dried particles, shreds, granules, powders, or a slurry, deposited on, mixed in, surrounded by, or otherwise combined with other ingredients in any format, such as flakes, films, tabs, foams, or beads. As used herein, the term 'smoke' is used to describe a type of aerosol that is produced by smoking articles, such as cigarettes, or by combusting an aerosol forming material.

In one embodiment, there is also provided cured plant material from the mutant, transgenic and non-naturally occurring plants described herein. Processes of curing green tobacco leaves are known by those having skills in the art and include without limitation air-curing, fire-curing, flue-curing and sun-curing as described herein.

In another embodiment, there is described tobacco products including tobacco-containing aerosol forming materials comprising plant material - such as leaves, preferably cured leaves - from the mutant tobacco plants, transgenic tobacco plants or non-naturally occurring tobacco plants described herein. The tobacco products described herein can be a blended tobacco product which may further comprise unmodified tobacco.

The amount of nicotine in these smokable articles and smokeless products and aerosols thereof may be at least about 5%, 10%, 15%, 20%, 25%, 30%, 35%, 40%, 45%, 50%, 55%, 60%, 65%, 70%, 75%, 80%, 85%, 90%, 95%, 96%, 97%, 98%, 99%, and 100% lower - such as about 200% or 300% lower - when compared to consumable products derived from non-mutant, non-naturally occurring or non-transgenic counterparts.

In embodiments, the product produced from the plants of the invention can be an alkaloid, including but not limited to nicotine, anatabine, caffeine, codeine, and morphine, in raw or purified form.

### 8. Products and methods for crop management and agriculture

The mutant, non-naturally occurring or transgenic plants may have other uses in, for example, agriculture. For example, mutant, non-naturally occurring or transgenic plants described herein can be used to make animal feed and human food products.

The invention also provides methods for producing seeds comprising cultivating the mutant plant, non-naturally occurring plant, or transgenic plant described herein, and collecting seeds from the cultivated plants. Seeds from plants described herein can be conditioned and bagged in packaging material by means known in the art to form an article of manufacture. Packaging material such as paper and cloth are well known in the art. A package of seed can have a label, for example, a tag or label secured to the packaging material, a label printed on the package that describes the nature of the seeds therein.

Compositions, methods and kits for genotyping plants for identification, selection, or breeding can comprise a means of detecting the presence of a polynucleotide (or any combination thereof as described herein) in a sample of polynucleotide. Accordingly, a composition is described comprising one or more primers for specifically amplifying at least a portion of one or more of the polynucleotides and optionally one or more probes and optionally one or more reagents for conducting the amplification or detection.

Accordingly, gene specific oligonucleotide primers or probes comprising about 10 or more contiguous polynucleotides corresponding to the polynucleotide(s) described herein are disclosed. Said primers or probes may comprise or consist of about 15, 20, 25, 30, 40, 45 or 50 more contiguous polynucleotides that hybridise (for example, specificially hybridise) to the polynucleotide(s) described herein. In some embodiments, the primers or probes may comprise or consist of about 10 to 50 contiguous nucleotides, about 10 to 40 contiguous nucleotides, about 10 to 30 contiguous nucleotides or about 15 to 30 contiguous nucleotides that may be used in sequence-dependent methods of gene identification (for example, Southern hybridization) or isolation (for example, *in situ* hybridization of bacterial colonies or bacteriophage plaques) or gene detection (for example, as one or more amplification primers in nucleic acid amplification or detection). The one or more specific primers or probes can be designed and used to amplify or detect a part or all of the polynucleotide(s). By way of specific example, two primers may be used in a polymerase chain reaction protocol to amplify a nucleic acid fragment encoding a nucleic acid - such as DNA or RNA. The polymerase chain reaction may also be performed using one primer that is derived from a nucleic acid sequence and a second primer that hybridises to the sequence upstream or downstream of the nucleic acid sequence - such as a promoter sequence, the 3' end of the mRNA precursor or a sequence derived from a vector. Examples of thermal and isothermal techniques useful for *in vitro* amplification of polynucleotides are well known in the art. The sample may be or may be derived from a plant, a plant cell or plant material or a tobacco product made or derived from the plant, the plant cell or the plant material as described herein.

In a further aspect, there is also provided a method of detecting a polynucleotide(s) described herein (or any combination thereof as described herein) in a sample comprising the step of: (a) providing a sample comprising, or suspected of comprising, a polynucleotide; (b) contacting said sample with one or more primers or one or more probes for specifically detecting at least a portion of the polynucleotide(s); and (c) detecting the presence of an amplification product, wherein the presence of an amplification product is indicative of the presence of the polynucleotide(s) in the sample. In a further aspect, there is also provided the use of one or more primers or probes for specifically detecting at least a portion of the polynucleotide(s). Kits for detecting at least a portion of the polynucleotide(s) are also provided which comprise one or more primers or probes for specifically detecting at least a portion of the polynucleotide(s). The kit may comprise reagents for polynucleotide amplification - such as PCR - or reagents for probe hybridization-detection technology - such as Southern Blots, Northern Blots, in-situ hybridization, or microarray. The kit may comprise reagents for antibody binding-detection technology such as Western Blots, ELISAs, SELDI mass spectrometry or test strips. The kit may comprise reagents for DNA sequencing. The kit may comprise reagents and instructions for determining the level of at least one alkaloid. Suitably, the kit comprises reagents and instructions for determining nicotine, anatabine, caffeine, codeine, or morphine content in plant material, cured plant material or cured leaves.

In some embodiments, a kit may comprise instructions for one or more of the methods described. The kits described may be useful for genetic identity determination, phylogenetic studies, genotyping, haplotyping, pedigree analysis or plant breeding particularly with co-dominant scoring.

The present invention also provides a method of genotyping a plant, a plant cell or plant material comprising a polynucleotide as described herein. Genotyping provides a means of distinguishing homologs of a chromosome pair and can be used to differentiate segregants in a plant population. Molecular marker methods can be used for phylogenetic studies, characterizing genetic relationships among crop varieties, identifying crosses or somatic hybrids, localizing chromosomal segments affecting monogenic traits, map based cloning, and the study of quantitative inheritance. The specific method of genotyping may employ any number of molecular marker analytic techniques including amplification fragment length polymorphisms (AFLPs). AFLPs are the product of allelic differences between amplification fragments caused by nucleotide sequence variability. Thus, the present invention further provides a means to follow segregation of one or more genes or nucleic acids as well as chromosomal sequences genetically linked to these genes or nucleic acids using such techniques as AFLP analysis.

### EXAMPLES

The invention is further described in the Examples below, which are provided to describe the invention in further detail. These examples, which set forth a preferred mode presently contemplated for carrying out the invention, are intended to illustrate and not to limit the invention.

The present invention has multiple aspects, illustrated by the following non-limiting examples.

### Example 1 - Genomic Analysis for ABCG1 variants

A proof-of-concept study using transgenic tobacco lines showed that such plants accumulate more nicotine and anatabine in leaves than control plants. Although the mechanism leading to higher alkaloids in leaf remains unclear, it is possible to speculate based on the observations of the orthologous protein function in *Arabidopsis* that modification of the root architecture would facilitate the translocation of nicotine and anatabine from root-to-shoot, NtABCG1-T and NtABCG1-S being also possibly nicotine transporters.

Based on transcriptomic experiments, two ABC transporter genes were identified in *Nicotiana tabacum* differentially expressed in PMT-RNAi lines and between vegetative and flowering tobacco plants. These two genes are homeologous copies (related to the tobacco ancestors *Nicotiana sylvestris, Nicotiana tomentosiformis)* because they present high identity to the same orthologous tomato gene. These two genes are predicted to encode for proteins presenting the hallmark signatures of the ABC-2 family of proteins. ABC-2 are members of the large ATP-binding cassette transporter family and more precisely ABC transporter G family member 1. ABCG transporters are half-size ABC transporters, requiring two proteins to be effective in the transport of molecules from one compartment to another, the transport energy being driven by ATP. In *Arabidopsis,* orthologous related ABCG proteins have been shown to be required for suberin and pollen wall extracellular barrier synthesis.

Two tobacco transcripts Ntab-TN90_AYMY-SS.Metabolites-Ntab-TN90.84076.1 and Ntab-TN90_AYMY-SS.Metabolites-Ntab-TN90.78588.1 (https://solgenomics.net) coding for NtABCG1-T and NtABCG1-S were identified by analogy with the tomato gene orthologue Solyc05g054890 and the Arabidopsis ABC transporters ABCG1, ABCG2, ABCG6, ABCG16 and ABCG20 (Figure 1).

### Example 2 - Study of NtABCG1 Genes in PMT-RNAi Lines

Nicotine content in leaves of Nicotiana tabacum PMT-RNAi lines is 25 fold lower (see Figure 2) in accordance with data presented in WO 2015/157359 A1 and in Chintapakorn Y, et Hamill JD (2003). Expression of these ABC transporters was found to be down-regulated by a factor 0.7 in PMT-RNAi lines when compared to control lines using Tobacco exon array Affymetrix chips (Martin F, Bovet L, Cordier A, et al. BMC Genomics. 2012; 13:674).

Such a down-regulation of ABCG transporters in PMT-RNAi lines suggests a possible interaction between nicotine accumulation in leaf and the root-to-shoot translocation mediated by *NtABCG1* genes.

Leaf nicotine content increased in tobacco leaves during plant growth and particularly during the transition period from vegetative to flowering stage (Figure 3A). In the same plants and at the same time, the expression of *NtABCG1* genes was measured in the roots via RNA-seq and found a concomitant decrease of the expression in both S and T copies (Figure 3B), thereby indicating a correlation between *NtABCG1* gene expression and nicotine leaf content.

Interestingly, *NtABCG1*-*S* and *-T* genes are not only expressed in roots but also in flowers, as can be seen in Figure 4. Expression of these genes could not be detected in other tissues of a flowering plant. The expression in both root and flower tissues is consistent with observations that the close Arabidopsis orthologues *ABCG2, ABCG6* and *ABCG20* (see Figure 1) are required for an effective suberin barrier in the roots and seed coats, and *ABCG1* and *ABCG16* for the synthesis of intact pollen wall (Yadav et al., 2014). Casparian strip is a suberized region likely controlling metabolite transports from cell-to-cell in the root, therefore the activity of *NtABCG1* genes, as for *ABCG2, ABCG6* and *ABCG20* in Arabidopsis (Yadav V, Molina I, Ranathunge K, Castillo IQ, Rothstein SJ, Reed JW. The Plant Cell. 2014;26(9):3569-3588) may change the shoot translocation of root synthesized products.

Our data show that *NtABCG1*-*S* and *-T* expression in roots is linked to nicotine accumulation in leaves. In order to investigate further the functional relevance of NtABCG1s we constructed proof of concept plants to reduce *NtABCG1s* expression through a RNA interference mechanism. RNAi is achieved by insertion of a DNA fragment promoting the expression of complementary inverted repeats partially covering the coding sequence of both genes in the plant. This tool is frequently used to study loss of function of a given gene or set of genes. Alternatively mutations in the gene sequence leading to similar loss of function can be identified by alternative methods, as described in detail above (e.g via EMS mutagenesis or via gene editing techniques like CRISPR-Cas based systems etc). All the techniques leading to loss of function are to be considered in this case. While we use RNAi as a quick proof of concept method, EMS mutagenesis is also a valid route.

### Example 3 - genetic manipulation of NtABCG1: NtABCG1-RNAi tobacco plants

T0 NtABCG1-RNAi tobacco plants were generated using the Gateway cloning System, (Invitrogen) which is a molecular biology tool kit that enables researchers to efficiently transfer DNA fragments between plasmids using a proprietary set of recombination sequences - the Gateway technology. The availability of gene cassettes in a standard Gateway cloning plasmid helps researchers quickly transfer such cassettes into plasmids that facilitate the analysis of gene function in tobacco for example. 13 transgenic independent events and 10 control plants were cultivated in pots in a greenhouse environment.

To ensure that a RNAi line in which the interference effect is efficient in suppressing the expression of targeted genes, the strategy is to produce several independent lines and quantify the expression of the targeted gene and consider this in relation to the observation of the phenotype/chemotype of each individual line. The alkaloid content of the 13 lines was quantified and is depicted in figure 5A and figure 6. As expected, the quantity of nicotine or anatabine is variable among the different lines. The line to line variation was evaluated using several control plants transformed with an empty vector (i.e. without RNAi but passed through all the transformation steps)

The data indicate that lines 10 and 11 had at least two times higher nicotine content than the control lines while the expression of *NtABCG1* genes is extremely low. This result show that plants lacking *NtABCG1* transcripts have a high nicotine content in leaves. Interestingly, **NtABCG1-RNAi** did not show any peculiar phenotype compared to the control plants. In addition, a lack of *NtABCG1* transcripts did not only contribute to an increase in nicotine content in leaves but also an increase in anatabine content (Figure 6A), thereby indicating that a general alkaloid increase is mediated by non-functional *NtABCG1* genes. The results also reasonably suggest that modified tobacco plants that overexpresses *NtABCG1* transcripts have a low nicotine content in leaves. Quality measurement of alkaloids is based on running tobacco sample replicates (Figure 6B). Concentration of nicotine was determined by HPLC-MS: Aliquots (-30 mg) of ground tobacco extracted with ethanol-water (1:1; 6 mL) for 45 minutes at 50°C. The centrifuged extracts are diluted 10-fold. Liquid chromatographic separation is performed on an amide column (Waters Acquity BEH amide, 2.1 x 150 mm, 1.7 µm) at 45°C eluting with a gradient of 2 mM ammonium formate in water + 0.25 % formic acid (eluent A) and acetonitrile + 0.1 % formic acid (eluent B), applying an eluent gradient (0 min - 10 % A, 0.5 min - 10 % A, 4 min - 60 % A, 4.5 min - 60 % A, 4.6 min - 10 % A, 6.8 min - 10 % A; flow 0.5 ml/min). Nicotine concentrations in the extracts are calculated from its peak area in the 260 nm trace of a photodiode UV/VIS detector.

Without wishing to be bound by theory, it is possible that the root of NtABCG1-RNAi plants by analogy with the *Arabidopsis* triple mutants of orthologue genes *abcg2, abcg6* and *abcg20* having a different root architecture. Such modification in the root fine structure may disturb some root activities by triggering the release of alkaloids from root-to-shoot or directly the synthesis of alkaloids and thus activating the alkaloids translocation from root-to-shoot in NtABCG1-RNAi plants.

### SEQUENCES:

SEQ ID NO. 1: Genomic sequence of the *N. tabacum* ABCG1-S gene

ATGTCACGGATAGTAGCCGAGAATACATTGCAAGGGGAAGAAAGTGTACAATTTTA
TGATCAAAGAGTACAACCCATGGAAATGTCACAGGCCAGCGCGTACAGTTCACCGA
CCCTTGGTCAGTTACTGAAGCGCGTGGGCGATGTCAGAAAGGAGGTCACCGGCGAC
GAAACTCCGGTACATCAAGTTCTTGATATGAGTGATCCAGGCATTCAACAAAACTCT
CTTCCATTTGTACTCTCCTTCAATAATCTCACATACAGCGTAAAAGTTCCCCGGAAA
ATGACTTTTCCGACGATCTTCCGGCGACCCGTAGCCGGAACCGCCGCAGCCACCGGG
GATCCGGTAGCCGGAGAAAACTTGTTCACGAGAACAAAAGTGCTCCTCGACAACAT

CTCCGGCGAGGCGCGTGATGGAGAAATCGTCGCCGTTCTAGGCGCGTCGGGCTCGG
GAAAATCGACACTCATCGACGGGTTGGCTAATCGGATTGCAAAAGAAAGCTTGAAA
GGAACAATAACGTTAAATGGAGAGCCATTGGATTCAAGATTGTTGAAAGTAATTTC
AGCATACGTCATGCAAGATGATCTTTTATATCCAATGTTAACAGTTGAAGAAACATT
AATGTTTGCTGCTGAATTCAGACTTCCTCGTACTCTGTCCAAATCAAAGAAGAAAAT
GAGAGTACAAGCTTTGATTGATCAATTAGGGTTACGAAATGCTGCAAAAACTATCAT
AGGCGATGAGGTACGTAAGCTTTTCATAAAATATTTTAAAAGCACAATTGCGAGTTT
ACGAACTTGAAAAATATAGAGTGCGTTCATAATATAAGGGTATGATAAATTCCACTC
TTACAAAATAGTATACTAGACTCGCACGTTTCTTCCATTTCTCTAAGTAAAGCTCTTT
GCATAGCAATGGTTAGTTGTTGTGGCTTAGCCTTTCATAAGTGGAGACAGAATAA
AGTGTTCATAATAAAGACGATCACTGTCTAATCTTGATTCAACACACTTCCACTGTG
CTATTCGAGTAGATATTGTTACTTTTTCTCGACCATAGTTCTAATATTATTTGATTAG
ATTATCCAATCTTTTATTTCTCTTGAGGCATATAGAGCATGGGAGGGAAAATAAAAA
TGCATATTGATAAATATTATATAATGATAAATTCCACTCTTACAAAATAGTATACTA
GACTCTCATATATCTATATATAGGAGCTATATTAGAACACTACAACGAAATGTTGTT
ATATAAAGCATATAGTAATATAAACATAACATTAAAGATTGATTTCAAACAAAACTT
GGCCTTTGTAATGAAATATTGTTATATAGAATGATTGTTATAGAGGTCTGATTGTATT
TCCAACAGGGTCATCGTGGAGTGTCTGGTGGAGAACGACGACGAGTTTCCATAGGA
ATTGATATTATTCATGACCCCATCATCTTATTTCTCGACGAACCAACTTCAGGGCTTG
ATTCCACTAGTGCATACATGGTAGTGAAAGTTCTTCAAAGAATTGCTCAAAGTGGAA
GTATTGTAATCATGTCAATTCATCAACCAAGTTATAGAATTCTTGGTTTATTGGATCG
TATGCTTTTCTTGTCCCGTGGACAAACTGTTTATAGTGGATCCCCTATGAATCTTCCA
CATTTCTTTGCTGATTTTGGTCACCCGATACCAGACAATGAAAATCGGACAGAGTTT
GCCCTGGATTTAATTCGTGAGCTAGAAGGATCCCCAGGAGGGACAAAAAGTTTAGT
CGAATTCAATAAAACATGGCAAAATACCAAAAGGCAGAGCAATCAAAATTGTGAAA
TAGCAACTCCAACACATGGATTATCATTGAAAGAAGCAATAAGTGCAAGCATTTCA
AGAGGGAAATTGGTTTCAGGAACAACAAGTGATCATACTTCCCCTGCATCAATGGTT
CCTACATACGCGAATCCCTTTTGGATCGAAATGACTACGTTGTCAAAGAGATCGTTT
ACTAATTCGTGGAGGGTGCCCGAGCTATTTGGCATTCGTTTAGGTGCAATCGTTGTC
ACGGGGTTCATCTTAGCTACTATGTTTTGGCAACTTGACAATTCACCTAAAGGGGTT
CAAGAAAGACTTGGTTTTTTTGCATTTGCAATGTCAACAACTTTCTACACTTGTGCTG

ATGCATTGCCTGTTTTCTACAAGAAAGGTATATTTTATGAGGGAAACAGCTTATA
ATGCATATAGGAGATCATCTTATTGTCTATCTCATGCTTTGGTTTCTTTACCAGCATT
GATTTTCCTATCATTTGCATTTGCTGCTATAACATTTTGGGCAGTTGGACTAGATGGT
GGATTTTCTGGTTTCATGTTTTATTTTGGGATAATCTTGGCTTCATTTTGGGCTGGAA
ATTCATTTGTCACATTCTTATCTGGAGTTGTACCTAGTGTTATGCTAGGTTACACAAT
TGTTGTGGCAATTCTTGCCTATTTCTTGCTCTTCTCTGGATTTTTCATGAATCGTGATC
GAATCCCACCTTATTGGATATGGTTTCACTATCTTTCCTTGGTGAAATATCCATATGA
AGCTGTGTTACAAAATGAATTTGATGATCCAACAAAATGTTTTGTTAAGGGAATTCA
AATGTTTGATAATTCTCCACTTGGATCAGTGCCAATTGAATTGAAAGAGAAGTTGTT
GAGTACAATGAGTAATACATTGAATGTCAAGATTACTGGTTCTACTTGTGTTACTAC
TGGTGCTGATATATTGGTTCAACAAGGGATTACTGAGTTAAGTATGTGGGGTTGTTT
GTGGATTACTATTGCATGGGGATTTTTCTTTAGGGTTTTGTTTTACTTCAGTTTGTTGC
TTGGAAGTAAGAACAAGAGAAGGTAA

Genomic sequence includes intron/exons.
SEQ ID NO. 2: Amino acid sequence of the *N. tabacum* ABCG1-S deduced from SEQ ID 1 
MSRIVAENTLQGEESVQFYDQRVQPMEMSQASAYSSPTLGQLLKRVGDVRKEVTGDET
PVHQVLDMSDPGIQQNSLPFVLSFNNLTYSVKVPRKMTFPTIFRRPVAGTAAATGDPVA
GENLFTRTKVLLDNISGEARDGEIVAVLGASGSGKSTLIDGLANRIAKESLKGTITLNGEP
LDSRLLKVISAYVMQDDLLYPMLTVEETLMFAAEFRLPRTLSKSKKKMRVQALIDQLGL
RNAAKTIIGDEGHRGVSGGERRRVSIGIDIIHDPIILFLDEPTSGLDSTSAYMVVKVLQRIA
QSGSIVIMSIHQPSYRILGLLDRMLFLSRGQTVYSGSPMNLPHFFADFGHPIPDNENRTEF
ALDLIRELEGSPGGTKSLVEFNKTWQNTKRQSNQNCEIATPTHGLSLKEAISASISRGKLV
SGTTSDHTSPASMVPTYANPFWIEMTTLSKRSFTNSWRVPELFGIRLGAIVVTGFILATMF
WQLDNSPKGVQERLGFFAFAMSTTFYTCADALPVFLQERYIFMRETAYNAYRRSSYCLS
HALVSLPALIFLSFAFAAITFWAVGLDGGFSGFMFYFGIILASFWAGNSFVTFLSGVVPSV
MLGYTIVVAILAYFLLFSGFFMNRDRIPPYWIWFHYLSLVKYPYEAVLQNEFDDPTKCF
VKGIQMFDNSPLGSVPIELKEKLLSTMSNTLNVKITGSTCVTTGADILVQQGITELSMWG
CLWITIAWGFFFRVLFYFSLLLGSKNKRR
SEQ ID NO. 3: Genomic sequence of the N. tabacum ABCG1-T gene

ATGTCACGGATAGTAGCGGAGAATACATTGCAAGGGGAGAAAGTGTACAATTTTA
TGATCAAAGAGTACAACCCATGGAAATGTCACAGGCCAGCGCGTACAGTTCACCGA
CCCTTGGTCAGTTATTGAAGCGCGTGGGCGATGTCAGAAAGGAAGTCACCGGCGAC
GAAACTCCGGTACACCAAGTTCTTGATATGAGTGATCCAGGCATTCAACAAAACTCT
CTTCCATTTGTACTCTCCTTCAATAACCTCACATACAGCGTAAAAGTCCGTCGTAAA
ATGACTTTCCCGACGTTATTCCGGCAACCCGTCGCCGGAACCGCCGCAGCCACCGGA
GAGCCTGTTCCCGGAGAAAACTTGTTCACGAGAACAAAAGTGCTCCTCGACAACAT
CTCCGGCGAGGCGCGTGACGGAGAAATCGTCGCCGTTCTAGGCGCGTCAGGCTCGG
GAAAATCGACACTCATCGACGGGTTAGCTAATCGGATTGCAAAAGAAAGCTTGAAA
GGAACTATAACGTTAAATGGGGAGCCACTGGATTCAAGATTGTTGAAAGTAATTTCA
GCATACGTCATGCAAGATGATCTTTTATATCCAATGTTAACAGTTGAAGAAACATTA
ATGTTTGCTGCTGAATTCAGACTTCCTCGTACTCTGTCCAAATCAAAGAAGAAAATG
AGAGTACAAGCTTTGATTGATCAACTAGGGTTACGAAATGCTGCAAAAACTATCATA
GGTGATGAGGTACGTAAGCTTTTCATAAATTATTATAATCACAATTTTGAGTTTACG
AACTTGATAAATATAGAGCGCATTCAAATAAATAGAATCAGTAAACATCCAATATC
ATGAAAAGCATACTAATAATATAAGAGTATGATAAATTCCACTCTTACAAAATAGTA
AACTAGACTCACACGCTCATTCTATTTCTCTAAGTAGAGCTCTTTGCATCGCAATAGT
TAGCTGTTGTCTGGCTTAGCTTTCATAGGTGTAGACAGAATAAAGCGTTCATAATAA
AGACGTTTACGGTCTATTCTTGATTCAACACACTTTCACTGTGATATTCGAGTAGATA
TTGTTACTTTTTCTCGACCATAGTTCTAATATTATTTGATTGGATTATCAAATCTTTTA
TTTCTCTTGAGGCACATAGAGTATGGAAGGGAAAATAAACATGCATATTGATAATAT
AACGATAAATTTCACTCTTACAAAATAGTACACTAGACTCACATATATATAGGTGCT
ATATTAGATCATTATAGTGAAATGTTGTTATATAAAGCATATAATATAATATAACAG
TAAAGACCGATTTCAAATAAAACTTAGCCATTATAATGAAATGTTGTTATAAAGAAT
GATTGTTATAGAGGTCTAATTATATTTCCAACAGGGTCATCGTGGAGTGTCCGGGGG
CGAACGACGACGAGTTTCCATAGGAATTGATATTATTCATGACCCCATCATCTTGTT
TCTCGATGAACCAACTTCAGGACTTGATTCCACTAGTGCATACATGGTAGTGAAAGT
TCTTCAAAGAATTGCTCAAAGTGGAAGTATTGTGATCATGTCAATTCATCAGCCAAG
TTATAGAATTCTTAGTTTATTGGATCGTATGCTTTTCTTGTCCCGTGGACAAACTGTT
TATAGTGGATCCCCTATGAATCTTCCACATTTCTTTGCTGATTTTGGTCATCCGATAC

CAGATAATGAAAATCGGACAGAGTTCGCCCTGGATTTAATTCGTGAGCTAGAAGGA
TCCCCAGGAGGGACAAAAAGTTTGGTTGAGTTCAATAAAACATGGCAAAATACAAA
AAGGCAGAGCAATCAAAATTGTGAAATAGTAACTCCAACACATGGATTATCATTGA
AGGAAGCAATAAGTGCAAGCATTTCAAGAGGTAAATTGGTTTCAGGAACAACAAGT
GATCACACTTCCCCTGCTTCAATGGTTCCTACATACGCGAATCCCTTTTGGATCGAAA
TGACTACGTTGTCCAAGAGATCGTTTACTAATTCGTGGAGGGTGCCCGAGCTATTTG
GCATTCGTTTAGGTGCAATCGTTGTCACGGGGTTCATCTTAGCTACCATGTTTTGGCA
ACTTGACAATTCACCTAAAGGTGTTCAAGAAAGACTTGGTTTTTTGCATTTGCAAT
GTCAACAACTTTCTATACTTGTGCTGATGCATTGCCTGTTTTCTACAAGAAAGGTAC
ATTTTATGAGGGAAACAGCTTATAATGCATATAGGAGATCTTCTTATTGTCTATCTC
ATGCTTTGGTTTCTTTACCAGCATTGATTTCCTATCATTTGCATTTGCTGCTATAACT
TTTTGGGCAGTTGGACTTGATGGTGGATTTTCTGGTTTCTTGTTCTATTTTGGGATAA
TTTTAGCCTCTTTTTGGGCTGGAAATTCATTTGTCACATTCCTTTCTGGAGTTGTACCT
AGTGTTATGCTAGGATACACAATTGTTGTGGCAATTCTTGCCTATTTCTTGCTCTTCT
CTGGATTTTTTATGAATCGCGATCGAATCCCACCTTATTGGATATGGTTTCACTATCT
TTCCTTAGTGAAGTATCCATATGAAGCTGTATTACAAAATGAATTTGATGATCCAAC
AAAATGTTTTGTTAAGGGAATTCAAATGTTTGATAATTCACCACTTGGATCAGTGCC
AATTGCTTTGAAAGAGAAGTTGTTGAGTACAATGAGTAATACATTGAATGTCAAGAT
TACTGGATCTACTTGTGTGACTACTGGAGCTGATATATTGGTACAACAAGGATTAC
TGAGTTAAGTATGTGGGGTTGCTTGTGGATTACTATTGCATGGGGGTTTTTCTTTAGG
GTTTTGTTTTACTTCAGTTTGTTGCTTGGAAGTAAGAACAAGAGAAGGTAA

Genomic sequence includes intron/exons.
SEQ ID NO. 4: Amino acid sequence of the *N. tabacum* ABCG1-T deduced from SEQ ID 3 
MSRIVAENTLQGGESVQFYDQRVQPMEMSQASAYSSPTLGQLLKRVGDVRKEVTGDET
PVHQVLDMSDPGIQQNSLPFVLSFNNLTYSVKVRRKMTFPTLFRQPVAGTAAATGEPVP
GENLFTRTKVLLDNISGEARDGEIVAVLGASGSGKSTLIDGLANRIAKESLKGTITLNGEP
LDSRLLKVISAYVMQDDLLYPMLTVEETLMFAAEFRLPRTLSKSKKKMRVQALIDQLGL
RNAAKTIIGDEGHRGVSGGERRRVSIGIDIIHDPIILFLDEPTSGLDSTSAYMVVKVLQRIA
QSGSIVIMSIHQPSYRILSLLDRMLFLSRGQTVYSGSPMNLPHFFADFGHPIPDNENRTEF
 
ALDLIRELEGSPGGTKSLVEFNKTWQNTKRQSNQNCEIVTPTHGLSLKEAISASISRGKLV
SGTTSDHTSPASMVPTYANPFWIEMTTLSKRSFTNSWRVPELFGIRLGAIVVTGFILATMF
WQLDNSPKGVQERLGFFAFAMSTTFYTCADALPVFLQERYIFMRETAYNAYRRSSYCLS
HALVSLPALIFLSFAFAAITFWAVGLDGGFSGFLFYFGIILASFWAGNSFVTFLSGVVPSV
MLGYTIVVAILAYFLLFSGFFMNRDRIPPYWIWFHYLSLVKYPYEAVLQNEFDDPTKCF
VKGIQMFDNSPLGSVPIALKEKLLSTMSNTLNVKITGSTCVTTGADILVQQGITELSMWG
CLWITIAWGFFFRVLFYFSLLLGSKNKRR
SEQ ID NO. 5: DNA sequence used for silencing NtABCG1-S/T in NtABCG1-RNAi plants 
TTTATTGGATCGTATGCTTTCTTGTCCCGTGGACAAACTGTTATAGTGGATCCCCT
ATGAATCTTCCACATTTCTTTGCTGATTTTGGTCA
SEQ ID NO. 6: DNA sequence of qPCR P1 Forward primer
   P1-Forward CATACGTCATGCAAGATGATCTTTT
SEQ ID NO. 7: DNA sequence of qPCR P1 Reverse primer
   P1 -Reverse ACAGAGTACGAGGAAGTCTGAATTC
SEQ ID NO. 8: DNA sequence of qPCR P2 Forward primer
   P2-Forward ATGCATTGCCTGTTTTTCTACAAGA
SEQ ID NO. 9: DNA sequence of qPCR P2 Reverse primer
   P2-Reverse TGGTAAAGAAACCAAAGCATGAGAT

### OTHER EMBODIMENTS

Other aspects and embodiments of the invention are described in the following numbered paragraphs:
1. A method for producing a plant, comprising the steps of:
   (a) providing a plant cell comprising a polynucleotide comprising, consisting or consisting essentially of a sequence having at least 80% sequence identity to at least one of SEQ ID NO: 1 or SEQ ID No: 3;
   (b) modifying the plant cell to modulate the expression or activity of said polynucleotide or polypeptide as compared to a control plant cell in which the expression or activity of said polynucleotide or polypeptide has not been altered; and
   (c) propagating the plant cell into a plant.
2. A method according to paragraph 1, wherein step (c) comprises cultivating the plant from a cutting or seedling comprising the plant cell.
3. A method according to paragraph 1 or paragraph 2, wherein the plant cell is also modified to modulate the expression or activity of one or more of *A622, BBLa, BBLb, JRESL1, JRE5L2, MATE1, MATE 2, MPO1, MPO2, MYC2a, MYC2b, NBB1, nic1, nic2, NUP1, NUP2, PMT1, PMT2, PMT3, PMT4, QPT,* and their gene products.
4. A method according to any of paragraphs 1 to 3, wherein the step of modifying the plant cell comprises modifying the genome of the cell by a genome editing technology or by genome engineering techniques.
5. A method according to paragraph 4, wherein the genome editing technology or genome engineering technique is selected from CRISPR/Cas technology, zinc finger nuclease-mediated mutagenesis, chemical or radiation mutagenesis, homologous recombination, oligonucleotide-directed mutagenesis and meganuclease-mediated mutagenesis.
6. A method according to any of paragraphs 1 to 5, wherein the step of modifying the plant cell comprises operably linking the polynucleotide to a constitutive promoter.
7. A method according to any of paragraphs 1 to 6, wherein the step of modifying the plant cell comprises transfecting the cell with a construct comprising a polynucleotide comprising, consisting, or consisting essentially of a sequence having at least 80% sequence identity to SEQ ID NO: 1 or SEQ ID NO: 3 operably linked to a constitutive promoter.
8. A method according to any of paragraphs 1 to 5 wherein the step of modifying the plant cell comprises introducing an interference polynucleotide comprising a sequence that is at least 80% complementary to an RNA transcribed from the polynucleotide of step (a) into the cell.
9. A method according to paragraph 8 wherein the plant cell is transfected with a construct expressing an interference polynucleotide comprising a sequence that is at least 80% complementary to at least 19 nucleotides of an RNA transcribed from the polynucleotide of step (a).
10. A method for producing cured plant material with an altered alkaloid concentration as compared to control plant material comprising the steps of:
   (a) providing a plant or plant material as described herein;
   (b) optionally harvesting the plant material therefrom; and
   (c) curing the plant material.
11. A method according to paragraph 10, wherein the plant material comprises cured leaves, stems or flowers, a mixture thereof.
12. A method according to paragraph 10 or paragraph 11, wherein the curing procedure is selected from the group consisting of air curing, fire curing, smoke curing, and flue curing.

## Claims

1. A plant cell comprising:
(i) a polynucleotide comprising, consisting or consisting essentially of a sequence having at least 80% sequence identity to SEQ ID NO: 1 or SEQ ID No: 3;
(ii) a polypeptide encoded by the polynucleotide set forth in (i);
(iii) a polypeptide comprising, consisting or consisting essentially of a sequence having at least 80% sequence identity to SEQ ID NO: 2 or SEQ ID No: 4; or
(iv) a construct, vector or expression vector comprising the isolated polynucleotide set forth in (i),
wherein said plant cell comprises at least one modification which modulates the expression or activity of said polynucleotide or polypeptide as compared to a control plant cell in which the expression or activity of said polynucleotide or polypeptide has not been altered.

2. A plant cell according to claim 1, wherein the plant cell also comprises a modification which modulates the expression or activity of one or more of *A622, BBLa, BBLb, JRESL1, JRE5L2, MATE1, MATE 2, MPO1, MPO2, MYC2a, MYC2b, NBB1, nic1, nic2, NUP1, NUP2, PMT1, PMT2, PMT3, PMT4, QPT,* and their gene products.

3. A plant cell according to claim 1 or claim 2, wherein at least one modification is a modification of the genome or of the construct, vector, expression vector, or a transgenic modification; preferably wherein the modification of the genome, construct, vector, or expression vector is a mutation or edit.

4. A plant cell according to claim 3, wherein the mutation or edit is located in a regulatory sequence governing expression from the polynucleotide.

5. A plant cell according to any preceding claim, wherein at least one modification reduces the expression or activity of said polynucleotide or polypeptide as compared to the control plant cell.

6. A plant cell according to any of claims 1 to 4, wherein at least one modification increases the expression or activity of said polynucleotide or polypeptide.

7. A plant cell according to any of claims 2 to 4, wherein at least one modification increases the expression or activity of said polynucleotide or polypeptide, and at least one modification has one or more of the following effects:
i) increasing the expression or activity of one or both of *MATE1* and *MATE2;* and
ii) reducing the expression or activity of one or more of *A622, BBLa, BBLb, JRE5L1, JRE5L2, MPO1, MPO2, MYC2a, MYC2b, NBB1, nic1, nic2, NUP1, NUP2, PMT1, PMT2, PMT3, PMT4,* and *QPT,*
as compared to a control plant cell in which the expression or activity of said polynucleotide, polypeptide, or gene has not been altered.

8. A plant cell according to claim 7, wherein the expression or activity of both genes in one of the following pairs of genes, or of their gene products, has been reduced as compared to a control plant cell in which the expression or activity of said polynucleotide, polypeptide, gene, or gene product has not been altered: *A622* and *NBB; A622* and *QPT; QPT* and *PMT1; QPT* and *PMT2; QPT* and *PMT3; QPT* and *PMT4; MYC2a* and *PMT1; MYC2a* and *PMT2; MYC2a* and *PMT3; MYC2a* and *PMT4; MYC2b* and *PMT1; MYC2b* and *PMT2; MYC2b* and *PMT3; MYC2b* and *PMT4; MPO1* and *PMT1; MPO1* and *PMT2; MPO1* and *PMT3; MPO1* and *PMT4; MPO1* and *A622; MPO1* and *NBB1; MPO1* and *QPT; MPO2* and *PMT1; MPO2* and *PMT2; MPO2* and *PMT3; MPO2* and *PMT4; MPO2* and *A622; MPO2* and *NBB1; MPO2* and *QPT.*

9. A plant or part thereof comprising the plant cell according to any preceding claim.

10. A plant or part thereof according to claim 9, wherein the plant is one of the following genotypes:
i) SEQ ID NO.1 homozygous, SEQ ID NO.3 homozygous;
ii) SEQ ID NO. 1 homozygous, SEQ ID NO.3 heterozygous;
iii) SEQ ID NO. 1 heterozygous, SEQ ID NO.3 homozygous; or
iv) SEQ ID NO. 1 heterozygous, SEQ ID NO.3 heterozygous.

11. A plant or part thereof according to claim 9 or 10, wherein the concentration of at least one alkaloid is modified in at least a part of the plant as compared to a control plant that does not comprise a plant cell according to claims 1 to 8.

12. A plant or part thereof according to claim 11, wherein the at least one alkaloid is one or more of nicotine, anatabine, caffeine, codeine, and morphine.

13. Plant material, cured plant material, or homogenized plant material, derived from the plant of any of claims 9 to 12; preferably comprising biomass, seed, stem, flowers, or leaves from the plant of any of claims 9 to 12.

14. Plant material, cured plant material, or homogenized plant material according to claim 13, wherein the plant is a tobacco plant.

15. A tobacco product comprising the plant cell of any of claims 1 to 8, at least a part of the plant of claims 9 to 12 or the plant material according to claim 13 or claim 14.
